# EUROPEAN PATENT SPECIFICATION

(11) **EP 1 446 403 B1**
(45) Date of publication and mention of the grant of the patent: **12.04.2006**
(21) Application number: 02770098.8
(22) Date of filing: 23.10.2002
(51) Int. Cl.: C07D 417/10, A61K 31/433, A61P 31/04

(54) **ARYL SUBSTITUTED OXAZOLIDINONES WITH ANTIBACTERIAL ACTIVITY**
ARYL SUBSTITUIERTE OXAZOLIDINONE MIT ANTIBACTERIELLER ACTIVITÄT
OXAZOLIDINONES SUBSTITUEES PAR UN GROUPE ARYLE PRESENTANT UNE ACTIVITE ANTIBACTERIENNE

(30) Priority: 25.10.2001 US 330589 P
(43) Date of publication of application: 18.08.2004
(73) Proprietor: AstraZeneca AB, 151 85 Södertälje (SE)
(72) Inventor: GRAVESTOCK, Michael, Barry, Waltham, MA 02451 (US)
(86) International application number: PCT/GB2002/004796
(87) International publication number: WO 2003/035648

(56) References cited:
- WO-A-93/23384
- WO-A-99/64416
- US-A- 3 318 878
- PAE A N ET AL: "Synthesis and in vitro activity of new oxazolidinone antibacterial agents having substituted isoxazoles" BIOORGANIC & MEDICINAL CHEMISTRY LETTERS, OXFORD, GB, vol. 9, no. 18, 20 September 1999 (1999-09-20), pages 2679-2684, XP004179951 ISSN: 0960-894X

## Description

The present invention relates to antibiotic compounds and in particular to antibiotic compounds containing a substituted oxazolidinone ring. This invention further relates to processes for their preparation, to intermediates useful in their preparation, to their use as therapeutic agents and to pharmaceutical compositions containing them.

The international microbiological community continues to express serious concern that the evolution of antibiotic resistance could result in strains against which currently available antibacterial agents will be ineffective. In general, bacterial pathogens may be classified as either Gram-positive or Gram-negative pathogens. Antibiotic compounds with effective activity against both Gram-positive and Gram-negative pathogens are generally regarded as having a broad spectrum of activity. The compounds of the present invention are regarded as effective against both Gram-positive and certain Gram-negative pathogens.

Gram-positive pathogens, for example Staphylococci, Enterococci, and Streptococci are particularly important because of the development of resistant strains which are both difficult to treat and difficult to eradicate from the hospital environment once established. Examples of such strains are methicillin resistant staphylococcus (MRSA), methicillin resistant coagulase negative staphylococci (MRCNS), penicillin resistant Streptococcus pneumoniae and multiply resistant Enterococcus faecium.

The major clinically effective antibiotic for treatment of such resistant Gram-positive pathogens is vancomycin. Vancomycin is a glycopeptide and is associated with nephrotoxicity and ototoxicity. Furthermore, and most importantly, antibacterial resistance to vancomycin and other glycopeptides is also appearing. This resistance is increasing at a steady rate rendering these agents less and less effective in the treatment of Gram-positive pathogens. There is also now increasing resistance appearing towards agents such as β-lactams, quinolones and macrolides used for the treatment of upper respiratory tract infections, also caused by certain Gram negative strains including H.influenzae and M.catarrhalis.

Certain antibacterial compounds containing an oxazolidinone ring have been described in the art (for example, Walter A. Gregory et al in J.Med.Chem. 1990, 33, 2569-2578 and Chung-Ho Park et al in J.Med.Chem. 1992, 35, 1156-1165). Such antibacterial oxazolidinone compounds with a 5-acetamidomethyl sidechain may be subject to mammalian peptidase metabolism. Furthermore, bacterial resistance to known antibacterial agents may develop, for example, by (i) the evolution of active binding sites in the bacteria rendering a previously active pharmacophore less effective or redundant, (ii) the evolution of means to chemically deactivate a given pharmacophore and/or (iii) the development and/or up-regulation of efflux mechanisms. Therefore, there remains an ongoing need to find new antibacterial agents with a favourable pharmacological profile, in particular for compounds containing new pharmacophores.

We have discovered a new class of antibiotic compounds containing an aryl substituted oxazolidinone ring in which the aryl ring is itself further substituted. These compounds have useful activity against Gram-positive pathogens including MRSA and MRCNS and, in particular, against various strains exhibiting resistance to vancomycin and against E. faecium strains resistant to both aminoglycosides and clinically used β-lactams, but also to fastidious Gram negative strains such as H.influenzae, M.catarrhalis, mycoplasma spp. and chlamydial strains.

Accordingly the present invention provides a compound of the formula (I), or a pharmaceutically-acceptable salt, or an in-vivo-hydrolysable ester thereof, 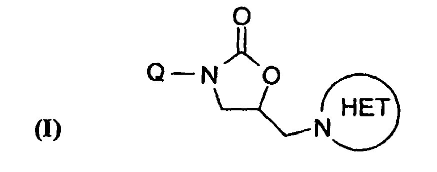 wherein
HET is an N-linked 5-membered, fully or partially unsaturated heterocyclic ring, containing either (i) 1 to 3 further nitrogen heteroatoms or (ii) a further heteroatom selected from O and S together with an optional further nitrogen heteroatom; which ring is optionally substituted on a C atom, other than a C atom adjacent to the linking N atom, by an oxo or thioxo group; and/or which ring is optionally substituted on any available C atom, other than a C atom adjacent to the linking N atom, by a substituent Rs wherein;
Rs is selected from the group:
(Rsa): halogen, (1-4C)alkoxy, (2-4C)alkenyloxy, (2-4C)alkenyl, (2-4C)alkynyl, (3-6C)cycloalkyl, (3-6C)cycloalkenyl, amino, (1-4C)alkylamino, di-(1-4C)alkylamino, (2-4C)alkenylamino, (1-4C)alkylcarbonylamino, (1-4C)alkylthiocarbonylamino, (1-4C)alkyl-OCO-NH-, (1-4C)alkyl-NH-CO-NH-, (1-4C)alkyl-NH-CS-NH-, (1-4C)alkyl-SO₂-NH- or (1-4C)alkyl-S(O)q- (wherein q is 0, 1 or 2);
   or Rs is selected from the group
(Rsb): (1-4C)alkyl group which is optionally substituted by one substituent selected from hydroxy, (1-4C)alkoxy, amino, cyano, azido, (2-4C)alkenyloxy, (1-4C)alkylcarbonyl, (1-4C)alkoxycarbonyl, (1-4C)alkylamino, (2-4C)alkenylamino, (1-4C)alkyl-SO₂-NH-, (1-4C)alkylcarbonylamino, (1-4C)alkylthiocarbonylamino, (1-4C)alkyl-OCO-NH-, (1-4C)alkyl-NH-CO-NH-, (1-4C)alkyl-NH-CS-NH-, (1-4C)alkyl-SO₂-NH-, (1-4C)alkyl-S(O)q- (wherein q is 0, 1 or 2), (3-6C)cycloalkyl, (3-6C)cycloalkenyl, or an N-linked 5-membered heteroaryl ring, which ring contains either (i) 1 to 3 further nitrogen heteroatoms or (ii) a further heteroatom selected from O and S together with an optional further nitrogen heteroatom; which ring is optionally substituted on a carbon atom by an oxo or thioxo group; and/or the ring is optionally substituted on a carbon atom by 1 or 2 (1-4C)alkyl groups; and/or on an available nitrogen atom (provided that the ring is not thereby quaternised) by (1-4C)alkyl;
and wherein at each occurrence of an Rs substituent containing an alkyl, alkenyl, alkynyl, cycloalkyl or cycloalkenyl moiety in (Rsa) or (Rsb) each such moiety is optionally further substituted on an available carbon atom with one or more substituents independently selected from F, Cl and Br and/or by one cyano group; and/or which ring is optionally substituted on an available nitrogen atom (provided that the ring is not thereby quaternised) by (1-4C)alkyl; or
HET is an N-linked 6-membered di-hydro-heteroaryl ring containing up to three nitrogen heteroatoms in total (including the linking heteroatom), which ring is substituted on a suitable C atom, other than a C atom adjacent to the linking N atom, by oxo or thioxo and/or which ring is optionally substituted on any available C atom, other than a C atom adjacent to the linking N atom, by one or two substituents Rs, wherein Rs is as hereinbefore defined, and/or on an available nitrogen atom (provided that the ring is not thereby quaternised) by (1-4C)alkyl; and wherein at each occurrence of alkyl, alkenyl and cycloalkyl HET substituents, each is optionally substituted with one or more substituents independently selected from F, Cl and Br and/or by one cyano group;
Q is Q1:- 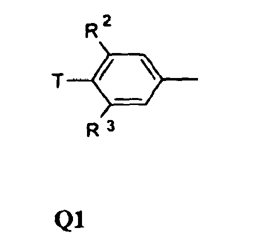 wherein R² and R³ are independently hydrogen or fluoro;
T is selected from the following groups of formula (TAa1 to (TAa6) below (wherein AR1, AR2, AR2a, AR2b, AR3, AR3a, AR3b, AR4, AR4a, CY1 and CY2 are defined hereinbelow); 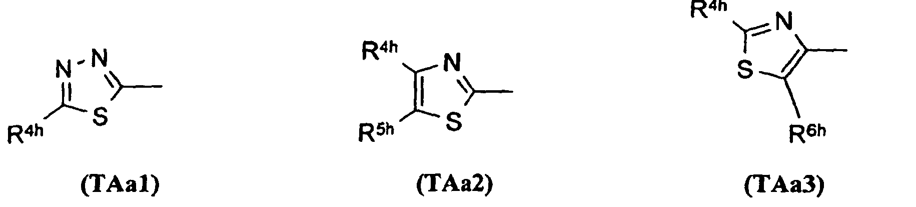 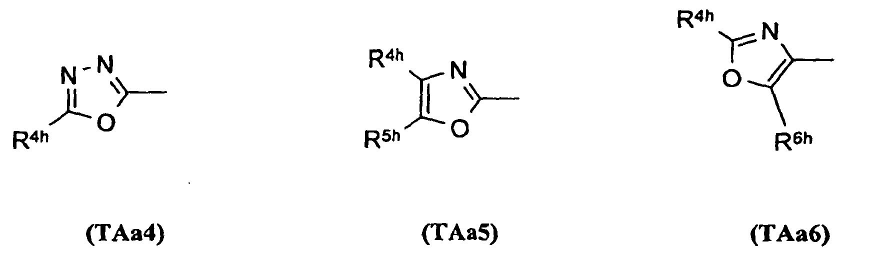 wherein :
R^{6h} is hydrogen or (1-4C)alkyl, and R^{4h} and R^{5h} are independently selected from hydrogen, cyano, (1-4C)alkoxycarbonyl, -CONRvRw, hydroxy(1-4C)alkyl,
NRvRw(1-4C)alkyl, -NRcRv(1-4C)alkyl; wherein Rv is hydrogen or (1-4C)alkyl; Rw is hydrogen or (1-4C)alkyl};
wherein Rc is selected from groups (Rc1) to (Rc5) :-
***(Rc1)*** (1-6C)alkyl {optionally substituted by one or more (1-4C)alkanoyl groups (including geminal disubstitution) and/or optionally monosubstituted by cyano, (1-4C)alkoxy, trifluoromethyl, (1-4C)alkoxycarbonyl, phenyl (optionally substituted as for AR1 defined hereinafter), (1-4C)alkylS(O)_{q}- (q is 0, 1 or 2); or, on any but the first carbon atom of the (1-6C)alkyl chain, optionally substituted by one or more groups (including geminal disubstitution) each independently selected from hydroxy and fluoro, and/or optionally monosubstituted by oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is 1 or 2)};
***(Rc2)*** R¹³CO- , R¹³SO₂- or R¹³CS-
   wherein R¹³ is selected from (Rc2a) to (Rc2e) :-
   ***(Rc2a)*** AR1, AR2, AR2a, AR2b, AR3, AR3a, AR3b, AR4, AR4a, CY1, CY2;
   ***(Rc2b)*** hydrogen, (1-4C)alkoxycarbonyl, trifluoromethyl, -NRvRw [wherein Rv is hydrogen or (1-4C)alkyl; Rw is hydrogen or (1-4C)alkyl], ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, 2-(AR2a)ethenyl;
   ***(Rc2c)*** (1-10C)alkyl {optionally substituted by one or more groups (including geminal disubstitution) each independently selected from hydroxy, (1-10C)alkoxy, (1-4C)alkoxy-(1-4C)alkoxy, (1-4C)alkoxy-(1-4C)alkoxy-(1-4C)alkoxy, (1-4C)alkanoyl, carboxy, phosphoryl [-OP(O)(OH)₂, and mono- and di-(1-4C)alkoxy derivatives thereof], phosphiryl [-O-P(OH)₂ and mono- and di-(1-4C)alkoxy derivatives thereof], and amino; and/or optionally substituted by one group selected from phosphonate [phosphono, -P(O)(OH)₂, and mono- and di-(1-4C)alkoxy derivatives thereof], phosphinate [-P(OH)₂ and mono- and di-(1-4C)alkoxy derivatives thereof], cyano, halo, trifluoromethyl, (1-4C)alkoxycarbonyl, (1-4C)alkoxy-(1-4C)alkoxycarbonyl, (1-4C)alkoxy-(1-4C)alkoxy-( 1-4C)alkoxycarbonyl, (1-4C)alkylamino, di((1-4C)alkyl)amino, (1-6C)alkanoylamino, (1-4C)alkoxycarbonylamino, N-(1-4C)alkyl-N-(1-6C)alkanoylamino, (1-4C)alkylaminocarbonyl, di((1-4C)alkyl)aminocarbonyl, (1-4C)alkylS(O)ₚNH-, (1-4C)alkylS(O)ₚ-((1-4C)alkyl)N-, fluoro(1-4C)alkylS(O)ₚNH-, fluoro(1-4C)alkylS(O)ₚ((1-4C)alkyl)N-, (1-4C)alkylS(O)_{q}- [the (1-4C)alkyl group of (1-4C)alkylS(O)_{q}- being optionally substituted by one substituent selected from hydroxy, (1-4C)alkoxy, (1-4C)alkanoyl, phosphoryl [-O-P(O)(OH)₂, and mono- and di-(1-4C)alkoxy derivatives thereof], phosphiryl [-O-P(OH)₂ and mono- and di-(1-4C)alkoxy derivatives thereof], amino, cyano, halo, trifluoromethyl, (1-4C)alkoxycarbonyl, (1-4C)alkoxy-(1-4C)alkoxycarbonyl, (1-4C)alkoxy-(1-4C)alkoxy-(1-4C)alkoxycarbonyl, carboxy, (1-4C)alkylamino, di((1-4C)alkyl)amino, (1-6C)alkanoylamino, (1-4C)alkoxycarbonylamino, N-(1-4C)alkyl-N-(1-6C)alkanoylamino, (1-4C)alkylaminocarbonyl, di((1-4C)alkyl)aminocarbonyl, (1-4C)alkylS(O)ₚNH-, (1-4C)alkylS(O)ₚ-((1-4C)alkyl)N-, (1-4C)alkylS(O)_{q}-, AR1-S(O)_{q}- , AR2-S(O)_{q}-, AR3-S(O)_{q}- and also AR2a, AR2b, AR3a and AR3b versions of AR2 and AR3 containing groups], CY1, CY2, AR1, AR2, AR3, AR1-O-, AR2-O-, AR3-O-, AR1-S(O)_{q}-, AR2-S(O)_{q}-, AR3-S(O)_{q}-, AR1-NH-, AR2-NH-, AR3-NH- (p is 1 or 2 and q is 0, 1 or 2), and also AR2a, AR2b, AR3a and AR3b versions of AR2 and AR3 containing groups};
   ***(Rc2d)*** R¹⁴C(O)O(1-6C)alkyl wherein R¹⁴ is AR1, AR2, (1-4C)alkylamino (the (1-4C)alkyl group being optionally substituted by (1-4C)alkoxycarbonyl or by carboxy), benzyloxy-(1-4C)alkyl or (1-10C)alkyl {optionally substituted as defined for (Rc2c)};
   ***(Rc2e)*** R¹⁵O- wherein R¹⁵ is benzyl, (1-6C)alkyl {optionally substituted as defined for (Rc2c)}, CY1, CY2 or AR2b;
***(Rc3)*** hydrogen, cyano, 2-cyanoethenyl, 2-cyano-2-((1-4C)alkyl)ethenyl, 2-((1-4C)alkylaminocarbonyl)ethenyl, 2-((1-4C)alkoxycarbonyl)ethenyl, 2-nitroethenyl, 2-nitro-2-((1-4C)alkyl)ethenyl, 2-(AR1)ethenyl, 2-(AR2)ethenyl, or of the formula **(Rc3a)** 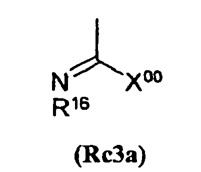 wherein X⁰⁰ is -OR¹⁷, -SR¹⁷, -NHR¹⁷ and -N(R¹⁷)₂ ;
   wherein R¹⁷ is hydrogen (when X⁰⁰ is -NHR¹⁷ and -N(R¹⁷)₂), and R¹⁷ is (1-4C)alkyl, phenyl or AR2 (when X⁰⁰ is -OR¹⁷, -SR¹⁷ and -NHR¹⁷); and R¹⁶ is cyano, nitro, (1-4C)alkylsulfonyl, (4-7C)cycloalkylsulfonyl, phenylsulfonyl, (1-4C)alkanoyl and (1-4C)alkoxycarbonyl;
***(Rc4)*** trityl, AR1, AR2, AR2a, AR2b, AR3, AR3a, AR3b;
***(Rc5)*** RdOC(Re)=CH(C=O)-, RfC(=O)C(=O)-, RgN=C(Rh)C(=O)- or RiNHC(Rj)=CHC(=O)- wherein Rd is (1-6C)alkyl; Re is hydrogen or (1-6C)alkyl, or Rd and Re together form a (3-4C)alkylene chain; Rf is hydrogen, (1-6C)alkyl, hydroxy(1-6C)alkyl, (1-6C)alkoxy(1-6C)alkyl, -NRvRw [wherein Rv is hydrogen or (1-4C)alkyl; Rw is hydrogen or (1-4C)alkyl], (1-6C)alkoxy, (1-6C)alkoxy(1-6C)alkoxy, hydroxy(2-6C)alkoxy, (1-4C)alkylamino(2-6C)alkoxy, di-(1-4C)alkylamino(2-6C)alkoxy; Rg is (1-6C)alkyl, hydroxy or (1-6C)alkoxy; Rh is hydrogen or (1-6C)alkyl; Ri is hydrogen, (1-6C)alkyl, AR1, AR2, AR2a, AR2b and Rj is hydrogen or (1-6C)alkyl;
wherein
**AR1** is an optionally substituted phenyl or optionally substituted naphthyl;
**AR2** is an optionally substituted 5- or 6-membered, fully unsaturated (i.e with the maximum degree of unsaturation) monocyclic heteroaryl ring containing up to four heteroatoms independently selected from O N and S (but not containing any O-O, O-S or S-S bonds), and linked via a ring carbon atom, or a ring nitrogen atom if the ring is not thereby quatemised; **AR2a** is a partially hydrogenated version of AR2 (i.e. AR2 systems retaining some, but not the full, degree of unsaturation), linked via a ring carbon atom or linked via a ring nitrogen atom if the ring is not thereby quaternised;
**AR2b** is a fully hydrogenated version of AR2 (i.e. AR2 systems having no unsaturation), linked via a ring carbon atom or linked via a ring nitrogen atom;
**AR3** is an optionally substituted 8-, 9- or 10-membered, fully unsaturated (i.e with the maximum degree of unsaturation) bicyclic heteroaryl ring containing up to four heteroatoms independently selected from O, N and S (but not containing any O-O, O-S or S-S bonds), and linked via a ring carbon atom in either of the rings comprising the bicyclic system;
**AR3a** is a partially hydrogenated version of AR3 (i.e. AR3 systems retaining some, but not the full, degree of unsaturation), linked via a ring carbon atom, or linked via a ring nitrogen atom if the ring is not thereby quaternised, in either of the rings comprising the bicyclic system;
**AR3b** is a fully hydrogenated version of AR3 (i.e. AR3 systems having no unsaturation), linked via a ring carbon atom, or linked via a ring nitrogen atom, in either of the rings comprising the bicyclic system;
**AR4** is an optionally substituted 13- or 14-membered, fully unsaturated (i.e with the maximum degree of unsaturation) tricyclic heteroaryl ring containing up to four heteroatoms independently selected from O, N and S (but not containing any O-O, O-S or S-S bonds), and linked via a ring carbon atom in any of the rings comprising the tricyclic system;
**AR4a** is a partially hydrogenated version of AR4 (i.e. AR4 systems retaining some, but not the full, degree of unsaturation), linked via a ring carbon atom, or linked via a ring nitrogen atom if the ring is not thereby quaternised, in any of the rings comprising the tricyclic system;
**CY1** is an optionally substituted cyclobutyl, cyclopentyl or cyclohexyl ring;
**CY2** is an optionally substituted cyclopentenyl or cyclohexenyl ring.

In another embodiment, the present invention provides a compound of the formula (I) as hereinbefore described, or a pharmaceutically-acceptable salt, or an in-vivo hydrolysable ester thereof, wherein:
HET is an N-linked 5-membered, fully or partially unsaturated heterocyclic ring, containing either (i) 1 to 3 further nitrogen heteroatoms or (ii) a further heteroatom selected from O and S together with an optional further nitrogen heteroatom; which ring is optionally substituted on a C atom, other than a C atom adjacent to the linking N atom, by an oxo or thioxo group; and/or which ring is optionally substituted on any available C atom, other than a C atom adjacent to the linking N atom, by a substituent selected from (1-4C)alkyl, (2-4C)alkenyl, (3-6C)cycloalkyl, amino, (1-4C)alkylamino, di-(1-4C)alkylamino, (1-4C)alkylthio, (1-4C)alkoxy, (1-4C)alkoxycarbonyl, halogen, cyano and trifluoromethyl and/or on an available nitrogen atom (provided that the ring is not thereby quaternised) by (1-4C)alkyl; or HET is an N-linked 6-membered di-hydro-heteroaryl ring containing up to three nitrogen heteroatoms in total (including the linking heteroatom), which ring is substituted on a suitable C atom, other than a C atom adjacent to the linking N atom, by oxo or thioxo and/or which ring is optionally substituted on any available C atom, other than a C atom adjacent to the linking N atom, by one or two substituents independently selected from (1-4C)alkyl, (2-4C)alkenyl, (3-6C)cycloalkyl, amino, (1-4C)alkylamino, di-(1-4C)alkylamino, (1-4C)alkylthio, (1-4C)alkoxy, (1-4C)alkoxycarbonyl, halogen, cyano and trifluoromethyl and/or on an available nitrogen atom (provided that the ring is not thereby quaternised) by (1-4C)alkyl; and wherein at each occurrence of alkyl, alkenyl and cycloalkyl HET substituents, each is optionally substituted with one or more F, Cl or CN.

In this specification, HET as an N-linked 5-membered ring may be a fully or partially unsaturated heterocyclic ring, provided there is some degree of unsaturation in the ring.

Particular examples of N-linked 5-membered heteroaryl rings containing 2 to 4 heteroatoms independently selected from N, O and S (with no O-O, O-S or S-S bonds) are preferably rings containing 2 to 4 N atoms, in particular pyrazole, imidazole, 1,2,3-triazole (preferably 1,2,3-triazol-1-yl), 1,2,4-triazole (preferably 1,2,4-triazol-1-yl) and tetrazole (preferably tetrazol-2-yl).

Particular examples of N-linked 6-membered di-hydro-heteroaryl rings containing up to three nitrogen heteroatoms in total (including the linking heteroatom) include di-hydro versions of pyrimidine, pyridazine, pyrazine, 1,2,3-triazine, 1,2,4-triazine, 1,3,5-triazine and pyridine.

It is to be understood that when a value for -X¹- is a two-atom link and is written, for example, as -CH₂NH- it is the left hand part (-CH₂- here) which is bonded to the group of formula (TAa1) to (TAa6) and the right hand part (-NH- here) which is bonded to -Y¹- in the definition in (TAac). Similarly, when -Y¹- is a two-atom link and is written, for example, as -CONH- it is the left hand part of -Y¹- (-CO- here) which is bonded to the right hand part of -X¹-, and the right hand part of -Y¹- (-NH- here) which is bonded to the AR2, AR2a, AR2b, AR3, AR3a or AR3b moiety in the definition in (TAac).

In this specification the term 'alkyl' includes straight chained and branched structures. For example, (1-6C)alkyl includes propyl, isopropyl and tert-butyl. However, references to individual alkyl groups such as "propyl" are specific for the straight chained version only, and references to individual branched chain alkyl groups such as "isopropyl" are specific for the branched chain version only. A similar convention applies to other radicals, for example halo(1-4C)alkyl includes 1-bromoethyl and 2-bromoethyl.

There follow particular and suitable values for certain substituents and groups referred to in this specification. These values may be used where appropriate with any of the definitions and embodiments disclosed hereinbefore, or hereinafter.

Examples of **(1-4C)alkyl** and **(1-5C)alkyl** include methyl, ethyl, propyl, isopropyl and t-butyl; examples of **(1-6C)alkyl** include methyl, ethyl, propyl, isopropyl, t-butyl, pentyl and hexyl; examples of **(1-10C)alkyl** include methyl, ethyl, propyl, isopropyl, pentyl, hexyl, heptyl, octyl and nonyl; examples of **(1-4C)alkanoylamino-(1-4C)alkyl** include formamidomethyl, acetamidomethyl and acetamidoethyl; examples of **hydroxy(1-4C)alkyl** and **hydroxy(1-6C)alkyl** include hydroxymethyl, 1-hydroxyethyl, 2-hydroxyethyl and 3-hydroxypropyl; examples of **(1-4C)alkoxycarbonyl** include methoxycarbonyl, ethoxycarbonyl and propoxycarbonyl; examples of **2-((1-4C)alkoxycarbonyl)ethenyl** include 2-(methoxycarbonyl)ethenyl and 2-(ethoxycarbonyl)ethenyl; examples of **2-cyano-2-((1-4C)alkyl)ethenyl** include 2-cyano-2-methylethenyl and 2-cyano-2-ethylethenyl; examples of **2-nitro-2-((1-4C)alkyl)ethenyl** include 2-nitro-2-methylethenyl and 2-nitro-2-ethylethenyl; examples of **2-((1-4C)alkylaminocarbonyl)ethenyl** include 2-(methylaminocarbonyl)ethenyl and 2-(ethylaminocarbonyl)ethenyl; examples of **(2-4C)alkenyl** include allyl and vinyl; examples of **(2-4C)alkynyl** include ethynyl and 2-propynyl; examples of **(1-4C)alkanoyl** include formyl, acetyl and propionyl; examples of **(1-4C)alkoxy** include methoxy, ethoxy and propoxy; examples of **(1-6C)alkoxy** and **(1-10C)alkoxy** include methoxy, ethoxy, propoxy and pentoxy; examples of **(1-4C)alkylthio** include methylthio and ethylthio; examples of **(1-4C)alkylamino** include methylamino, ethylamino and propylamino; examples of **di-((1-4C)alkyl)amino** include dimethylamino, N-ethyl-N-methylamino, diethylamino, N-methyl-N-propylamino and dipropylamino; examples of **halo** groups include fluoro, chloro and bromo; examples of **(1-4C)alkylsulfonyl** include methylsulfonyl and ethylsulfonyl; examples of **(1-4C)alkoxy-(1-4C)alkoxy** and **(1-6C)alkoxy-(1-6C)alkoxy** include methoxymethoxy, 2-methoxyethoxy, 2-ethoxyethoxy and 3-methoxypropoxy; examples of **(1-4C)alkaxy-(1-4C)alkaxy-(1-4C)alkoxy** include 2-(methoxymethoxy)ethoxy, 2-(2-methoxyethoxy)ethoxy; 3-(2-methoxyethoxy)propoxy and 2-(2-ethoxyethoxy)ethoxy; examples of **(1-4C)alkylS(O)**_{**2**}**amino** include methylsulfonylamino and ethylsulfonylamino; examples of **(1-4C)alkanoylamino** and **(1-6C)alkanoylamino** include formamido, acetamido and propionylamino; examples of **(14C)alkoxycarbonylamino** include methoxycarbonylamino and ethoxycarbonylamino; examples of **N-(1-4C)alkyl-N-(1-6C)alkanoylamino** include N-methylacetamido, N-ethylacetamido and N-methylpropionamido; examples of **(1-4C)alkylthiocarbonylamino** include MeS-C(=O)-N-and EtS-C(=O)-N-; examples of **(1-4C)alkylS(O)**_{**p**}**NH-** wherein p is 1 or 2 include methylsulfinylamino, methylsulfonylamino, ethylsulfinylamino and ethylsulfonylamino; examples of **(1-4C)alkylS(O)**_{**p**}**((1-4C)alkyl)N-** wherein p is 1 or 2 include methylsulfinylmethylamino, methylsulfonylmethylamino, 2-(ethylsulfinyl)ethylamino and 2-(ethylsulfonyl)ethylamino; examples of **fluoro(1-4C)alkylS(O)**_{**p**}**NH-** wherein p is 1 or 2 include trifluoromethylsulfinylamino and trifluoromethylsulfonylamino; examples of **fluoro(1-4C)alkylS(O)**_{**p**}**((1-4C)alkyl)NH-** wherein p is 1 or 2 include trifluoromethylsulfinylmethylamino and trifluoromethylsulfonylmethylamino; examples of **(1-4C)alkoxy(hydroxy)phosphoryl** include methoxy(hydroxy)phosphoryl and ethoxy(hydroxy)phosphoryl; examples of **di-(1-4C)alkoxyphosphoryl** include di-methoxyphosphoryl, di-ethoxyphosphoryl and ethoxy(methoxy)phosphoryl; examples of **(1-4C)alkylS(O)**_{**q**}**-** wherein q is 0, 1 or 2 include methylthio, ethylthio, methylsulfinyl, ethylsulfinyl, methylsulfonyl and ethylsulfonyl; examples of **phenylS(O)**_{**q**} and **naphthylS(O)**_{**q**}**-** wherein q is 0, 1 or 2 are phenylthio, phenylsulfinyl, phenylsulfonyl and naphthylthio, naphthylsulfinyl and naphthylsulfonyl respectively; examples of **benzyloxy-(1-4C)alkyl** include benzyloxymethyl and benzyloxyethyl; examples of a **(3-4C)alkylene** chain are trimethylene or tetramethylene; examples of **(1-6C)alkoxy-(1-6C)alkyl** include methoxymethyl, ethoxymethyl and 2-methoxyethyl; examples of **hydroxy-(2-6C)alkoxy** include 2-hydroxyethoxy and 3-hydroxypropoxy; examples of **(1-4C)alkylamino-(2-6C)alkoxy** include 2-methylaminoethoxy and 2-ethylaminoethoxy; examples of **di-(1-4C)alkylamino-(2-6C)alkoxy** include 2-dimethylaminoethoxy and 2-diethylaminoethoxy; examples of **phenyl(1-4C)alkyl** include benzyl and phenethyl; examples of **(1-4C)alkylcarbamoyl** include methylcarbamoyl and ethylcarbamoyl; examples of **di((1-4C)alkyl)carbamoyl** include di(methyl)carbamoyl and di(ethyl)carbamoyl; examples of **hydroxyimino(1-4C)alkyl** include hydroxyiminomethyl, 2-(hydroxyimino)ethyl and 1-(hydroxyimino)ethyl; examples of **(1-4C)alkoxyimino-(1-4C)alkyl** include methoxyiminomethyl, ethoxyiminomethyl, 1-(methoxyimino)ethyl and 2-(methoxyimino)ethyl; examples of **halo(1-4C)alkyl** include, halomethyl, 1-haloethyl, 2-haloethyl, and 3-halopropyl; examples of **nitro(1-4C)alkyl** include nitromethyl, 1-nitroethyl, 2-nitroethyl and 3-nitropropyl; examples of **amino(1-4C)alkyl** include aminomethyl, 1-aminoethyl, 2-aminoethyl and 3-aminopropyl; examples of **cyano(1-4C)alkyl** include cyanomethyl, 1-cyanoethyl, 2-cyanoethyl and 3-cyanopropyl; examples of **(1-4C)alkanesulfonamido** include methanesulfonamido and ethanesulfonamido; examples of **(1-4C)alkylaminosulfonyl** include methylaminosulfonyl and ethylaminosulfonyl; and examples of **di-(1-4C)alkylaminosulfonyl** include dimethylaminosulfonyl, diethylaminosulfonyl and N-methyl-N-ethylaminosulfonyl; examples of **(1-4C)alkanesulfonyloxy** include methylsulfonyloxy, ethylsulfonyloxy and propylsulfonyloxy; examples of **(1-4C)alkanoyloxy** include acetoxy; examples of **(1-4C)alkylaminocarbonyl** include methylaminocarbonyl and ethylaminocarbonyl; examples of **di((1-4C)alkyl)aminocarbonyl** include dimethylaminocarbonyl and diethylaminocarbonyl; examples of **(3-6C)cycloalkyl and (3-8C)cycloalkyl** include cyclopropyl, cyclobutyl, cyclopentyl and cyclohexyl; examples of **(4-7C)cycloalkyl** include cyclobutyl, cyclopentyl and cyclohexyl; examples of **(3-6C)cycloalkenyl** include cyclopentenyl and cyclohexenyl; examples of **di(N-(1-4C)alkyl)aminomethylimino** include dimethylaminomethylimino and diethylaminomethylimino.

Particular values for AR2 include, for example, for those AR2 containing one heteroatom, furan, pyrrole, thiophene; for those AR2 containing one to four N atoms, pyrazole, imidazole, pyridine, pyrimidine, pyrazine, pyridazine, 1,2,3- & 1,2,4-triazole and tetrazole; for those AR2 containing one N and one O atom, oxazole, isoxazole and oxazine; for those AR2 containing one N and one S atom, thiazole and isothiazole; for those AR2 containing two N atoms and one S atom, 1,2,4- and 1,3,4-thiadiazole.

Particular examples of AR2a include, for example, dihydropyrrole (especially 2,5-dihydropyrrol-4-yl) and tetrahydropyridine (especially 1,2,5,6-tetrahydropyrid-4-yl).

Particular examples of AR2b include, for example, tetrahydrofuran, pyrrolidine, morpholine (preferably morpholino), thiomorpholine (preferably thiomorpholino), piperazine (preferably piperazino), imidazoline and piperidine, 1,3-dioxolan-4-yl, 1,3-dioxan-4-yl, 1,3-dioxan-5-yl and 1,4-dioxan-2-yl.

Particular values for AR3 include, for example, bicyclic benzo-fused systems containing a 5- or 6-membered heteroaryl ring containing one nitrogen atom and optionally 1-3 further heteroatoms chosen from oxygen, sulfur and nitrogen. Specific examples of such ring systems include, for example, indole, benzofuran, benzothiophene, benzimidazole, benzothiazole, benzisothiazole, benzoxazole, benzisoxazole, quinoline, quinoxaline, quinazoline, phthalazine and cinnoline.

Other particular examples of AR3 include 5/5-, 5/6 and 6/6 bicyclic ring systems containing heteroatoms in both of the rings. Specific examples of such ring systems include, for example, purine and naphthyridine.

Further particular examples of AR3 include bicyclic heteroaryl ring systems with at least one bridgehead nitrogen and optionally a further 1-3 heteroatoms chosen from oxygen, sulfur and nitrogen. Specific examples of such ring systems include, for example, 3H-pyrrolo[1,2-a]pyrrole, pyrrolo[2,1-b]thiazole, 1H-imidazo[1,2-a]pyrrole, 1H-imidazo[1,2-a]imidazole, 1H,3H-pyrrolo[1,2-c]oxazole, 1H-imidazo[1,5-a]pyrrole, pyrrolo[1,2-b]isoxazole, imidazo[5,1-b]thiazole, imidazo[2,1-b]thiazole, indolizine, imidazo[1,2-a]pyridine, imidazo[1,5-a]pyridine, pyrazolo[1,5-a]pyridine, pyrrolo[1,2-b]pyridazine, pyrrolo[1,2-c]pyrimidine, pyrrolo[1,2-a]pyrazine, pyrrolo[1,2-a]pyrimidine, pyrido[2,1-c]-s-triazole, s-triazole[1,5-a]pyridine, imidazo[1,2-c]pyrimidine, imidazo[1,2-a]pyrazine, imidazo[1,2-a]pyrimidine, imidazo[1,5-a]pyrazine, imidazo[1,5-a]pyrimidine, imidazo[1,2-b]-pyridazine, s-triazolo[4,3-a]pyrimidine, imidazo[5,1-b]oxazole and imidazo[2,1-b]oxazole. Other specific examples of such ring systems include, for example, [1H]-pyrrolo[2,1-c]oxazine, [3H]-oxazolo[3,4-a]pyridine, [6H]-pyrrolo[2,1-c]oxazine and pyrido[2,1-c][1,4]oxazine. Other specific examples of 5/5- bicyclic ring systems are imidazooxazole or imidazothiazole, in particular imidazo[5,1-b]thiazole, imidazo[2,1-b]thiazole, imidazo[5,1-b]oxazole or imidazo[2,1-b]oxazole.

Particular examples of AR3a and AR3b include, for example, indoline, 1,3,4,6,9,9a-hexahydropyrido[2,1c][1,4]oxazin-8-yl, 1,2,3,5,8,8a-hexahydroimidazo[1,5a]pyridin-7-yl, 1,5,8,8a-tetrahydrooxazolo[3,4a]pyridin-7-yl, 1,5,6,7,8,8a-hexahydrooxazolo[3,4a]pyridin-7-yl, (7aS)[3H,5H]-1,7a-dihydropyrrolo[1,2c]oxazol-6-yl, (7aS)[5H]-1,2,3,7a-tetrahydropyrrolo[1,2c]imidazol-6-yl, (7aR)[3H,5H]-1,7a-dihydropyrrolo[1,2c]oxazol-6-yl, [3H,5H]-pyrrolo[1 ,2-c]oxazol-6-yl, [5H]-2,3-dihydropyrrolo[1,2-c]imidazol-6-yl, [3H,SH]-pyrrolo[1,2-c]thiazol-6-yl, [3H.5H]-1,7a-dihydropyrrolo[1,2-c]thiazol-6-yl, [5H]-pyrrolo[1,2-c]imidazol-6-yl, [1H]-3,4,8,8a-tetrahydropyrrolo[2,1-c]oxazin-7-yl, [3H]-1,5,8,8a-tetrahydrooxazolo [3,4-a]pyrid-7-yl, [3H]-5,8-dihydroxazolo[3,4-a]pyrid-7-yl and 5,8-dihydroimidazo [1,5-a] pyrid-7-yl.

Particular values for AR4 include, for example, pyrrolo[a]quinoline, 2,3-pyrroloisoquinoline, pyrrolo[a]isoquinoline, 1H-pyrrolo[1,2-a]benzimidazole, 9H-imidazo[1,2-a]indole,5H-imidazo[2,1-a]isoindole, 1H-imidazo[3,4-a]indole, imidazo[1,2-a]quinoline, imidazo[2,1-a]isoquinoline, imidazo[1,5-a]quinoline and imidazo[5,1-a]isoquinoline.

The nomenclature used is that found in, for example, "Heterocyclic Compounds (Systems with bridgehead nitrogen), W.L.Mosby (Intercsience Publishers Inc., New York), 1961, Parts 1 and 2.

Where optional substituents are listed such substitution is preferably not geminal disubstitution unless stated otherwise. If not stated elsewhere suitable optional substituents for a particular group are those as stated for similar groups herein.

Suitable substituents on AR1, AR2, AR2a, AR2b, AR3, AR3a, AR3b, AR4, AR4a, CY1 and CY2 are (on an available carbon atom) up to three substituents independently selected from (1-4C)alkyl {optionally substituted by (preferably one) substituents selected independently from hydroxy, trifluoromethyl, (1-4C)alkyl S(O)_{q}- (q is 0, 1 or 2) (this last substituent preferably on AR 1 only), (1-4C)alkoxy, (1-4C)alkoxycarbonyl, cyano, nitro,
(1-4C)alkanoylamino, -CONRvRw or -NRvRw}, trifluoromethyl, hydroxy, halo, nitro, cyano, thiol, (1-4C)alkoxy, (1-4C)alkanoyloxy, dimethylaminomethyleneaminocarbonyl, di(N-(1-4C)alkyl)aminomethylimino, carboxy, (1-4C)alkoxycarbonyl, (1-4C)alkanoyl, (1-4C)alkylSO₂amino, (2-4C)alkenyl {optionally substituted by carboxy or (1-4C)alkoxycarbonyl}, (2-4C)alkynyl, (1-4C)alkanoylamino, oxo (=O), thioxo (=S),
(1-4C)alkanoylamino {the (1-4C)alkanoyl group being optionally substituted by hydroxy}, (1-4C)alkyl S(O)_{q}- (q is 0, 1 or 2) {the (1-4C)alkyl group being optionally substituted by one or more groups independently selected from cyano, hydroxy and
(1-4C)alkoxy}, -CONRvRw or -NRvRw [wherein Rv is hydrogen or (1-4C)alkyl; Rw is hydrogen or (1-4C)alkyl].

Further suitable substituents on AR1, AR2, AR2a, AR2b, AR3, AR3a, AR3b, AR4, AR4a, CY1 and CY2 (on an available carbon atom), and also on alkyl groups (unless indicated otherwise) are up to three substituents independently selected from trifluoromethoxy, benzoylamino, benzoyl, phenyl {optionally substituted by up to three substituents independently selected from halo, (1-4C)alkoxy or cyano}, furan, pyrrole, pyrazole, imidazole, triazole, pyrimidine, pyridazine, pyridine, isoxazole, oxazole, isothiazole, thiazole, thiophene, hydroxyimino(1-4C)alkyl, (1-4C)alkoxyimino(1-4C)alkyl, halo-(1-4C)alkyl, (1-4C)alkanesulfonamido, -SO₂NRvRw [wherein Rv is hydrogen or (1-4C)alkyl; Rw is hydrogen or (1-4C)alkyl].

Preferable optional substituents on Ar2b as 1,3-dioxolan-4-yl, 1,3-dioxan-4-yl, 1,3-dioxan-5-yl or 1,4-dioxan-2-yl are mono- or disubstitution by substituents independently selected from (1-4C)alkyl (including geminal disubstitution), (1-4C)alkoxy, (1-4C)alkylthio, acetamido, (1-4C)alkanoyl, cyano, trifluoromethyl and phenyl].

Preferable optional substituents on CY1 & CY2 are mono- or disubstitution by substituents independently selected from (1-4C)alkyl (including geminal disubstitution), hydroxy, (1-4C)alkoxy, (1-4C)alkylthio, acetamido, (1-4C)alkanoyl, cyano, and trifluoromethyl.

Suitable substituents on AR2, AR2a, AR2b, AR3, AR3a, AR3b, AR4 and AR4a are (on an available nitrogen atom, where such substitution does not result in quaternization) (1-4C)alkyl, (1-4C)alkanoyl {wherein the (1-4C)alkyl and (1-4C)alkanoyl groups are optionally substituted by (preferably one) substituents independently selected from cyano, hydroxy, nitro, trifluoromethyl, (1-4C)alkyl S(O)_{q}- (q is 0, 1 or 2), (1-4C)alkoxy, (1-4C)alkoxycarbonyl, (1-4C)alkanoylamino, -CONRvRw or -NRvRw [wherein Rv is hydrogen or (1-4C)alkyl; Rw is hydrogen or (1-4C)alkyl]}, (2-4C)alkenyl, (2-4C)alkynyl, (1-4C)alkoxycarbonyl or oxo (to form an N-oxide).

Suitable pharmaceutically-acceptable salts include acid addition salts such as methanesulfonate, fumarate, hydrochloride, citrate, maleate, tartrate and (less preferably) hydrobromide. Also suitable are salts formed with phosphoric and sulfuric acid. In another aspect suitable salts are base salts such as an alkali metal salt for example sodium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine, tris-(2-hydroxyethyl)amine, N-methyl d-glucamine and amino acids such as lysine. There may be more than one cation or anion depending on the number of charged functions and the valency of the cations or anions. A preferred pharmaceutically-acceptable salt is the sodium salt.

However, to facilitate isolation of the salt during preparation, salts which are less soluble in the chosen solvent may be preferred whether pharmaceutically-acceptable or not.

An in-vivo hydrolysable ester of a compound of the formula (I) or a pharmaceutically-acceptable salt thereof containing carboxy or hydroxy group is, for example, a pharmaceutically-acceptable ester which is hydrolysed in the human or animal body to produce the parent acid or alcohol.

Suitable pharmaceutically-acceptable esters for carboxy include (1-6C)alkoxymethyl esters for example methoxymethyl, (1-6C)alkanoyloxymethyl esters for example pivaloyloxymethyl, phthalidyl esters, (3-8C)cycloalkoxycarbonyloxy(1-6C)alkyl esters for example 1-cyclohexylcarbonyloxyethyl; 1,3-dioxolan-2-onylmethyl esters for example 5-methyl-1,3-dioxolan-2-ylmethyl; and (1-6C)alkoxycarbonyloxyethyl esters for example 1-methoxycarbonyloxyethyl and may be formed at any carboxy group in the compounds of 1 this invention.

An in-vivo hydrolysable ester of a compound of the formula (I) or a pharmaceutically-acceptable salt thereof containing a hydroxy group or groups includes inorganic esters such as phosphate esters (including phosphoramidic cyclic esters) and α-acyloxyalkyl ethers and related compounds which as a result of the in-vivo hydrolysis of the ester breakdown to give the parent hydroxy group/s. Examples of α-acyloxyalkyl ethers include acetoxymethoxy and 2,2-dimethylpropionyloxymethoxy. A selection of in-vivo hydrolysable ester forming groups for hydroxy include (1-10C)alkanoyl, benzoyl, phenylacetyl and substituted benzoyl and phenylacetyl, (1-10C)alkoxycarbonyl (to give alkyl carbonate esters), di-(1-4C)alkylcarbamoyl and N-(di-(1-4C)alkylaminoethyl)-N-(1-4C)alkylcarbamoyl (to give carbamates), di-(1-4C)alkylaminoacetyl and carboxyacetyl. Examples of substituents on benzoyl and phenylacetyl include chloromethyl or aminomethyl, (1-4C)alkylaminomethyl and di-((1-4C)alkyl)aminomethyl, and morpholino or piperazino linked from a ring nitrogen atom via a methylene linking group to the 3- or 4-position of the benzoyl ring.

Certain suitable in-vivo hydrolysable esters of a compound of the formula (I) are described within the definitions listed in this specification, for example esters described by the definition (Rc2d), and some groups within (Rc2c). Suitable in-vivo hydrolysable esters of a compound of the formula (I) are described as follows. For example, a 1,2-diol may be cyclised to form a cyclic ester of formula (PD1) or a pyrophosphate of formula (PD2) :

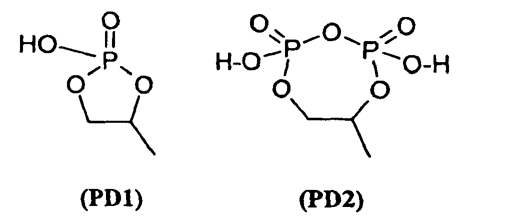

Particularly interesting are such cyclised pro-drugs when the 1,2-diol is on a (1-4C)alkyl chain linked to a carbonyl group in a substituent of formula Rc borne by a nitrogen atom in (TC4). Esters of compounds of formula (I) wherein the HO- function/s in (PD1) and (PD2) are protected by (1-4C)alkyl, phenyl or benzyl are useful intermediates for the preparation of such pro-drugs.

Further in-vivo hydrolysable esters include phosphoramidic esters, and also compounds of formula (I) in which any free hydroxy group independently forms a phosphoryl (npd is 1) or phosphiryl (npd is 0) ester of the formula (PD3)), wherein npd is independently 0 or 1 for each oxo group :

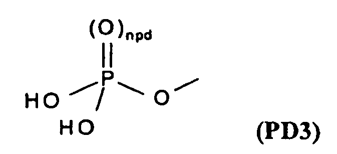

For the avoidance of doubt, phosphono is -P(O)(OH)₂; (1-4C)alkoxy(hydroxy)-phosphoryl is a mono-(1-4C)alkoxy derivative of -O-P(O)(OH)₂; and di-(1-4C)alkoxyphosphoryl is a di-(1-4C)alkoxy derivative of -O-P(O)(OH)₂.

Useful intermediates for the preparation of such esters include compounds containing a group/s of formula (PD3) in which either or both of the -OH groups in (**P**D3) is independently protected by (1-4C)alkyl (such compounds also being interesting compounds in their own right), phenyl or phenyl-(1-4C)alkyl (such phenyl groups being optionally substituted by 1 or 2 groups independently selected from (1-4C)alkyl, nitro, halo and (1-4C)alkoxy).

Thus, prodrugs containing groups such as (PD 1), (PD2) and (PD3) may be prepared by reaction of a compound of formula (I) containing suitable hydroxy group/s with a suitably protected phosphorylating agent (for example, containing a chloro or dialkylamino leaving group), followed by oxidation (if necessary) and deprotection. Prodrugs containing a group such as (PS1) may be obtained by analagous chemistry.

When a compound of formula (1) contains a number of free hydroxy group, those groups not being converted into a prodrug functionality may be protected (for example, using a t-butyl-dimethylsilyl group), and later deprotected. Also, enzymatic methods may be used to selectively phosphorylate or dephosphorylate alcohol functionalities.

Other interesting in-vivo hydrolysable esters include, for example, those in which Rc is defined by, for example, R¹⁴C(O)O(1-6C)alkyl-CO- (wherein R¹⁴ is for example, benzyloxy-(1-4C)alkyl, or phenyl). Suitable substituents on a phenyl group in such esters include, for example, 4-(1-4C)piperazino-(1-4C)alkyl, piperazino-(1-4C)alkyl and morpholino-(1-4C)alkyl.

Where pharmaceutically-acceptable salts of an in-vivo hydrolysable ester may be formed this is achieved by conventional techniques. Thus, for example, compounds containing a group of formula (PD1), (PD2) and/or (PD3) may ionise (partially or fully) to form salts with an appropriate number of counter-ions. Thus, by way of example, if an in-vivo hydrolysable ester prodrug of a compound of formula (I) contains two (PD3) groups, there are four HO-P- functionalities present in the overall molecule, each of which may form an appropriate salt (i.e. the overall molecule may form, for example, a mono-, di-, tri- or tetrasodium salt).

The compounds of the present invention have a chiral centre at the C-5 position of the isoxazoline ring. The pharmaceutically active enantiomer is of the formula (IA):

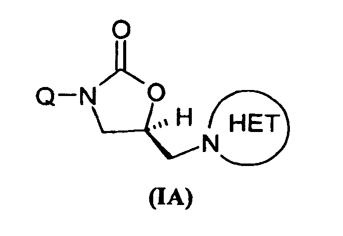

The present invention includes the pure enantiomer depicted above or mixtures of the 5R and 5S enantiomers, for example a racemic mixture. If a mixture of enantiomers is used, a larger amount (depending upon the ratio of the enantiomers) will be required to achieve the same effect as the same weight of the pharmaceutically active enantiomer. For example, the enantiomer depicted above is the 5(R) isomer when HET is 1,2,3- or 1,2,4-triazole or tetrazole.

Furthermore, some compounds of the formula (I) may have other chiral centres. It is to be understood that the invention encompasses all such optical and diastereo-isomers, and racemic mixtures, that possess antibacterial activity. It is well known in the art how to prepare optically-active forms (for example by resolution of the racemic form by recrystallisation techniques, by chiral synthesis, by enzymatic resolution, by biotransformation or by chromatographic separation) and how to determine antibacterial activity as described hereinafter.

Furthermore, some compounds of the formula (I) may exist as cis- and trans- isomers. It is to be understood that the invention encompasses all such isomers, and mixtures thereof, that possess antibacterial activity.

The invention relates to all tautomeric forms of the compounds of the formula (I) that possess antibacterial activity.

It is also to be understood that certain compounds of the formula (I) can exist in solvated as well as unsolvated forms such as, for example, hydrated forms. It is to be understood that the invention encompasses all such solvated forms which possess antibacterial activity.

It is also to be understood that certain compounds of the formula (I) may exhibit polymorphism, and that the invention encompasses all such forms which possess antibacterial activity.

As stated before, we have discovered a range of compounds that have good activity against a broad range of Gram-positive pathogens including organisms known to be resistant to most commonly used antibiotics, together with activity against fastidious Gram negative pathogens such as H.influenzae & M.catarrhalis. They have good physical and/or pharmacokinetic properties in general, and favourable toxicological profiles.

Particularly preferred compounds of the invention comprise a compound of formula (I), or a pharmaceutically-acceptable salt or an in-vivo hydrolysable ester thereof, wherein the substituents Q, HET, T and other substituents mentioned above have values disclosed hereinbefore, or any of the following values (which may be used where appropriate with any of the definitions and embodiments disclosed hereinbefore or hereinafter):

In one embodiment of the invention are provided compounds of formula (IB), or a pharmaceutically-acceptable salt, or in-vivo hydrolysable ester thereof, 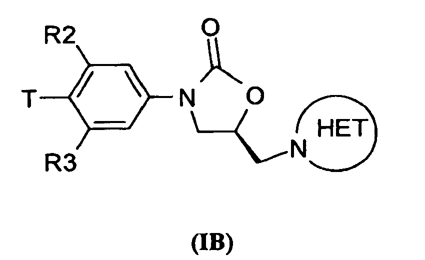 wherein HET is 1,2,3-triazole, 1,2,4-triazole or tetrazole, or HET is a di-hydro version of pyrimidine, pyridazine, pyrazine, 1,2,3-triazine, 1,2,4-triazine, 1,3,5-triazine and pyridine; R² and R³ are independently hydrogen or fluoro; and T is selected from (TAa1 to TAa6).

In another embodiment is provided a compound of (IB) or a pharmaceutically-acceptable salt, or in-vivo hydrolysable ester thereof, wherein wherein HET is 1,2,3-triazole, 1,2,4-triazole or tetrazole; R² and R³ are independently hydrogen or fluoro; and T is selected from (TAa1 & 2).

In another embodiment is provided a compound of (IB) or a pharmaceutically-acceptable salt, or in-vivo hydrolysable ester thereof, wherein HET is 1,2,3-triazol-1-yl, 1,2,4-triazol-1-yl or tetrazol-2-yl; R² and R³ are independently hydrogen or fluoro; and T is selected from (TAa 1 & 2).

In another embodiment is provided a compound of (IB) or a pharmaceutically-acceptable salt, or in-vivo hydrolysable ester thereof, wherein Rs is selected from fluoromethyl, difluoromethyl, trifluoromethyl, chloromethyl, bromomethyl, cyanomethyl, cyano, amino, azido, alkylthioalkyl such as methylthiomethyl and 2-propynyl.

In all of the above aspects and preferred compounds of formula (IB), in-vivo hydrolysable esters are preferred where appropriate, especially phosphoryl esters (as defined by formula (PD3) with npd as 1).

Particular compounds of the present invention include the following Examples, in particular Examples No. 1, and No. 3 and the individual (5R) isomers thereof.

### Process section :

In a further aspect the present invention provides a process for preparing a compound of formula (I) or a pharmaceutically-acceptable salt or an in-vivo hydrolysable ester thereof. It will be appreciated that during certain of the following processes certain substituents may require protection to prevent their undesired reaction. The skilled chemist will appreciate when such protection is required, and how such protecting groups may be put in place, and later removed.

For examples of protecting groups see one of the many general texts on the subject, for example, 'Protective Groups in Organic Synthesis' by Theodora Green (publisher: John Wiley & Sons).

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Thus, if reactants include, for example, groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t*-butyl group which may be removed, for example, by treatment with an acid, for example an organic acid such as trifluoroacetic acid, or for example a benzyl group which may be removed, for example, by hydrogenation over a catalyst such as palladium-on-carbon.

Resins may also be used as a protecting group.

The protecting groups may be removed at any convenient stage in the synthesis using conventional techniques well known in the chemical art.

A compound of the formula (I), or a pharmaceutically-acceptable salt or an in-vivo hydrolysable ester thereof, may be prepared by any process known to be applicable to the preparation of chemically-related compounds. Such processes, when used to prepare a compound of the formula (I), or a pharmaceutically-acceptable salt or an in-vivo hydrolysable ester thereof, are provided as a further feature of the invention and are illustrated by the following representative examples. Necessary starting materials may be obtained by standard procedures of organic chemistry (see, for example, Advanced Organic Chemistry (Wiley-Interscience), Jerry March). The preparation of such starting materials is described within the accompanying non-limiting Examples (in which, for example, 3,5-difluorophenyl, 3-fluorophenyl and (des-fluoro)phenyl containing intermediates may all be prepared by analagous procedures; or by alternative procedures - for example, the preparation of (T group)-(fluoro)phenyl intermediates by reaction of a (fluoro)phenylstannane with, for example, a pyran or (tetrahydro)pyridine compound, may also be prepared by anion chemistry (see, for example, WO97/30995).

Alternatively, necessary starting materials are obtainable by analogous procedures to those illustrated which are within the ordinary skill of an organic chemist. Information on the preparation of necessary starting materials or related compounds (which may be adapted to form necessary starting materials) may also be found in the following Patent and Application Publications, the contents of the relevant process sections of which are hereby incorporated herein by reference : WO99/02525; WO98/54161; WO97/37980; WO97/30981 (& US5,736,545); WO97/21708 (& US5,719,154); WO97/10223; WO97/09328; WO96/35691; WO96/23788; WO96/15130; WO96/13502; WO95/25106 (& US5,668,286); WO95/14684 (& US5,652,238); WO95/07271 (& US5,688,792); WO94/13649; WO94/01110; WO93/23384 (& US5,547,950 & US 5,700,799); WO93/09103 (& US5,565,571, US5,654,428, US5,654,435, US5,756,732 & US5,801,246); US5,231,188; US5,247,090; US5,523,403; WO97/27188; WO97/30995; WO97/31917; WO98/01447; WO98/01446; WO99/10342; WO99/10343; WO99/11642; WO99/64416; WO99/64417 and GB99/03299; European Patent Application Nos. 0,359,418 and 0,609,905; 0,693,491 A 1 (& US5,698,574); 0,694,543 A 1 (& AU 24985/95); 0,694,544 A1 (& CA 2,154,024); 0,697,412 A 1 (& US5,529,998); 0,738,726 A1 (& AU 50735/96); 0,785,201 A1 (& AU 10123/97); German Patent Application Nos. DE 195 14 313 A1 (& US5,529,998); DE 196 01 264 A1 (& AU 10098/97); DE 196 01 265 A 1 (& AU 10097/97); DE 196 04 223 A 1 (& AU 12516/97); DE 196 49 095 A 1 (& AU 12517/97).

The following Patent and Application Publications may also provide useful information and the contents of the relevant process sections are hereby incorporated herein by reference : FR 2458547; FR 2500450(& GB 2094299, GB 2141716 & US 4,476,136); DE 2923295 (& GB 2028306, GB 2054575, US4,287,351, US4,348,393, US4,413,001, US4,435,415 & US4,526,786), DE 3017499 (& GB 2053196, US4,346,102 & US4,372,967); US4,705,799; European Patent Application Nos. 0,312,000; 0,127,902; 0,184,170; 0,352,781; 0,316,594.

The skilled organic chemist will be able to use and adapt the information contained and referenced within the above references to obtain necessary starting materials. Thus, the present invention also provides that the compounds of the formulae (I) and pharmaceutically-acceptable salts and in-vivo hydrolysable esters thereof, can be prepared by a process (a) to (i) as follows (wherein the variables are as defined hereinbefore or after unless otherwise stated) :
**(a)** by modifying a substituent in or introducing a substituent into another compound of formula (I); such changes may be usefully made in many positions of compounds of formula (I), for instance a heterocyclyl group linked through nitrogen (optionally substituted on a carbon other than a carbon atom adjacent to the linking nitrogen ring atom) may be converted into another heterocyclyl group linked through nitrogen (optionally substituted on a carbon other than a carbon atom adjacent to the linking nitrogen ring atom) by introduction of a new ring substituent or by refunctionalisation of an existing ring substituent, for instance by modifying the 4-substituent of a 4-substituted 1,2,3-triazol-1-yl group; or for instance such changes may be usefully made in the group Q; for example an alkylthio group may be oxidised to an alkylsulfinyl or alkysulfonyl group, for instance a group R^{4h} that contains an alkylthio group may be oxidized to an alkylsulfinyl or alkylsulfonyl group or for example a group R^{4h} that contains an amino group may be converted into its acylamino derivative in the last step of the preparation of a compound of the formula (I);
   or
**(b)** by reaction of a compound of formula (II) : 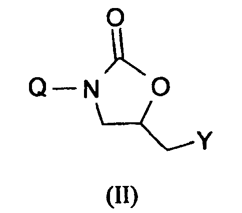 wherein Y is a displaceable group (which may be (i) generated in-situ, for example under Mitsunobu conditions, or (ii) preformed, such as chloro or mesylate) with a compound of the formula (III) :

   HET (III)

   wherein HET is HET-H free-base form or HET- anion formed from the free base form; or
**(c)** by reaction of a compound of the formula (IV) :

   Q-Z (IV)

   wherein Z is an isocyanate, amine or urethane group with an epoxide of the formula (V): 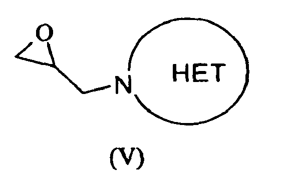 or
**(d)** by reaction of a compound of formula (VI) : 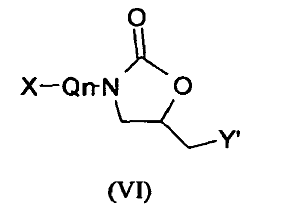 wherein Y' is a group HET as hereinabove defined, X is a replaceable substituent - such as chloride, bromide, iodide, trifluoromethylsulfonyloxy, trimethylstannyl, trialkoxysilyl, or a boronic acid residue - located at a position substituted by T in any of the aromatic embodiments Q1 - Q8 of Qn as hereinabove defined for Q, but with X in place of the substituent T, with a compound of the formula (VII) :

   T-X' (VII)

   wherein T-X' is a five-membered heterocycle with 1-3 heteroatoms drawn in combination from O, N, and S and X' is a replaceable C-linked substituent - such as chloride, bromide, iodide, trifluoromethylsulfonyloxy, trimethylstannyl, trialkoxysilyl, or a boronic acid residue;
   wherein the substituents X and X' are chosen to be complementary pairs of substituents known in the art to be suitable as complementary substrates for coupling reactions catalysed by transition metals such as palladium(0); or
**(e)** by reaction of a compound of formula (VIII): 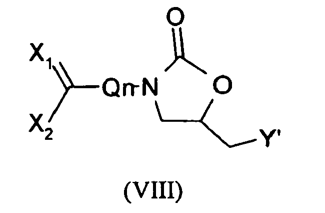 wherein Y' is a group HET as defined herein above and X1 and X2 here are independently optionally substituted heteroatoms drawn in combination from O, N, and S such that C(X1)X2 constitutes a substituent that is a carboxylic acid derivative substituent located at a position substituted by T in any of the aromatic embodiments Q1-Q10 of Qn as hereinabove defined for Q with a compound of the formula (IX) and X3 and X4 are independently optionally substituted heteroatoms drawn in combination from O, N, and S: 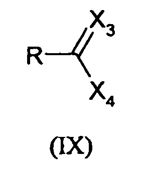 and wherein one of C(X1)X2 and C(X3)X4 constitutes an optionally substituted hydrazide, thiohydrazide, or amidrazone, and the other one of C(X1)X2 and C(X3)X4 constitutes an optionally substituted acylating, thioacylating, or imidoylating agent such that C(X1)X2 and C(X3)X4 may be condensed together to form a 5-membered heterocycle containing 3 heteroatoms drawn in combination from O, N, and S, for instance thiadiazole, by methods well-known in the art; or
**(f)** by reaction of a compound of formula (X): 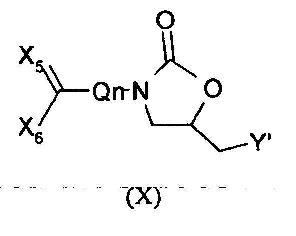 wherein Y' is a group HET as defined herein above and C(X5)X6 constitutes a substituent located at a position substituted by T in any of the aromatic embodiments Q1 - Q8 of Qn as hereinabove defined for Q with a compound of the formula (XI): 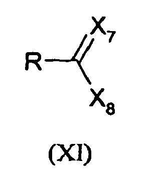 wherein one of C(X5)X6 and C(X7)X8 constitutes an optionally substituted alpha-(leaving-group-substituted)ketone, wherein the leaving group is for example a halo-group or an (alkyl or aryl)-sulfonyloxy-group, and the other one of C(X5)X6 and C(X7)X8 constitutes an optionally substituted amide, thioamide, or amidine, such that C(X5)X6 and C(X7)X8 are groups that may be condensed together to form a 5-membered heterocycle containing 2 heteroatoms drawn in combination from O, N, and S, for instance thiazole, by methods well-known in the art; or
**(g)** for HET as optionally substituted 1,2,3-triazoles, compounds of the formula (I) may be made by cycloaddition via the azide (wherein e.g. Y in (II) is azide) to acetylenes, or to acetylene equivalents such as optionally substituted cylcohexa-1,4-dienes or optionally substituted ethylenes bearing eliminatable substituents such as arylsulfonyl; or
**(h)** for HET as 4-substituted 1,2,3-triazole compounds of formula (I) may be made by reacting aminomethylisoxazolines with 1,1-dihaloketone sulfonylhydrazones;
**(i)** for HET as 4-substituted 1,2,3-triazole compounds of formula (I) may also be made by reacting azidomethyl isoxazolines with terminal alkynes using Cu(1) catalysis; and thereafter if necessary: (i) removing any protecting groups; (ii) forming a pharmaceutically-acceptable salt; (iii) forming an in-vivo hydrolysable ester.

(a) Methods for converting substituents into other substituents are known in the art by using standard chemistry (see for example, Comprehensive Organic Functional Group Transformations (Pergamon), Katritzky, Meth-Cohn & Rees); for example:
   a hydroxy group may be converted into a silyloxy group; an azido or an acylamino or thioacylamino group, for instance an acetamide group (optionally substituted or protected on the amido-nitrogen atom); into an acyloxy group, for instance an acetoxy group; a heterocyclylamino group (optionally substituted or protected on the amino-nitrogen atom), for instance an isoxazol-3-ylamino group or a 1,2,5-thiadiazol-3-ylamino group; a heterocyclyl group linked through nitrogen (optionally substituted on a carbon other than a carbon atom adjacent to the linking nitrogen ring atom), for instance an optionally substituted 1,2,3-triazol-1-yl group; or an amidino group, for instance an 1-(N-cyanoimino)ethylamino group; a hydroxy group may be alkylated to a methoxy group, a hydroxy group may be converted into a halo- group, or into a cyano- group; or into an alkylthio-, an arylthio- or a heteroarylthio-group (see, for example, Tet.Lett., 585, 1972); such conversions of the hydroxy group taking place directly (for instance by acylation or Mitsunobu reaction) or through the intermediacy of one or more derivatives (for instance a mesylate or an azide); moreover, a hydroxy-group may be oxidized to a carbonyl group including a carboxylic acid group.
   an acyloxy group may be converted into a hydroxy group or into the groups that may be obtained from a hydroxy group (either directly or through the intermediacy of a hydroxy group);
   a silyloxy group may be converted into a hydroxy group or into the groups that may be obtained from a hydroxy group (either directly or through the intermediacy of a hydroxy group);
   an acylamino group or thioacylamino group may be converted into another acylamino group or thioacylamino group; or into a heterocyclylamino group (optionally substituted or protected on the amino-nitrogen atom);
   a carboxylic acid group may be converted into an ester or an amide, an ester may be converted into a carboxylic acid or an amide, and an amide may be converted into an acid or a nitrile; a nitrile may be converted into a carboxylic acid or an amide or an imidate.
   an imidate or a nitrile may be converted into a wide range of 5 membered heterocycles such as tetrazoles or 1,2,4-triazoles;
   a carbonyl group can be reduced to a hydroxy group and a carboxylic acid group or a derivative thereof can be reduced to a carbonyl group or to a hydroxy group;
   a carbonyl group may also be converted into a CF₂ group and a carboxylic acid group may be converted into a CF₃ group;
   an alkylthio group may be oxidised to an alkylsulfinyl or alkysulfonyl group;
   a cyano group may be reduced to an amino group, a nitro group may be reduced to an amino group; a carbonyl group may be converted into a thiocarbonyl group (eg. using Lawsson's reagent) or a bromo group converted to an alkylthio group. It is possible in this way and in closely analogous ways using standard methods well known to skilled organic chemists to interconvert compounds of formula (I).
**(b)(i)** Reaction (b)(i) (in which Y is initially hydroxy) is performed under Mitsunobu conditions, for example, in the presence of tri-n-butylphosphine and diethyl azodicarboxylate (DEAD) in an organic solvent such as THF, and in the temperature range 0°C - 60°C, but preferably at ambient temperature. Details of Mitsunobu reactions are contained in Tet. Letts., 31, 699, (1990); The Mitsunobu Reaction, D.L.Hughes, Organic Reactions, 1992, Vol.42, 335-656 and Progress in the Mitsunobu Reaction, D.L.Hughes, Organic Preparations and Procedures International, 1996, Vol.28, 127-164.
**(b)(ii)** Reactions (b)(ii) are performed conveniently in the presence of a suitable base such as, for example, an alkali or alkaline earth metal carbonate, alkoxide or hydroxide, for example sodium carbonate or potassium carbonate, or, for example, an organic amine base such as, for example, pyridine, 2,6-lutidine, collidine, 4-dimethylaminopyridine, triethylamine, morpholine or diazabicyclo-[5.4.0]undec-7-ene, the reaction is also preferably carried out in a suitable inert solvent or diluent, for example methylene chloride, acetonitrile, tetrahydrofuran, 1,2-dimethoxyethane, *N,N* dimethylformamide, *N,N-*dimethylacetamide, *N*-methylpyrrolidin-2-one or dimethylsulfoxide at and at a temperature in the range 25-60°C.
   When Y is chloro, the compound of the formula (II) may be formed by reacting a compound of the formula (II) wherein Y is hydroxy (hydroxy compound) with a chlorinating agent. For example, by reacting the hydroxy compound with thionyl chloride, in a temperature range of ambient temperature to reflux, optionally in a chlorinated solvent such as dichloromethane or by reacting the hydroxy compound with carbon tetrachloride/triphenyl phosphine in dichloromethane, in a temperature range of 0°C to ambient temperature. A compound of the formula (II) wherein Y is chloro or iodo may also be prepared from a compound of the formula (II) wherein Y is mesylate or tosylate, by reacting the latter compound with lithium chloride or lithium iodide and crown ether, in a suitable organic solvent such as THF, in a temperature range of ambient temperature to reflux
   When Y is (1-4C)alkanesulfonyloxy or tosylate the compound (II) may be prepared by reacting the hydroxy compound with (1-4C)alkanesulfonyl chloride or tosyl chloride in the presence of a mild base such as triethylamine or pyridine.
   When Y is a phosphoryl ester (such as PhO₂-P(O)-O-) or Ph₂-P(O)-O- the compound (II) may be prepared from the hydroxy compound under standard conditions.
   If not commercially available, compounds of the formula (III) may be prepared by procedures which are selected from standard chemical techniques, techniques which are analogous to the synthesis of known, structurally similar compounds, or techniques which are analogous to the procedures described in the Examples. For example, standard chemical techniques are as described in Houben Weyl.
**(c)** by reaction of Q-Z wherein Z is an amine, urethane, or isocyanate with an N-epoxypropyl hetercycle (V). Epoxides of the formula (V) may be prepared from the corresponding N-allylheterocycle of formula (XII): 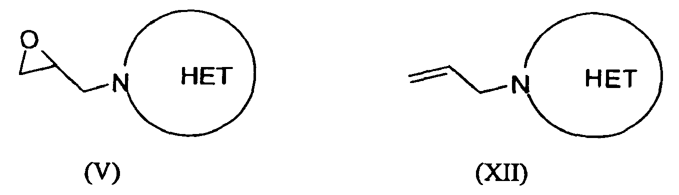
   Certain such epoxide and alkene intermediates are novel and are provided as a further feature of the invention. Asymmetric epoxidation may be used to give the desired optical isomer. Furthermore, a similar reaction to reaction (c) may be performed in which Q-Z (wherein Z is a amine group) is reacted with the epoxide (V) (optionally in the presence of an organic base), and the product is reacted with, for example, phosgene to form the oxazolidinone ring.
   Alternatively, a precursor of the group HET may be incorporated in place of the group HET in the epoxide of formula (V).
   Variations on this process in which the oxirane is replaced by an equivalent reagent X-CH₂CH(O-optionally protected)CH₂HET where X is a displaceable group are also well known in the art.
   Such reactions and the preparation of starting materials in within the skill of the ordinary chemist with reference to the above-cited documents disclosing analogous reactions and preparations.
   In particular, compounds of the formula (II), and (IV) may be prepared by the skilled man, for example as described in International Patent Application Publication Nos. cited herein, and by analogous processes.
   Compounds of the formula (II) wherein Y is hydroxy may be obtained as described in the references cited herein, for example, by reacting a compound Q-Z (IV) where Z is an amine, an isocyanate, or a urethane, especially a compound of the formula (IV, Z = NHCO₂R²¹) with a compound of formula (XIII): 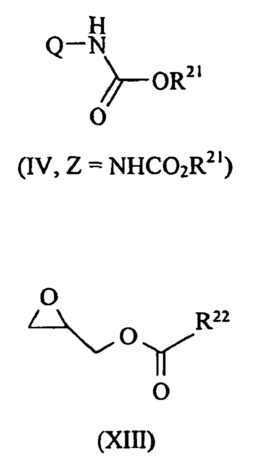 wherein R²¹ is (1-6C)alkyl or benzyl and R²² is (1-4C)alkyl or -S(O)ₙ(1-4C)alkyl where n is 0, 1 or 2. Preferably R²² is (1-4C)alkyl. Compounds of the formula (II), (IV), and (XIII) may be prepared by the skilled man, for example as described in International Patent Application Publication Nos. cited herein, the contents of which are hereby incorporated by reference, and by analogous processes.
   Compounds of the formula Q-Z wherein Z is a urethane may be prepared by the skilled chemist, for example by analogous processes to those described in International Patent Application Publication Nos. WO 97/30995 and WO 97/37980. Compounds of the formula Q-Z wherein Z is an isocyanate may be prepared by the skilled chemist, for example by analogous processes to those described in Walter A. Gregory et al in J. Med. Chem. 1990, 33, 2569-2578 and Chung-Ho Park et al in J. Med. Chem. 1992, 35, 1156-1165.
**(d)** Compounds of formula (II) wherein Y is is HET as hereinabove defined or Y is a group that may be converted to HET, such as hydroxy, may be obtained by coupling together two appropriately substituted fragments to form a carbon-carbon bond in the place of the two substituents X and X' of (VI) and (VII) respectively. X and X' may be selected from substituents such as chloro, bromo, iodo, trifluoromethanesulfonyloxy, trialkylstannyl, trialkoxysilyl, or a boronic acid residue provided that the selected substituents X and X' form a pair of complementary substituents known in the art to be suitable pairs of substituents for transition metal mediated reactions. For instance one of X and X' prime may be chloro and the other may be trimethylstannyl, as shown in the Scheme.
   If not commercially available, the X and X' substituted fragments used as coupling partners in the transition metal mediated coupling reaction may be prepared by procedures that are selected from standard chemical techniques, techniques that are analogous to the synthesis of known, structurally similar compounds, or techniques which are analogous to the procedures described in the Examples. For example, standard chemical techniques are described in Houben Weyl, Methoden der Organischen Chemie.
   The chemistry of process **(e)** may also be utilised to prepare compounds of formula (II) wherein Y is hydroxy or a group that may be converted into a HET ring, and then process **(b)** or other suitable chemistry used to prepare compounds of formula (I).
**(e)** The reaction between thioacylhydrazides and acylating agents to form intermediate acylthioacylhydrazides and subsequently 1,3,4-thiadiazoles is performed under conventional conditions, for instance as indicated in Comprehensive Heterocyclic Chemistry. Suitable acylating agents include acid chlorides and anhydrides. Either of the reaction components may constitute the thioacylhydrazide and the other constitutes the acylating agent, according to synthetic convenience.
   The intermediate acylthioacylhydrazides may also be converted into 1,3,4-oxadiazoles. Carboxylic acid derivatives other than thioacylhydrazide are also well known in the art to be substrates for similar reactions leading to other C-linked 5-membered heterocycles containing 3 heteroatoms. For instance, acylhydrazides and amidrazones may be used in place of thioacylhydrazides and thioacylataing agents such as methyl dithioacetate and imidolyating agents such as ethyl acetimidate may be used in place of acylating agents. The products of such reactions are well known in the art. Thus, acylation of acylhydrazides and cyclisation of the resultant diacylhydrazides is known to give 1,3,4-oxadiazoles and acylation amidrazones and cyclisation of the resultant acylamidrazones is known to give 1,2,4-triazoles. Such reactions are described in the literature as indicated in e.g. Comprehensive Heterocyclic Chemistry and are incorporated by reference into the process of method (e). It is further well known that these reactions, for instance the reaction between a (thio)acylating agent and a (thio)hydrazide proceed stepwise to give a cyclic product and that the reactions proceed through intermediates that can themselves be isolated under appropriate reaction conditions. Accordingly this method also includes a process in which an isolable intermediate for instance as di(thio)acylhydrazide is converted into a compound of formula (I) under the conditions already well known for reactions of this type. Moreover, an intermediate in this reaction may be produced under conditions where the cyclization to a compound of formula (I) proceeds spontaneously and without isolation of the intermediate compound, for instance in the conversion of a diacylhydrazide into a thiadiazole under conditions where monothio or dithio diacylhydrazides are formed from the diacylhydrazide and for example Lawesson's reagent. Accordingly this method also includes a process in which an intermediate of the process (e) is formed and spontaneously consumed to give a compound of formula (I) under the conditions already well known for reactions of process (e).
   If not commercially available, compounds of the formula (VIII) and (IX) may be prepared by procedures which are selected from standard chemical techniques, techniques which are analogous to the synthesis of known, structurally similar compounds, or techniques which are analogous to the procedures described in the Examples. For example, standard chemical techniques are as described in Houben Weyl.
   Certain such thioacylhydrazide and acylating intermediates are novel and are provided as a further feature of the invention (R, Y and Y' suitable to give compounds of formula (I)). 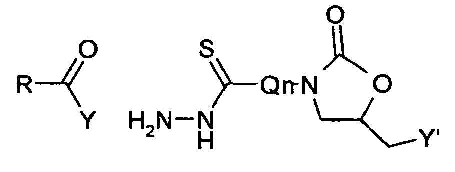 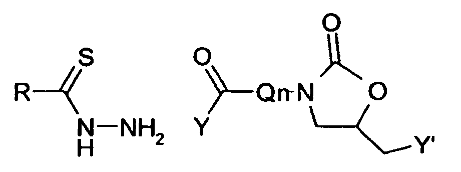
**(f)** The reaction between thioamides and alpha-haloketones to form thiazoles is performed under conventional conditions, for instance as indicated in Comprehensive Heterocyclic Chemistry , The Chemistry of the Thioamide Group, or Thiazoles (Heterocyclic Chemistry, Weisberger). Suitable halogens include bromine and chlorine. It is well known that other leaving groups may be suitable alternatives to the halogen of an alpha-haloketone, for instance methansulfonyloxy- or hydroxy-ketones or diazoketones may also be used in reaction (f). The regioisomer of thiazole obtained by this method depends on which reaction partner constitutes the thioamide and which reaction partner constitutes the alpha-(leaving-group)substituted-ketone. In the reaction shown in the Scheme the thiazole produced is C2-linked. Carboxylic acid derivatives other than thioamide are also well known in the art to be substates for similar reactions leading to other C-linked 5-membered heterocycles. Such analogous reactions are described in e.g. Comprehensive Heterocyclic Chemistry and are incorporated by reference into the process of method (f). It is further well known that the reaction between an alpha-(leaving-group)substituted-ketone and a thioamide proceeds stepwise to give a 4-hydroxy-4,5-dihydrothiazole as one of the intermediates and that this intermediate can be isolated under appropriate reaction conditions. Accordingly this method also includes a process in which an isolated 4-hydroxy-4,5-dihydrothiazole is converted into a compound of formula (I) under dehydrating conditions already well known for reactions of this type.
   If not commercially available, compounds of the formula (X) and (XI) may be prepared by procedures which are selected from standard chemical techniques, techniques which are analogous to the synthesis of known, structurally similar compounds, or techniques which are analogous to the procedures described in the Examples. For example, standard chemical techniques are as described in Houben Weyl. Certain such thioamide and haloketone intermediates are novel and are provided as a further feature of the invention. 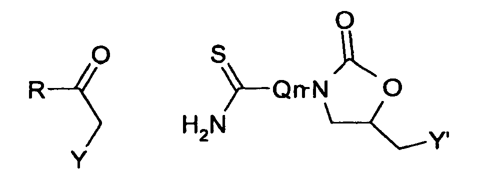 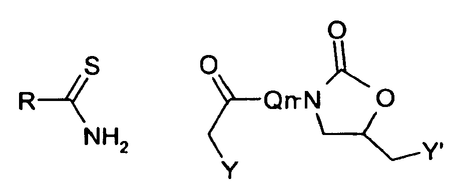
**(g)** The cycloaddition reaction to form 1,2,3 triazoles from the corresponding azide is performed under conventional conditions.. The reaction may use acetylenes or equivalent synthons that react as olefins and then eliminate the elements of a molecule to regenerate a double bond between the carbon atoms of the original olefin. Suitable olefins or their close analogues, which include those able to eliminate the elements of cyclopentadiene, of optionally substituted naphthalenes, of secondary amines, or of sulfinic or sulfenic acids, have been described in the litereature as equivalents to or as synthons for alkynes. The method is illustrated in the Schemes.
**(g)** 4-Substituted 1,2,3-triazoles may be constructed from a primary amino compound according to the method of Sakai *et al.* by reacting it with sulfonylhydrazones of 1,1-dihalomethylketones. (see for example Sakai et al., *Bull. Chem. Soc. Japan,* **1985,** *59*, 179); as illustrated in the Schemes;
**(i)** 4-Substituted 1,2,3-triazoles may be constructed from terminal alkynes in a mild and regioselective reaction according to the method of Sharpless.(see V.V. Rostov, L.G. Green, V.V. Folkin, and K.B. Sharpless, *Angew. Chem. Int. Ed.,* **2002**, *41*, 2596); ); as illustrated in the Schemes; The preparation of suitable alkynes or their close analogues from simpler commercially available acetylenes such as acetylene itself or trimethylsilylacetylene is well-known in the chemical literature;

Compounds of the formula (II) wherein Y is azide may be obtained as described in the references cited herein (particularly in the section proceeding the discussion of protecting groups), for example from the corresponding compounds in which Y is hydroxy or mesylate.

The main synthetic routes are illustrated in the Schemes below (with Q as phenyl, and X, R and A defined with reference to analogous substituents defined elsewhere herein). The compounds of the invention may be prepared by analogous chemistry adapted from the Schemes. The Schemes also show the preparation of 1,2,3-triazoles via the azide (prepared from the relevant hydroxy compound).

The Schemes may be genericised by the skilled man to apply to compounds within the present specification which are not specifically illustrated in the Schemes (for example to HET as a 6-membered ring as defined herein).

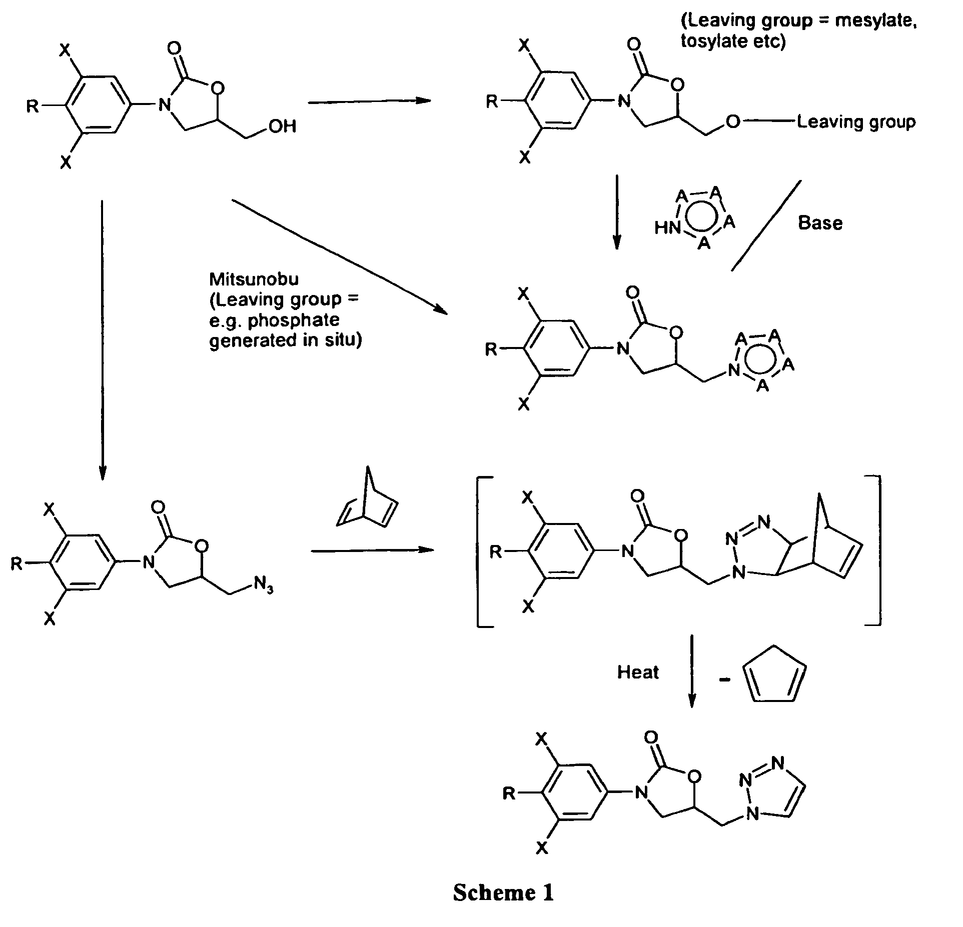

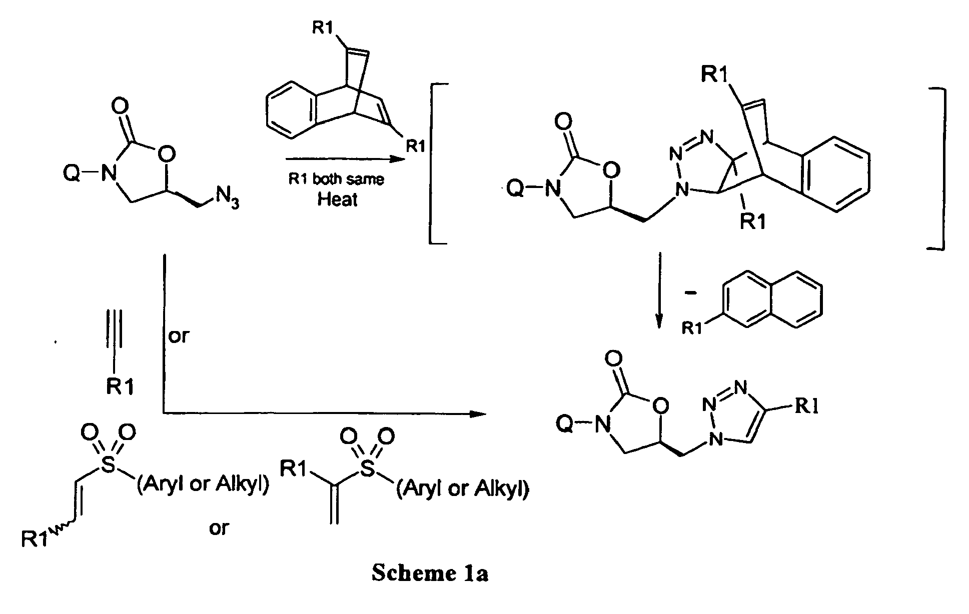

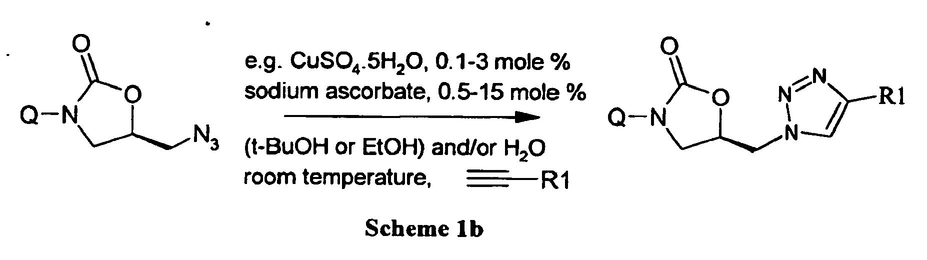

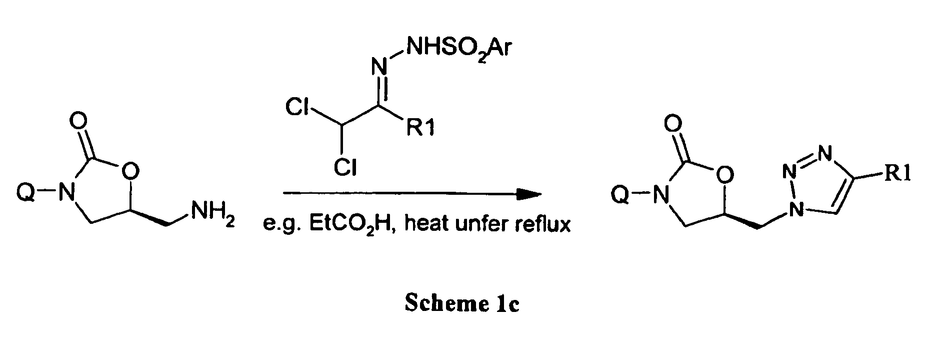

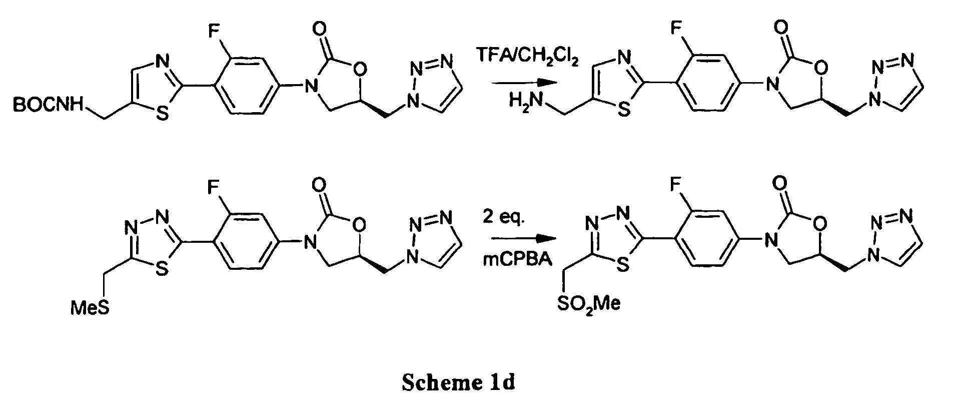

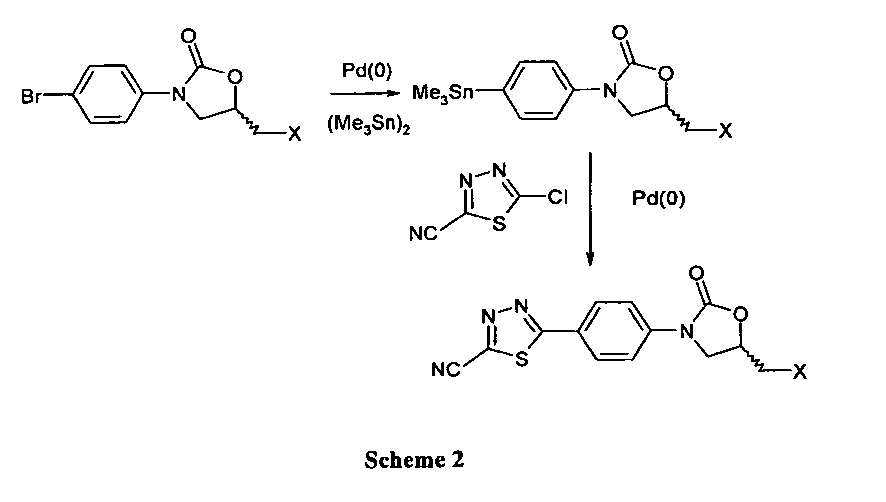

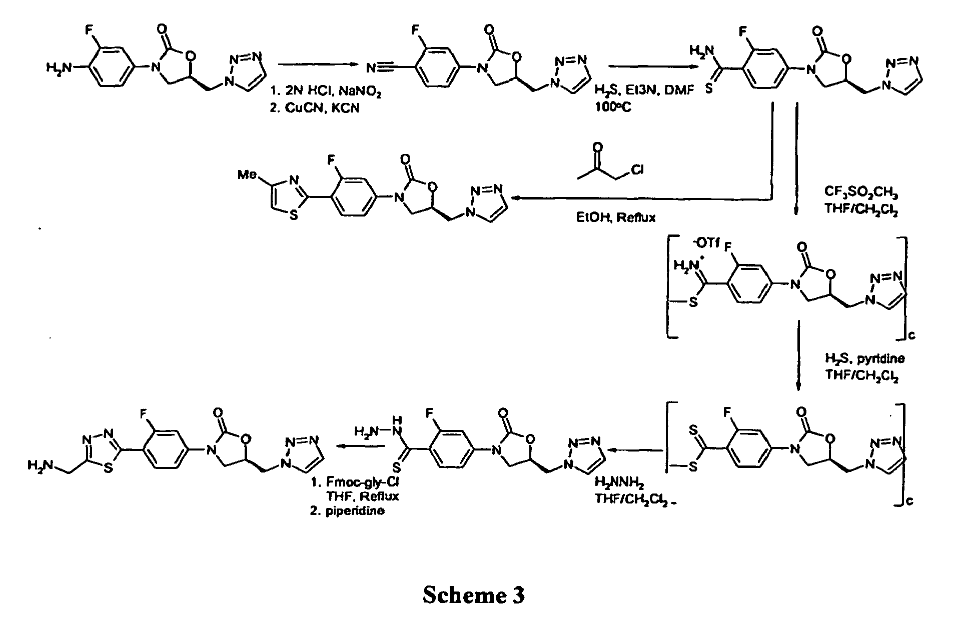

Deprotection, salt formation or in-vivo hydrolysable ester formation may each be provided as a specific final process step.

The N-linked hetereocycle can of course be prepared early in the overall synthesis, and then other functional groups changed.

Where Y is a displaceable group, suitable values for Y are for example, a halogeno or sulfonyloxy group, for example a chloro, bromo, methanesulfonyloxy or toluene-4-sulfonyloxy group.

General guidance on reaction conditions and reagents may be obtained in Advanced Organic Chemistry, 4th Edition, Jerry March (publisher : J.Wiley & Sons), 1992. Necessary starting materials may be obtained by standard procedures of organic chemistry, such as described in this process section, in the Examples section or by analogous procedures within the ordinary skill of an organic chemist. Certain references are also provided which describe the preparation of certain suitable starting materials, for example International Patent Application Publication No. WO 97/37980, the contents of which are incorporated here by reference. Processes analogous to those described in the references may also be used by the ordinary organic chemist to obtain necessary starting materials.

The removal of any protecting groups, the formation of a pharmaceutically-acceptable salt and/or the formation of an in-vivo hydrolysable ester are within the skill of an ordinary organic chemist using standard techniques. Furthermore, details on the these steps, for example the preparation of in-vivo hydrolysable ester prodrugs has been provided in the section above on such esters, and in certain of the following non-limiting Examples.

When an optically active form of a compound of the formula (I) is required, it may be obtained by carrying out one of the above procedures using an optically active starting material (formed, for example, by asymmetric induction of a suitable reaction step), or by resolution of a racemic form of the compound or intermediate using a standard procedure, or by chromatographic separation of diastereoisomers (when produced). Enzymatic techniques may also be useful for the preparation of optically active compounds and/or intermediates.

Similarly, when a pure regioisomer of a compound of the formula (I) is required, it may be obtained by carrying out one of the above procedures using a pure regioisomer as a starting material, or by resolution of a mixture of the regioisomers or intermediates using a standard procedure.

According to a further feature of the invention there is provided a compound of the formula (I), or a pharmaceutically-acceptable salt, or in-vivo hydrolysable ester thereof for use in a method of treatment of the human or animal body by therapy.

According to a further feature of the present invention there is provided a method for producing an antibacterial effect in a warm blooded animal, such as man, in need of such treatment, which comprises administering to said animal an effective amount of a compound of the present invention, or a pharmaceutically-acceptable salt, or in-vivo hydrolysable ester thereof.

The invention also provides a compound of the formula (I), or a pharmaceutically-acceptable salt, or in-vivo hydrolysable ester thereof, for use as a medicament; and the use of a compound of the formula (I) of the present invention, or a pharmaceutically-acceptable salt, or in-vivo hydrolysable ester thereof, in the manufacture of a medicament for use in the production of an antibacterial effect in a warm blooded animal, such as man.

In order to use a compound of the formula (I), an in-vivo hydrolysable ester or a pharmaceutically-acceptable salt thereof, including a pharmaceutically-acceptable salt of an in-vivo hydrolysable ester, (hereinafter in this section relating to pharmaceutical composition "a compound of this invention") for the therapeutic (including prophylactic) treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula (I), an in-vivo hydrolysable ester or a pharmaceutically-acceptable salt thereof, including a pharmaceutically-acceptable salt of an in-vivo hydrolysable ester, and a pharmaceutically-acceptable diluent or carrier.

The pharmaceutical compositions of this invention may be administered in standard manner for the disease condition that it is desired to treat, for example by oral, rectal or parenteral administration. For these purposes the compounds of this invention may be formulated by means known in the art into the form of, for example, tablets, capsules, aqueous or oily solutions or suspensions, (lipid) emulsions, dispersible powders, suppositories, ointments, creams, aerosols (or sprays), drops and sterile injectable aqueous or oily solutions or suspensions.

In addition to the compounds of the present invention the pharmaceutical composition of this invention may also contain or be co-administered (simultaneously, sequentially or separately) with one or more known drugs selected from other clinically useful antibacterial agents (for example, β-lactams or aminoglycosides) and/or other anti-infective agents (for example, an antifungal triazole or amphotericin). These may include carbapenems, for example meropenem or imipenem, to broaden the therapeutic effectiveness. Compounds of this invention may also contain or be co-administered with bactericidal/permeability-increasing protein (BPI) products or efflux pump inhibitors to improve activity against gram negative bacteria and bacteria resistant to antimicrobial agents.

A suitable pharmaceutical composition of this invention is one suitable for oral administration in unit dosage form, for example a tablet or capsule which contains between 1mg and 1g of a compound of this invention, preferably between 100mg and 1g of a compound. Especially preferred is a tablet or capsule which contains between 50mg and 800mg of a compound of this invention, particularly in the range 100mg to 500mg.

In another aspect a pharmaceutical composition of the invention is one suitable for intravenous, subcutaneous or intramuscular injection, for example an injection which contains between 0.1 % w/v and 50% w/v (between 1 mg/ml and 500mg/ml) of a compound of this invention.

Each patient may receive, for example, a daily intravenous, subcutaneous or intramuscular dose of 0.5 mgkg⁻¹ to 20 mgkg⁻¹ of a compound of this invention, the composition being administered 1 to 4 times per day. In another embodiment a daily dose of 5 mgkg⁻¹ to 20 mgkg⁻¹ of a compound of this invention is administered. The intravenous, subcutaneous and intramuscular dose may be given by means of a bolus injection. Alternatively the intravenous dose may be given by continuous infusion over a period of time. Alternatively each patient may receive a daily oral dose which may be approximately equivalent to the daily parenteral dose, the composition being administered I to 4 times per day.

A pharmaceutical composition to be dosed intravenously may contain advantageously (for example to enhance stability) a suitable bactericide, antioxidant or reducing agent, or a suitable sequestering agent.

In the above other, pharmaceutical composition, process, method, use and medicament manufacture features, the alternative and preferred embodiments of the compounds of the invention described herein also apply.

### Antibacterial Activity :

The pharmaceutically-acceptable compounds of the present invention are useful antibacterial agents having a good spectrum of activity in-vitro against standard Gram-positive organisms, which are used to screen for activity against pathogenic bacteria. Notably, the pharmaceutically-acceptable compounds of the present invention show activity against enterococci, pneumococci and methicillin resistant strains of S.aureus and coagulase negative staphylococci, together with haemophilus and moraxella strains. The antibacterial spectrum and potency of a particular compound may be determined in a standard test system.

The (antibacterial) properties of the compounds of the invention may also be demonstrated and assessed in-vivo in conventional tests, for example by oral and/or intravenous dosing of a compound to a warm-blooded mammal using standard techniques.

The following results were obtained on a standard in-vitro test system. The activity is described in terms of the minimum inhibitory concentration (MIC) determined by the agar-dilution technique with an inoculum size of 10⁴ CFU/spot. Typically, compounds are active in the range 0.01 to 256 µg/ml.

Staphylococci were tested on agar, using an inoculum of 10⁴ CFU/spot and an incubation temperature of 37°C for 24 hours - standard test conditions for the expression of methicillin resistance.

Streptococci and enterococci were tested on agar supplemented with 5% defibrinated horse blood, an inoculum of 10⁴ CFU/spot and an incubation temperature of 37°C in an atmosphere of 5% carbon dioxide for 48 hours - blood is required for the growth of some of the test organisms. Fastidious Gram negative organisms were tested in Mueller-Hinton broth, supplemented with hemin and NAD, grown aerobically for 24 hours at 37°C, and with an innoculum of 5x10⁴ CFU/well.

For example, the following results were obtained for the compound of Example 1:

| Organism | | MIC (µg/ml) |
|---|---|---|
| Staphylococcus aureus: | MSQS | 0.125 |
| | MRQR | 0.25 |
| Streptococcus pneumoniae | | 0.125 |
| Streptococcus pyogenes | | 0.125 |
| i Haemophilus influenzae | | 2 |
| Moraxella catarrhalis | | 0.5 |

| | | |
|---|---|---|
| MSQS = methicillin sensitive and quinolone sensitive | | |
| MRQR = methicillin resistant and quinolone resistant | | |

Certain intermediates and/or Reference Examples described hereinafter within the scope of the invention may also possess useful activity, and are provided as a further feature of the invention.

The invention is now illustrated but not limited by the following Examples in which unless otherwise stated :-
(i) evaporations were carried out by rotary evaporation in vacuo and work-up procedures were carried out after removal of residual solids by filtration;
(ii) operations were carried out at ambient temperature, that is typically in the range 18-26°C and without exclusion of air unless otherwise stated, or unless the skilled person would otherwise work under an inert atmosphere;
(iii) column chromatography (by the flash procedure) was used to purify compounds and was performed on Merck Kieselgel silica (Art. 9385) unless otherwise stated;
(iv) yields are given for illustration only and are not necessarily the maximum attainable;
(v) the structure of the end-products of the invention were generally confirmed by NMR and mass spectral techniques [proton magnetic resonance spectra were generally determined in DMSO-d₆ unless otherwise stated using a Varian Gemini 2000 spectrometer operating at a field strength of 300 MHz, or a Bruker AM250 spectrometer operating at a field strength of 250 MHz; chemical shifts are reported in parts per million downfield from tetramethysilane as an internal standard (δ scale) and peak multiplicities are shown thus: s, singlet; d, doublet; AB or dd, doublet of doublets; dt, doublet of triplets; dm, doublet of multiplets; t, triplet, m, multiplet; br, broad; fast-atom bombardment (FAB) mass spectral data were generally obtained using a Platform spectrometer (supplied by Micromass) run in electrospray and, where appropriate, either positive ion data or negative ion data were collected];
(vi) each intermediate was purified to the standard required for the subsequent stage and was characterised in sufficient detail to confirm that the assigned structure was correct; purity was assessed by HPLC, TLC, or NMR and identity was determined by infra-red spectroscopy (IR), mass spectroscopy or NMR spectroscopy as appropriate;
(vii) in which the following abbreviations may be used :-
   DMF is N,N-dimethylformamide; DMA is N,N-dimethylacetamide; TLC is thin layer chromatography; HPLC is high pressure liquid chromatography; MPLC is medium pressure liquid chromatography; DMSO is dimethylsulfoxide; CDCl₃ is deuterated chloroform; MS is mass spectroscopy; ESP is electrospray; EI is electron impact; CI is chemical ionisation; EtOAc is ethyl acetate; MeOH is methanol.

Each of the following Examples comprises an independent aspect of the invention.

### Example 1: (5R)-3-(3-Fluoro-4-(5-cyano-1,3,4-thiadiazol-2-yl)phenyl)-5-(1H-1,2,3-triazol-1-ylmethyl)-1,3-oxazolidin-2-one

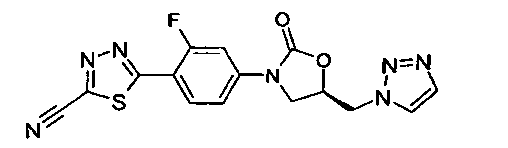

A mixture of (*5R*)-3-(3-fluoro-4-(trimethylstannyl)phenyl)-5-(1*H*-1,2,3-triazol-1-ylmethyl)-1,3-oxazolidin-2-one (442mg, 1.0 mmol), 5-chloro-1,3,4-thiadiazole-2-carbonitrile (151 mg, 1.0 mmol), and triphenylarsine (32 mg, 0.1 mmol) in *N*-methyl-2-pyrrolidinone (5 ml) under an atmosphere of nitrogen was treated with tris(dibenzylideneacetone)dipalladium(0) (48 mg, 0.05 mmol) and then stirred under an atmosphere of nitrogen for 14 hours at 75°C. The solvent was removed under reduced pressure. A solution of the involatile dark oily residue in ethyl acetate (10 ml) was treated with an aqueous solution of potassium fluoride (2M; 10 ml). The mixture was vortexed for two minutes and then stirred for 15 minutes. The mixture was extracted with ethyl acetate (150 ml) and the extract was dried (MgSO₄). The dried extract was filtered, the filtrate was concentrated under reduced pressure, and the residue was purified by chromatography on silica gel (20 g) (dichloromethane to 2% methanol in dichloromethane gradient) to give the desired product (199 mg). MS (ESP) 372.06 (MH⁺) for C₁₅H₁₀FN₇O₂S. ¹H-NMR (DMSO-d₆) δ: 3.93 (dd, 1H); 4.27 (t, 1H); 4.86 (d, 2H); 5.16 (m, 1H); 7.27 (dd, 1H); 7.44 (m, 1H); 6.98 (t, 1H); 7.78 (s, 1H); 8.16 (s, 1H).

The intermediate for this compound was prepared as follows:

### (5R)-5-Azidomethyl-3-(3-fluoro-4-iodophenyl)-1,3-oxazolidin-2-one

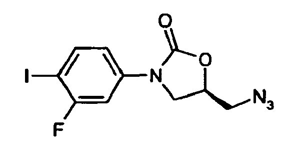

Methanesulfonyl chloride (17.9 ml) was added dropwise to a stirred solution of (5R)-3-(3-fluoro-4-iodophenyl)-5-hydroxymethyl-1,3-oxazolidin-2-one (55.8 g) and triethylamine (46.1 ml) in dry dichloromethane (800 ml) under an atmosphere of dry nitrogen and maintained below room temperature by an ice-bath. The stirred reaction mixture was allowed to warm to room temperature during 3 hours and then washed sequentially with water and brine and then dried (Na₂SO₄). Solvent was removed under reduced pressure to give the intermediate mesylate as a yellow solid (68 g) that was used without further purification.

A stirred solution in DMF (800 ml) of a mixture of the intermediate mesylate (68 g) and sodium azide (32.3 g) was heated at 75°C overnight. The mixture was allowed to cool to room temperature, diluted with water, and extracted twice with ethyl acetate. The combined extracts were washed sequentially with water and brine, and then dried (Na₂SO₄). Solvent was removed under reduced pressure to give a yellow oil that was purified by column chromatography on silica-gel. Elution with ethyl acetate-hexanes (1:1) gave the product azide as an off-white solid (49 g). The product could be further purified by trituration with ethyl acetate/hexanes.
¹H-NMR (DMSO-d₆) δ: 3.57-3.64 (dd, 1H); 3.70-3.77 (dd, 1H); 3.81-3.87 (dd, 1H); 4.06 (t, 1H); 4.78-4.84 (m, 1H); 7.05-7.09 (ddd, 1H); 7.45 (dd, 1H); 7.68-7.74 (dd, 1H).

### (5R)-3-(3-Fluoro-4-iodophenyl)-5-(1H-1.2.3-triazol-1-ylmethyl)-1.3-oxazolidin-2-one

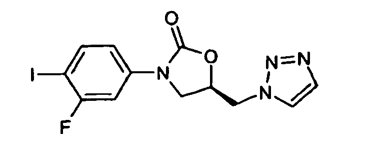

A stirred solution in dioxan (300 ml) of a mixture of the (5R)-5-azidomethyl-3-(3-fluoro-4-iodophenyl)-1,3-oxazolidin-2-one (30 g) and bicyclo[2.2.1]heptadiene (30 ml) was heated under reflux overnight. The mixture was allowed to cool to room temperature and then evaporated to dryness under reduced pressure to give a brown solid. The brown solid was purified by column chromatography on silica-gel. Elution with methanol:chloroform (98:2 to 95:5) gave the product triazole as a pale yellow solid (20 g). The product could be further purified by trituration with dichloromethane/hexanes (1:1) to give an off- white solid.
¹H-NMR (DMSO-d₆) δ: 3.86-3.92 (dd, 1H); 4.23 (t, 1H); 4.83 (d, 2H); 5.11-5.19 (m, 1H); 7.12-7.16 (dd, 1H); 7.47-7.51 (dd, 1H); 7.76 (s, 1H); 7.79-7.85 (dd, 1H); 8.16 (s, 1H).

### (5R)-3-[3-Fluoro-4-(trimethylstannyl)phenyl]-5-(1H-1,2,3-triazol-1-ylmethyl)-1,3-oxazolidin-2-one

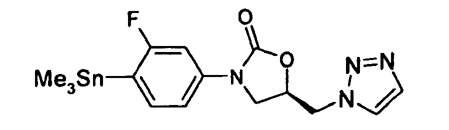

A mixture of (5*R*)-3-(3-fluoro-4-iodophenyl)-5-(1*H*-1,2,3-triazol-1-ylmethyl)-1,3-oxazolidin-2-one (5.39 g, 13.9 mmol) and hexamethylditin (5 g, 15.3 mmol) in dioxane (50 ml) under an atmosphere of nitrogen was treated with dichlorobis(triphenylphoshine)palladium (II) (487 mg, 0.69 mmol) and then stirred at 90°C under an atmosphere of nitrogen for 90 minutes. Silica gel (5 g) was added then the solvent removed under reduced pressure. The residual powder was placed on top of a silica gel column (100 g) and eluted (1% methanol in dichloromethane to 2.5% methanol in dichloromethane gradient) to give the desired product (4.545 g).
MS (ESP) 423, 425, 427 (MH⁺) for C₁₅H₁₉FN₄O₂Sn.
¹H-NMR (DMSO-d₆) δ: 0.32 (s, 9H); 3.90 (dd, 1H); 4.25 (t, 1H); 4.85 (d, 2H); 5.16 (m, 1H); 7.26 (dd, 1H); 7.33 (dd, 1H); 7.41 (dd, 1H); 7.78 (s, 1H); 8.18 (s, 1H).

The preparation of 5-chloro-[1,3,4]thiadiazole-2-carbonitrile is described in
- Gadwood, Robert C.; Barbachyn, Michael Robert; Toops, Dana Scott; Smith, Herman Walden; Vaillancourt, Valerie Ann. Preparation of azolylpiperazinylphenyloxazolidinones as antimicrobials. U.S. (1998), 34 pp. CODEN: USXXAM US 5736545 A 19980407 CAN 128:270612 AN 1998:219349 CAPLUS
- Gadwood, Robert C.; Barbachyn, Michael R.; Toops, Dana S.; Smith, Herman W.; Vaillancourt, Valerie A. Preparation of 3-[4-(4-azolyl-1-piperazinyl)phenyl]oxazolidin-2-ones as bactericides. PCT Int. Appl. (1997), 79 pp. CODEN: PIXXD2 WO 9730981 A1 19970828 CAN 127:278210 AN 1997:579705 CAPLUS

### Example 2: (5R)-3-(3-Fluoro-4-(5-ethoxycarbonyl-1,3,4-thiadiazol-2-yl)phenyl)-5-(1H-1,2,3-triazol-1-ylmethyl)-1,3-oxazolidin-2-one

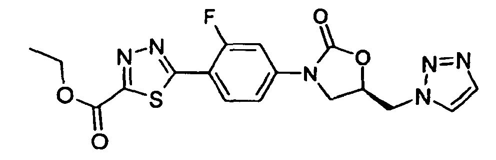

A mixture of (*5R*)-3-[3-fluoro-4-(trimethylstannyl)phenyl]-5-(1*H*-1,2,3-triazol-1-ylmethyl)-1,3-oxazolidin-2-one (436 mg, 1.0 mmol), ethyl 5-chloro-1,3,4-thiadiazole-2-carboxylate (197 mg, 0.9 mmol), and tris(2-furyl)phosphine (24 mg, 0.1 mmol) in tetrahydrofuran (5 ml) under an atmosphere of nitrogen was treated with tris(dibenzylideneacetone)dipalladium(0) (47 mg, 0.05 mmol). The mixture was stirred under an atmosphere of nitrogen for 14 hours at 75°C. The solvent was removed under reduced pressure. A solution of the involatile dark oily residue in ethyl acetate (10 ml) was treated with an aqueous solution of potassium fluoride (2M; 10 ml). The mixture was vortexed for two minutes and then stirred for 15 minutes. The mixture was extracted with ethyl acetate (150 ml) and the extract was dried (MgSO₄). The dried extract was filtered, the filtrate was concentrated under reduced pressure, and the residue was purified by chromatography on silica gel (20 g) (dichloromethane to 2% methanol in dichloromethane gradient) to give the desired product (40 mg).
MS (ESP) 419.09 (MH⁺) for C₁₇H₁₅FN₆O₄S
¹H-NMR (DMSO-d₆) δ: 1.31 (t, 3H); 3.93 (dd, 1H); 4.26 (t, 1H); 4.39 (q, 2H); 4.80 (d, 2H); 5.14 (m, 1H); 7.50 (dd, 1H); 7.66 (dd, 1H); 7.70 (s, 1H); 8.12 (s, 1H); 8.32 (t, 1H).

The preparation of ethyl 2-chloro-[1,3,4]thiadiazole-5-carboxylate is described in
- Gadwood, Robert C.; Barbachyn, Michael R.; Toops, Dana S.; Smith, Herman W.; Vaillancourt, Valerie A. Preparation of 3-[4-(4-azolyl-1-piperazinyl)phenyl]oxazolidin-2-ones as bactericides. PCT Int. Appl. (1997), 79 pp. CODEN: PIXXD2 WO 9730981 A1 19970828 CAN 127:278210 AN 1997:579705 CAPLUS
- Demaree, Patricia; Doria, Marie Carmen; Muchowski, Joseph M. The reaction of certain α-diazocarbonyl compounds with thiophosgene and ethyl chlorodithioformate. Can. J. Chem. (1977), 55(2), 243-50. CODEN: CJCHAG CAN 88:6803 AN 1978:6803 CAPLUS

### Example 3: (5R)-3-(4-(5-(Aminomethyl)-1,3-thiazol-2-yl)-3-fluorophenyl)-5-(1H-1,2,3-triazol-1-ylmethyl)-1,3-oxazolidin-2-one

A mixture of(5*R*)-3-(3-fluoro-4-(trimethylstannyl)phenyl)-5-(1*H*-1,2,3-triazol-1-ylmethyl)-1,3-oxazolidin-2-one (425 mg, 1.0 mmol), *tert-*butyl (2-bromo-1,3-thiazol-5-yl)methylcarbamate (293 mg, 1.0 mmol), copper (I) iodide (38 mg, 0.2 mmol) in DMF (3 ml) under an atmosphere of nitrogen was treated with tetrakis(triphenylphoshine) palladium (0) (56 mg, 0.05 mmol). The mixture was stirred under an atmosphere of nitrogen for 5 hours at 75°C. The reaction mixture was treated with an aqueous solution of potassium fluoride (2M; 10 ml). Ethyl acetate (10 ml) was added and the mixture was vortexed for 5 minutes. The resulting precipitate was filtered off. The filtrate was extracted with ethyl acetate (100 ml) and the extract was dried (MgSO₄). The dried extract was filtered, the filtrate was concentrated under reduced pressure, and the residue was purified by chromatography on silica gel (20 g) (dichloromethane to 10% methanol in dichloromethane gradient) to give a yellow solid. This was dissolved in trifluoroacetic acid (5 ml) and stirred at room temperature for 20 minutes. The trifluoroacetic acid was removed *in vacuo* and the residue dissolved in DMSO (3 ml). This was purified by reverse phase chromatography (5% acetonitrile to 95% acetonitrile in water) to give the desired product (70 mg).
MS (ESP) 375.11 (MH⁺) for C₁₆H₁₅FN₆O₂S
¹H-NMR (DMSO-d₆) δ: 3.95 (dd, 1H); 4.28 (t, 1H); 4.39 (q, 2H); 4.84 (d, 2H); 5.18 (m, 1H); 7.45 (dd, 1 H); 7.63 (dd, 1H); 7.75 (s, 1H); 8.00 (s, 1H); 8.17 (s, 1H); 8.20 (t, 1H); 8.26 (s, 2H).

### Example 4: (5R)-3-(3-Fluoro-4-(5-methyl-1,3,4-thiadiazol-2-yl)phenyl)-5-(1H-1,2,3-triazol-1-ylmethyl)-1,3-oxazolidin-2-one

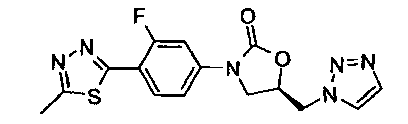

A mixture of 2-fluoro-N'-acetyl-4-((5*R*)-2-oxo-5-(1*H*-1,2,3-triazol-1-ylmethyl)-1,3-oxazolidin-3-yl)benzohydrazide (268 mg, 0.74 mmol), and Lawesson's reagent (597 mg, 1.47 mmol) in anhydrous toluene(5 ml) under an atmosphere of nitrogen was heated under reflux overnight. The solvent was removed under reduced pressure and the residue was purified by chromatography on C18 silica gel (Gilson HPLC) (dichloromethane to 2% methanol in dichloromethane gradient) to give the desired product (199 mg).
MS (ESP) 372.06 (MH⁺) for C₁₅H₁₃FN₆O₂S.
¹H-NMR (DMSO-d₆) δ: 2.82 (s, 3H); 3.99 (dd, 1H); 4.33 (t, 1H); 4.89 (d, 2H); 5.21 (m, 1H); 7.52 (m, 1H); 7.69 (dd, 1H); 7.79 (s, 1H); 8.21 (s, 1H); 8.27 (m, 1H).

The intermediates for this compound was prepared as follows:

### tert-Butyl 2-fluoro-4-((5R)-5-(1H-1,2.3-triazol-1-ylmethyl)-1,3-oxazolidin-2-on-3-yl)benzoate

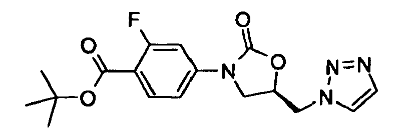

*tert*-Butyl-4-((5*R*)-5-(azidomethyl)-1,3-oxazolidin-2-on-3-yl)-2-fluorobenzoate (15 g, 44.6 mmol) was dissolved in dioxane (100 ml). Bicyclo[2.2.1]hepta-2,5-diene (12.3g, 133.8 mmol) was added and the mixture was heated under reflux under nitrogen for 18 hours. The solvent was evaporated *in vacuo* and the residue was redissolved in dichloromethane and treated with hexanes to give a precipitate that was filtered, washed with ethyl acetate and collected as the desired product. The filtrate was concentrated and subjected to chromatography on silica gel eluting with 100% ethylacetate to give a further sample of the title compound (combined product weight 14.3 g).
MS (ESP) 363.22 (MH⁺) for C₁₇H₁₉FN₄O₄.
¹H-NMR (DMSO-d₆) δ: 1.55 (s, 9H); 3.97 (m, 1H); 4.28 (t, 1H); 4.86 (d, 2H); 5.20 (m, 1H); 7.39 (dd, 1H); 7.52 (dd, 1H); 7.78 (s, 1H); 7.86 (t, 1H); 8.20 (s, 1H).

*tert-*Butyl 4-((*5R*)-5-(azidomethyl)-1,3-oxazolidin-2-on-3-yl)-2-fluorobenzoate 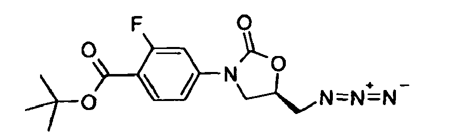 has been previously described in Gordeev, Mikhail F.; Luehr, Gary W.; Patel, Dinesh V.; Gadwood, Robert C. Preparation of N-acyl-3-aryl-2-oxooxazolidine-5-methanamines as bactericides. PCT Int. Appl. (2001), 134 pp. CODEN: PIXXD2 WO 0109107 A 1 20010208 CAN 134:163021 AN 2001:101116 CAPLUS

### 2-Fluoro-4-((5R)-5-(1H-1,2,3-triazol-1-ylmethyl)-1,3-oxazolidin-2-on-3-yl)benzoic acid

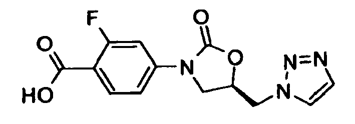

*tert*-Butyl 2-fluoro-4-((5*R*)-5-(1*H*-1,2,3-triazol-1-ylmethyl)-1,3-oxazolidin-2-on-3-yl)benzoate (14.2 g, 39.2 mmol) in dichloromethane was treated with 4*N* HCl in dioxane (5 equivalent) at 0°C, the mixture was stirred for 2 hours during which it was allowed to warn to room temperature. Solvent was evaporated under reduced pressure to give the desired product (11.9 g) in a form suitable for use without further purification.
MS (ESP) 307.14 (MH⁺) for C₁₃H₁₁FN₄O₄.
¹H-NMR (DMSO-d₆) δ: 3.97 (m, 1H); 4.29 (t, 1H); 4.86 (d, 2H); 5.20 (m, 1H); 7.39 (dd, 1H); 7.51 (dd, 1H); 7.78 (s, 1H); 7.90 (t, 1H); 8.20 (s, 1H); 13.11 (s, bd, 1H).

### N'-Acetyl-2-fluoro-4-((5R)-5-(1H-1,2,3-triazol-1-ylmethyl)-1,3-oxazolidin-2-on-3-yl)benzohydrazide

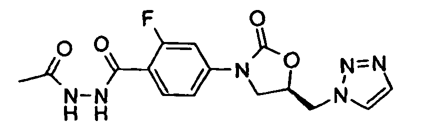

A mixture of 2-fluoro-4-((5*R*)-5-(1*H*-1,2,3-triazol-1-ylmethyl)-1,3-oxazolidin-2-on-3-yl)benzoic acid (450mg, 1.47mmol), HATU (565mg, 1.49mmol) and diisopropylethylamine (285mg, 2.21mmol) in dry DMF (5ml) was stirred at 0°C for 30 minutes, followed by the addition of acetic acid hydrazide (130.7mg, 1.76mmol). The reaction mixture was then warmed up to room temperature and stirred for 2 hours. The mixture was diluted with dichloromethane (20ml), washed with saturated aqueous NaHCO₃ and brine, dried over anhydrous MgSO₄ and concentrated under reduced pressure. The remaining residue was purified by column chromatography on silica gel (5% MeOH in dichloromethane) to give the desired product (289 mg).
MS (ESP+) 363.16 (MH⁺) for C₁₅H₁₅FN₆O₄.
¹H-NMR (DMSO-d₆) δ: 1.92 (s, 3H); 3.96 (dd, 1H); 4.29 (t, 1H); 4.88 (d, 2H); 5.20 (m, 1H); 7.38 (dd, 1H); 7.53 (dd, 1H); 7.69 (t, 1H); 7.79 (s, 1H); 8.20 (s, 1H); 9.97 (s, 1H); 10.05 (s, 1H).

### Example 5: (5R)-3-(3-Fluoro-4-(4-methyl-1,3-thiazol-2-yl)phenyl)-5-(1H-1,2,3-triazol-1-ylmethyl)-1,3-oxazolidin-2-one

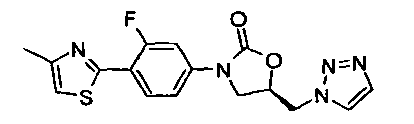

A mixture of 2-fluoro-4-((5*R*)-5-(1*H*-1,2,3-triazol-1-ylmethyl)-1,3-oxazolidin-2-on-3-yl)benzenecarbothioamide (30 mg, 0.094 mmol) and 1-chloroacetone (13 mg, 0.14 mmol) in dry DMF was stirred at 60°C overnight. Solvent was removed under reduced pressure and the residue was purified by reverse phase chromatography (Gilson MPLC C18 column, 5% to 95% acetonitrile in H₂O) to give the title compound (21 mg).
MS (ESP) 360.25 (MH⁺) for C₁₆H₁₄FN₅O₂S.
¹H-NMR (DMSO-d₆) δ: 2.47 (s, 3H); 3.99 (dd, 1H); 4.29 (t, 1H); 4.88 (d, 2H); 5.20 (m, 1H); 7.45 (s, 1H); 7.48 (dd, 1H); 7.64 (dd, 1H); 7.79 (s, 1H); 8.20 (s, 1H); 8.21 (m, 1H)..

The intermediates for this compound were prepared as follows:

### 2-Fluoro-4-((5R)-5-(1H-1,2,3-triazol-1-ylmethyl)-1,3-oxazolidin-2-on-3-yl)benzamide

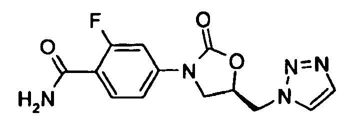

A solution of 2-fluoro-4-((5*R*)-5-(1*H* 1,2,3-triazol-1-ylmethyl)-1,3-oxazolidin-2-on-3-yl)benzoic acid (2.5 g, 8.17 mmol) in dry dichloromethane (25 ml) at 0°C was treated with oxalyl chloride (1.56 g, 12.26 mmol) along with one drop of dimethylformaldehyde. The reaction mixture was allowed to warm to room temperature and then stirred for one hour. Ammonium hydroxide (10 eq.) was then added and the reaction mixture was stirred overnight. The mixture was diluted with additional dichloromethane (20 ml) and then hexanes (30 ml) to give a precipitate that was isolated by filtration to give the desired product (1.5 g).
MS (ESP) 306 (MH⁺) for C₁₃H₁₂FN₅O₃.
¹H-NMR (DMS O-d₆) δ: 3.96 (m, 1H); 4.29 (t, 1H); 4.86 (d, 2H); 5.20 (m, 1H); 7.34 (m, 1H); 7.49 (dd, 1H); 7.60 (s, 2H); 7.74 (t, 1H); 7.78 (s, 1H); 8.20 (s, 1H).

### 2-Fluoro-4-((5R)-5-(1H-1,2,3-triazol-1-ylmethyl)-1,3-oxazolidin-2-on-3-yl)benzenecarbothioamide

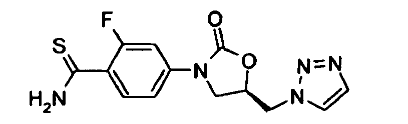

A mixture of 2-fluoro-4-((5*R*)-5-(1*H*-1,2,3-triazol-1-ylmethyl)-1,3-oxazolidin-2-on-3-yl)benzamide (95 mg, 0.31 mmol) and Lawesson's reagent (126 mg, 0.31 mmol) in dry toluene (1 ml) was sealed in a microwave reaction vessel and then irradiated in a Smith Microwave reactor at 160°C for 20 minutes. Solvent was evaporated under reduced pressure and the residue was purified by reverse phase chromatography (Gilson MPLC; C18 column; 5% to 95% acetonitrile in H₂O) to give the title compound (35 mg).
MS (ESP+) 322.24 (MH⁺) for C₁₃H₁₂FN₅O₂S.
¹H-NMR (DMSO-d₆) δ: 3.94 (dd, 1H); 4.28 (t, 1H); 4.87 (d, 2H); 5.19 (m, 1H); 7.28 (dd, 1H); 7.45 (dd, 1H); 7.71 (t, 1H); 7.78 (s, 1H); 8.19 (s, 1H); 9.48 (s, 1H); 10.10 (s, 1H).

### Example 6: (5R)-3-(3-Fluoro-4-(4-(trifluoromethyl)-1,3-thiazol-2-yl)phenyl)-5-(1H-1,2,3-triazol-1-ylmethyl)-1,3-oxazolidin-2-one

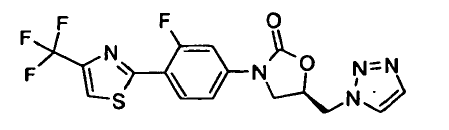

(5*R*)-3-(3-Fluoro-4-(4-hydroxy-4-(trifluoromethyl)-4,5-dihydro-1,3-thiazol-2-yl)phenyl)-5-(1*H*-1,2,3-triazol-1-ylmethyl)-1,3-oxazolidin-2-one (25 mg, 0.058 mmol) in ethanol (3 ml) was heated to 150°C in a sealed tube for 7 days. The solvent was then evaporated and the residue was purified by column chromatography (silica gel; 5% MeOH in dichloromethane) to give the title compound (14 mg).
MS (ESP) 414.18 (MH⁺) for C₁₆H₁₁F₄N₅O₂S.
¹H-NMR (DMSO-d₆) δ: 3.99 (dd, 1H); 4.29 (t, 1H); 4.88 (d, 2H); 5.20 (m, 1H); 7.45 (dd, 1H); 7.65 (dd, 1H); 7.79 (s, 1H); 8.10 (m, 1H); 8.21 (s, 1H); 8.38 (s, 1H).

The intermediate for this compound was prepared as follows:

### (5R)-3-(3-Fluoro-4-(4-hydroxy-4-(trifluoromethyl)-4,5-dihydro-1,3-thiazol-2-yl)phenyl)-5-(1H-1,2,3-triazol-1-ylmethyl)-1,3-oxazolidin-2-one

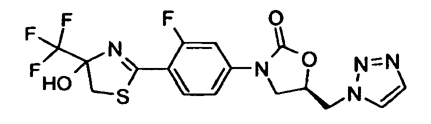

A mixture of 2-fluoro-4-((5*R*)-5-(1*H*-1,2,3-triazol-1-ylmethyl)-1,3-oxazolidin-2-on-3-yl)benzenecarbothioamide (30 mg, 0.094 mmol) and 1-bromo-3,3,3-trifluoroacetone (27 mg, 0.14 mmol) in dry DMF was stirred at 60°C overnight. Solvent was then evaporated under reduced pressure and the residue was purified by reverse phase chromatography (Gilson MPLC; C18 column, 5% to 95% acetonitrile in H₂O) to give the title compound (32 mg).
MS (ESP) 432.07 (MH⁺) for C₁₆H₁₆F₄N₅O₃S.
¹H-NMR (DMSO-d₆) δ: 3.52 (d, 1H); 3.82 (d, 1H); 3.98 (dd, 1H); 4.30 (t, 1H); 4.88 (d, 2H); 5.20 (m, 1H); 7.49 (dd, 1H); 7.62 (dd, 1H); 7.79 (s, 1H); 8.01 (t, 1H); 8.21 (s, 1H).

### Example 7: (5-(2-Fluoro-4-((5R)-5-(1H-1,2,3-triazol-1-ylmethyl)-1,3-oxazolidin-2-on-3-yl)phenyl)-1,3,4-thiadiazol-2-yl)acetonitrile

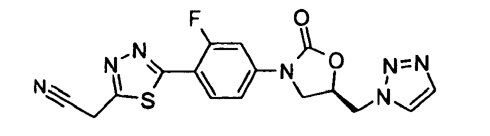

The title compound was prepared from N'-(cyanoacetyl)-2-fluoro-4-((5*R*)-5-(1*H*-1,2,3-triazol-1-ylmethyl)-1,3-oxazolidin-2-on-3-yl)benzohydrazide by the method described for Example 4. MS (ESP+) 386.06 (MH⁺) for C₁₆H₁₂FN₇O₂S.
¹H-NMR (DMSO-d₆) δ: 4.01 (dd, 1H); 4.33 (t, 1H); 4.80 (s, 2H); 4.88 (dd, 2H); 5.20 (m, 1H); 7.58 (dd, 1 H); 7.71 (dd, 1H); 7.79 (s, 1H); 8.20 (s, 1H); 8.30 (t, 1H).

The intermediate for this compound was prepared as follows:

### N'-(Cyanoacelyl)-2-fluoro-4-((5R)-5-(1H-1,2,3-triazol-1-ylmethyl)-1,3-oxazolidin-2-on-3-yl)benzohydrazide

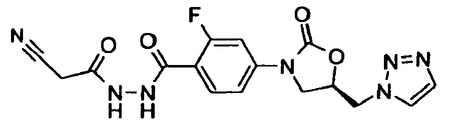

A mixture of 2-fluoro-4-((5*R*)-5-(1*H*-1,2,3-triazol-1-ylmethyl)-1,3-oxazolidin-2-on-3-yl)benzoic acid (450 mg, 1.47 mmol), HATU (671 mg, 1.764 mmol) and diisopropylethylamine (285 mg, 2.21 mmol) in dry DMF (5 ml) was stirred at 0°C for 30 minutes and then treated with cyanoacetohydrazide (219 mg, 2.21 mmol). The reaction mixture was then warmed up to room temperature and stirred for 2 hours. The mixture was diluted with dichloromethane (20 ml), washed with saturated aqueous NaHCO₃ and then brine, dried over anhydrous MgSO₄ and evaporated under reduced pressure. The remaining residue was purified by column chromatography on silica gel (5% MeOH in dichloromethane) to give the desired product (300mg).
¹H-NMR (DMSO-d₆) δ: 2.71 (s, 2H); 3.96 (dd, 1H); 4.29 (t, 1H); 4.88 (d, 2H); 5.20 (m, 1H); 7.38 (dd, 1H); 7.53 (dd, 1H); 7.69 (t, 1H); 7.79 (s, 1H); 8.20 (s, 1H); 10.32 (s, 1H); 10.44 (s, 1H).

## Claims

1. A compound of the formula (I), or a pharmaceutically-acceptable salt, or an in-vivo-hydrolysable ester thereof, 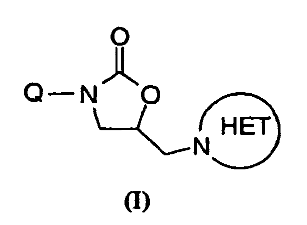 wherein
HET is an N-linked 5-membered, fully or partially unsaturated heterocyclic ring, containing either (i) 1 to 3 further nitrogen heteroatoms or (ii) a further heteroatom selected from O and S together with an optional further nitrogen heteroatom; which ring is optionally substituted on a C atom, other than a C atom adjacent to the linking N atom, by an oxo or thioxo group; and/or which ring is optionally substituted on any available C atom, other than a C atom adjacent to the linking N atom, by a substituent Rs wherein;
Rs is selected from the group:
(Rsa): halogen, (1-4C)alkoxy, (2-4C)alkenyloxy, (2-4C)alkenyl, (2-4C)alkynyl, (3-6C)cycloalkyl, (3-6C)cycloalkenyl, amino, (1-4C)alkylamino, di-(1-4C)alkylamino, (2-4C)alkenylamino, (1-4C)alkylcarbonylamino, (1-4C)alkylthiocarbonylamino, (1-4C)alkyl-OCO-NH-, (1-4C)alkyl-NH-CO-NH-, (1-4C)alkyl-NH-CS-NH-, (1-4C)alkyl-SO₂-NH or (1-4C)alkyl-S(O)q- (wherein q is 0, 1 or 2);
or Rs is selected from the group
(Rsb): (1-4C)alkyl group which is optionally substituted by one substituent selected from hydroxy, (1-4C)alkoxy, amino, cyano, azido, (2-4C)alkenyloxy, (1-4C)alkylcarbonyl, (1-4C)alkoxycarbonyl, (1-4C)alkylamino, (2-4C)alkenylamino, (1-4C)alkyl-SO₂-NH-, (1-4C)alkylcarbonylamino, (1-4C)alkylthiocarbonylamino, (1-4C)alkyl-OCO-NH-, (1-4C)aikyl-NH-CO-NH-, (1-4C)alkyl-NH-CS-NH-, (1-4C)alkylSO₂-NH-, (1-4C)alkyl-S(O)q- (wherein q is 0, 1 or 2), (3-6C)cycloalkyl, (3-6C)cycloalkenyl, or an N-linked 5-membered heteroaryl ring, which ring contains either (i) 1 to 3 further nitrogen heteroatoms or (ii) a further heteroatom selected from O and S together with an optional further nitrogen heteroatom; which ring is optionally substituted on a carbon atom by an oxo or thioxo group; and/or the ring is optionally substituted on a carbon atom by 1 or 2 (1-4C)alkyl groups; and/or on an available nitrogen atom (provided that the ring is not thereby quaternised) by (1-4C)alkyl;
and wherein at each occurrence of an Rs substituent containing an alkyl, alkenyl, alkynyl, cycloalkyl or cycloalkenyl moiety in (Rsa) or (Rsb) each such moiety is optionally further substituted on an available carbon atom with one or more substituents independently selected from F, Cl and Br and/or by one cyano group; and/or which ring is optionally substituted on an available nitrogen atom (provided that the ring is not thereby quaternised) by (1-4C)alkyl; or HET is an N-linked 6-membered di-hydro-heteroaryl ring containing up to three nitrogen heteroatoms in total (including the linking heteroatom), which ring is substituted on a suitable C atom, other than a C atom adjacent to the linking N atom, by oxo or thioxo and/or which ring is optionally substituted on any available C atom, other than a C atom adjacent to the linking N atom, by one or two substituents Rs, wherein Rs is as hereinbefore defined, and/or on an available nitrogen atom (provided that the ring is not thereby quaternised) by (1-4C)alkyl; and wherein at each occurrence of alkyl, alkenyl and cycloalkyl HET substituents, each is optionally substituted with one or more substituents independently selected from F, Cl and Br and/or by one cyano group;
Q is Q1:- 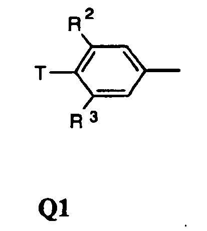 wherein R² and R³ are independently hydrogen or fluoro;
T is selected from the following groups of formula (TAa1 to (TAa6) below (wherein AR1, AR2, AR2a, AR2b, AR3, AR3a, AR3b, AR4, AR4a, CY1 and CY2 are defined hereinbelow); 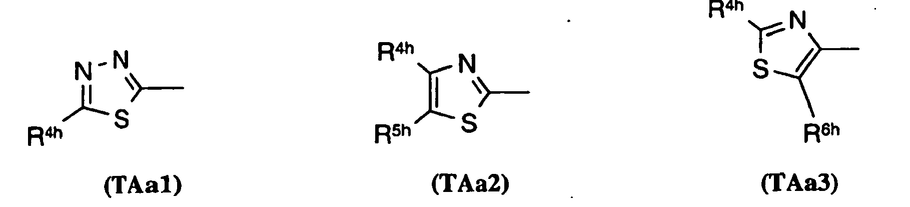 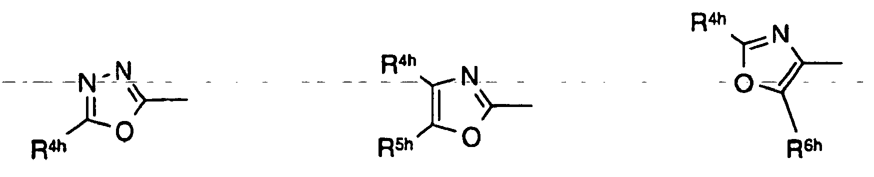 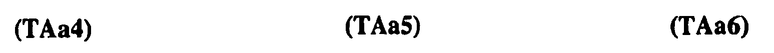 wherein :
R^{6h} is hydrogen or (1-4C)alkyl, and R^{4h} and R^{5h} are independently selected from hydrogen, cyano, (1-4C)alkoxycarbonyl, -CONRvRw, hydroxy(1-4C)alkyl,
NRvRw(1-4C)alkyl, -NRcRv(1-4C)alkyl; wherein Rv is hydrogen or (1-4C)alkyl; Rw is hydrogen or (1-4C)alkyl};
wherein Rc is selected from groups (Rc1) to (Rc5) :-
***(Rc1)*** (1-6C)alkyl {optionally substituted by one or more (1-4C)alkanoyl groups (including geminal disubstitution) and/or optionally monosubstituted by cyano, (1-4C)alkoxy, trifluoromethyl, (1-4C)alkoxycarbonyl, phenyl (optionally substituted as for AR1 defined hereinafter), (1-4C)alkylS(O)_{q}- (q is 0, 1 or 2); or, on any but the first carbon atom of the (1-6C)alkyl chain, optionally substituted by one or more groups (including geminal disubstitution) each independently selected from hydroxy and fluoro, and/or optionally monosubstituted by oxo, -NRvRw [wherein Rv is hydrogen or (1-4C)alkyl; Rw is hydrogen or (1-4C)alkyl], (1-6C)alkanoylanuno, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is 1 or 2)};
***(Rc2)*** R¹³CO- , R¹³SO₂- or R¹³CS-
wherein R¹³ is selected from (Rc2a) to (Rc2e) :-
***(Rc2a)*** AR1, AR2, AR2a, AR2b, AR3, AR3a, AR3b, AR4, AR4a, CY1, CY2;
***(Rc2b)*** hydrogen, (1-4C)alkoxycarbonyl, trifluoromethyl, -NRvRw [wherein Rv is hydrogen or (1-4C)alkyl; Rw is hydrogen or (1-4C)alkyl], ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, 2-(AR2a)ethenyl;
***(Rc2c)*** (1-10C)alkyl
{optionally substituted by one or more groups (including geminal disubstitution) each independently selected from hydroxy, (1-10C)alkoxy, (1-4C)alkoxy-(1-4C)alkoxy, (1-4C)alkoxy-(1-4C)alkoxy-(1-4C)alkoxy, (1-4C)alkanoyl, carboxy, phosphoryl [-O-P(O)(OH)₂, and mono- and di-(1-4C)alkoxy derivatives thereof], phosphiryl [-O-P(OH)₂ and mono- and di-(1-4C)alkoxy derivatives thereof], and amino; and/or optionally substituted by one group selected from phosphonate [phosphono, -P(O)(OH)₂, and mono- and
di-(1-4C)alkoxy derivatives thereof], phosphinate [-P(OH)₂ and mono- and di-(1-4C)alkoxy derivatives thereof], cyano, halo, trifluoromethyl, (1-4C)alkoxycarbonyl, (1-4C)alkoxy-(1-4C)alkoxycarbonyl, (1-4C)atkoxy-(1-4C)alkoxy-(1-4C)alkoxycarbonyl, (1-4C)alkylamino, di((1-4C)alkyl)amino, (1-6C)alkanoylamino, (1-4C)alkoxycarbonylamino, N-(1-4C)alkyl-N-(1-6C)alkanoylamino, (1-4C)alkylaminocarbonyl, di((1-4C)alkyl)aminocarbonyl, (1-4C)alkylS(O)ₚNH-, (1-4C)alkylS(O)ₚ-((1-4C)alkyl)N-, fluoro(1-4C)alkylS(O)ₚNH-, fluoro(1-4C)alkylS(O)ₚ((1-4C)alkyl)N-, (1-4C)alkylS(O)_{q}- [the (1-4C)alkyl group of (1-4C)alkylS(O)_{q}- being optionally substituted by one substituent selected from hydroxy, (1-4C)alkoxy, (1-4C)alkanoyl, phosphoryl [-O-P(O)(OH)₂, and mono- and di-(1-4C)alkoxy derivatives thereof], phosphiryl [-O-P(OH)₂ and mono- and di-(1-4C)alkoxy derivatives thereof], amino, cyano, halo, trifluoromethyl, (1-4C)alkoxycarbonyl, (1-4C)alkoxy-(1-4C)alkoxycarbonyl, (1-4C)alkoxy-(1-4C)alkoxy-(1-4C)alkoxycarbonyl, carboxy, (1-4C)alkylamino, di((1-4C)alkyl)amino, (1-6C)alkanoylamino, (1-4C)alkoxycarbonylamino, N-(1-4C)alkyl-N-(1-6C)alkanoylamino, (1-4C)alkylaminocarbonyl,
di((1-4C)alkyl)aminocarbonyl, (1-4C)alkylS(O)ₚNH-, (1-4C)alkylS(O)ₚ-((1-4C)alkyl)N-, (1-4C)alkylS(O)_{q}-, AR1-S(O)_{q}- , AR2-S(O)_{q}- , AR3-S(O)_{q}- and also AR2a, AR2b, AR3a and AR3b versions of AR2 and AR3 containing groups], CY1, CY2, AR1, AR2, AR3, AR1-O-, AR2-O-, AR3-O-, AR1-S(O)_{q}- , AR2-S(O)_{q}- , AR3-S(O)_{q}- , AR1-NH-, AR2-NH-, AR3-NH- (p is 1 or 2 and q is 0, 1 or 2), and also AR2a, AR2b, AR3a and AR3b versions of AR2 and AR3 containing groups};
***(Rc2d)*** R¹⁴C(O)O(1-6C)alkyl wherein R¹⁴ is AR1, AR2, (1-4C)alkylamino (the (1-4C)alkyl group being optionally substituted by (1-4C)alkoxycarbonyl or by carboxy), benzyloxy-(1-4C)alkyl or (1-10C)alkyl {optionally substituted as defined for (Rc2c)};
***(Rc2e)*** R¹⁵O- wherein R¹⁵ is benzyl, (1-6C)alkyl {optionally substituted as defined for (Rc2c)}, CY1, CY2 or AR2b;
***(Rc3)*** hydrogen, cyano, 2-cyanoethenyl, 2-cyano-2-((1-4C)alkyl)ethenyl, 2-((1-4C)alkylaminocarbonyl)ethenyl, 2-((1-4C)alkoxycarbonyl)ethenyl, 2-nitroethenyl, 2-nitro-2-((1-4C)alkyl)ethenyl, 2-(AR1)ethenyl, 2-(AR2)ethenyl, or of the formula **(Rc3a)** 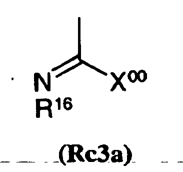 wherein X⁰⁰ is -OR¹⁷, -SR¹⁷, -NHR¹⁷ and -N(R¹⁷)₂ ;
wherein R¹⁷ is hydrogen (when X⁰⁰ is -NHR¹⁷ and -N(R¹⁷)₂), and R¹⁷ is (1-4C)alkyl, phenyl or AR2 (when X⁰⁰ is -OR¹⁷, -SR¹⁷ and -NHR¹⁷); and R¹⁶ is cyano, nitro, (1-4C)alkylsulfonyl, (4-7C)cycloalkylsulfonyl, phenylsulfonyl, (1-4C)alkanoyl and (1-4C)alkoxycarbonyl;
***(Rc4)*** trityl, AR1, AR2, AR2a, AR2b, AR3, AR3a, AR3b;
**(*Rc5*)** RdOC(Re)=CH(C=O)-, RfC(=O)C(=O)-, RgN=C(Rh)C(=O)- or RiNHC(Rj)=CHC(=O)- wherein Rd is (1-6C)alkyl; Re is hydrogen or (1-6C)alkyl, or Rd and Re together form a (3-4C)alkylene chain; Rf is hydrogen, (1-6C)alkyl, hydroxy(1-6C)alkyl, (1-6C)alkoxy(1-6C)alkyl, -NRvRw [wherein Rv is hydrogen or (1-4C)alkyl; Rw is hydrogen or (1-4C)alkyl], (1-6C)alkoxy, (1-6C)alkoxy(1-6C)alkoxy, hydroxy(2-6C)alkoxy, (1-4C)alkylamino(2-6C)alkoxy, di-(1-4C)alkylamino(2-6C)alkoxy; Rg is (1-6C)alkyl, hydroxy or (1-6C)alkoxy; Rh is hydrogen or (1-6C)alkyl; Ri is hydrogen, (1-6C)alkyl, AR1, AR2, AR2a, AR2b and Rj is hydrogen or (1-6C)alkyl;
wherein
**AR1** is an optionally substituted phenyl or optionally substituted naphthyl;
**AR2** is an optionally substituted 5- or 6-membered, fully unsaturated (i.e with the maximum degree of unsaturation) monocyclic heteroaryl ring containing up to four heteroatoms independently selected from O, N and S (but not containing any O-O, O-S or S-S bonds), and linked via a ring carbon atom, or a ring nitrogen atom if the ring is not thereby quatemised;
**AR2a** is a partially hydrogenated version of AR2 (i.e. AR2 systems retaining some, but not the full, degree of unsaturation), linked via a ring carbon atom or linked via a ring nitrogen atom if the ring is not thereby quaternised;
**AR2b** is a fully hydrogenated version of AR2 (i.e. AR2 systems having no unsaturation), linked via a ring carbon atom or linked via a ring nitrogen atom;
**AR3** is an optionally substituted 8-, 9- or 10-membered, fully unsaturated (i.e with the maximum degree of unsaturation) bicyclic heteroaryl ring containing up to four heteroatoms independently selected from O, N and S (but not containing any O-O, O-S or S-S bonds), and linked via a ring carbon atom in either of the rings comprising the bicyclic system;
**AR3a** is a partially hydrogenated version of AR3 (i.e. AR3 systems retaining some, but not the full, degree of unsaturation), linked via a ring carbon atom, or linked via a ring nitrogen atom if the ring is not thereby quaternised, in either of the rings comprising the bicyclic system;
**AR3b** is a fully hydrogenated version of AR3 (i.e. AR3 systems having no unsaturation), linked via a ring carbon atom, or linked via a ring nitrogen atom, in either of the rings comprising the bicyclic system;
**AR4** is an optionally substituted 13- or 14-membered, fully unsaturated (i.e with the maximum degree of unsaturation) tricyclic heteroaryl ring containing up to four heteroatoms independently selected from O, N and S (but not containing any O-O, O-S or S-S bonds), and linked via a ring carbon atom in any of the rings comprising the tricyclic system;
**AR4a** is a partially hydrogenated version of AR4 (i.e. AR4 systems retaining some, but not the full, degree of unsaturation), linked via a ring carbon atom, or linked via a ring nitrogen atom if the ring is not thereby quaternised, in any of the rings comprising the tricyclic system;
**CY1** is an optionally substituted cyclobutyl, cyclopentyl or cyclohexyl ring;
**CY2** is an optionally substituted cyclopentenyl or cyclohexenyl ring.

2. A compound of formula (IB), or a pharmaceutically-acceptable salt, or in-vivo hydrolysable ester thereof, 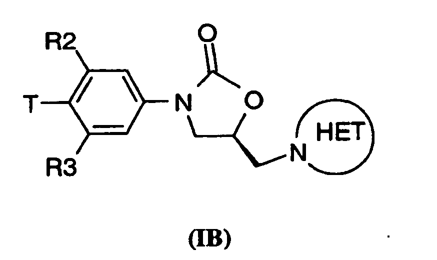 wherein HET is 1,2,3-triazole, 1,2,4-triazole or tetrazole, or HET is a di-hydro version of pyrimidine, pyridazine, pyrazine, 1,2,3-triazine, 1,2,4-triazine, 1,3,5-triazine and pyridine;
R² and R³ are independently hydrogen or fluoro; and
T is selected from (TAa1 to TAa6).

3. A compound of formula (IB) as claimed in claim 2, or a pharmaceutically-acceptable salt, or in-vivo hydrolysable ester thereof, wherein
wherein HET is 1,2,3-triazole, 1,2,4-triazole or tetrazole;
R² and R³ are independently hydrogen or fluoro; and
T is selected from (TAa1 & 2).

4. A compound of formula (IB) as claimed in claim 3, or a pharmaceutically-acceptable salt, or in-vivo hydrolysable ester thereof, wherein
wherein HET is 1,2,3-triazol-1-yl, 1,2,4-triazol-1-yl or tetrazol-2-yl;
R² and R³ are independently hydrogen or fluoro; and
T is selected from (TAa1 & 2).

5. A compound of the formula (I) as claimed in any preceding claim, or a pharmaceutically-acceptable salt, or in-vivo hydrolysable ester thereof, wherein Rs is selected from fluoromethyl, difluoromethyl, trifluoromethyl, chloromethyl, bromomethyl, cyanomethyl, cyano, amino, azido, alkylthioalkyl such as methylthiomethyl and 2-propynyl.

6. A compound of the formula (I) as claimed in claim 1, or a pharmaceutically-acceptable salt, or in-vivo hydrolysable ester thereof, for use as a medicament.

7. The use of a compound of the formula (I) as claimed in claim 1, or a pharmaceutically-acceptable salt, or in-vivo hydrolysable ester thereof, in the manufacture of a medicament for use in the production of an antibacterial effect in a warm blooded animal.

8. A pharmaceutical composition which comprises a compound of the formula (I) as claimed in claim 1, or a pharmaceutically-acceptable salt or an in-vivo hydrolysable ester thereof, and a pharmaceutically-acceptable diluent or carrier.

9. A compound of the formula (I), or a pharmaceutically-acceptable salt, or in-vivo hydrolysable ester thereof for use in a method of treatment of the human or animal body by therapy.

10. A process for the manufacture of a compound of the formula (I) comprising one or more of the processes **(a)** to **(i)** below:
**(a)** by modifying a substituent in or introducing a substituent into another compound of formula (I);
**(b)** by reaction of a compound of formula (II) : 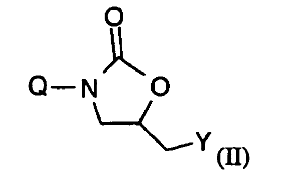 wherein Y is a displaceable group with a compound of the formula (III) :
HET (III)
wherein HET is HET-H free-base form or HET- anion formed from the free base form;
**(c)** by reaction of a compound of the formula (IV) :
Q-Z (IV)
wherein Z is an isocyanate, amine or urethane group with an epoxide of the formula (V): 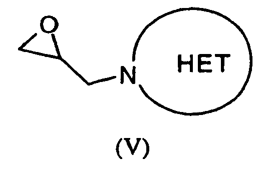
**(d)** by reaction of a compound of formula (VI) : 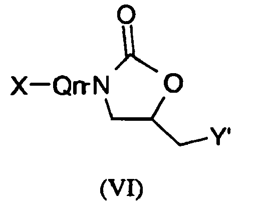 wherein Y' is a group HET as hereinabove defined, X is a replaceable substituent located at a position substituted by T in any of the aromatic embodiments Q1 - Q8 of Qn as hereinabove defined for Q, but with X in place of the substituent T, with a compound of the formula (VII) :
T-X' (Vll)
wherein T-X' is a five-membered heterocycle with 1-3 heteroatoms drawn in combination from O, N, and S and X' is a replaceable C-linked substituent; wherein the substituents X and X' are chosen to be complementary pairs of substituents known in the art to be suitable as complementary substrates for coupling reactions catalysed by transition metals such as palladium(0);
**(e)** by reaction of a compound of formula (VIII): 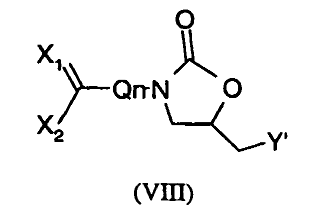 wherein Y' is a group HET as defined herein above and X1 and X2 here are independently optionally substituted heteroatoms drawn in combination from O N, and S such that C(X1)X2 constitutes a substituent that is a carboxylic acid derivative substituent located at a position substituted by T in any of the aromatic embodiments Q1 - Q10 of Qn as hereinabove defined for Q with a compound of the formula (IX) and X3 and X4 are independently optionally substituted heteroatoms drawn in combination from O, N, and S: 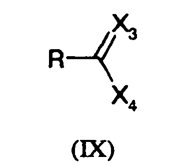 and wherein one of C(X1)X2 and C(X3)X4 constitutes an optionally substituted hydrazide, thiohydrazide, or amidrazone, and the other one of C(X1)X2 and C(X3)X4 constitutes an optionally substituted acylating, thioacylating, or imidoylating agent such that C(X1)X2 and C(X3)X4 may be condensed together to form a 5-membered heterocycle containing 3 heteroatoms drawn in combination from O, N, and S, for instance thiadiazole, by methods well-known in the art;
**(f)** by reaction of a compound of formula (X): 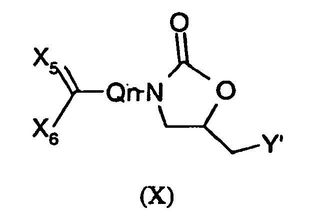 wherein Y' is a group HET as defined herein above and C(X5)X6 constitutes a substituent located at a position substituted by T in any of the aromatic embodiments Q1 - Q8 of Qn as hereinabove defined for Q with a compound of the formula (XI): 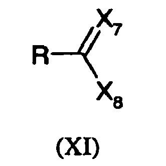 wherein one of C(X5)X6 and C(X7)X8 constitutes an optionally substituted alpha-(leaving-group-substituted)ketone, wherein the leaving group is for example a halo-group or an (alkyl or aryl)-sulfonyloxy-group, and the other one of C(X5)X6 and C(X7)X8 constitutes an optionally substituted amide, thioamide, or amidine, such that C(X5)X6 and C(X7)X8 are groups that may be condensed together to form a 5-membered heterocycle containing 2 heteroatoms drawn in combination from O, N, and S, for instance thiazole, by methods well-known in the art;
**(g)** for HET as optionally substituted 1,2,3-triazoles compounds of formula (I) may be made by cycloaddition via the azide (wherein e.g. Y in (II) is azide) to acetylenes, or to acetylene equivalents such as optionally substituted cylcohexa-1,4-dienes or optionally substituted ethylenes bearing eliminatable substituents such as arylsulfonyl;
**(h)** for HET as 4-substituted 1,2,3-triazole compounds of formula (I) may be made by reacting aminomethylisoxazolines with 1,1-dihaloketone sulfonylhydrazones;
**(i)** for HET as 4-substituted 1,2,3-triazole compounds of formula (I) may also be made by reacting azidomethyl isoxazolines with terminal alkynes using Cu(1) catalysis;
and thereafter if necessary: (i) removing any protecting groups; (ii) forming a pharmaceutically-acceptable salt; (iii) forming an in-vivo hydrolysable ester.

11. A compound selected from
(*5R*)-3-(3-Fluoro-4-(5-cyano-1,3,4-thiadiazol-2-yl)phenyl)-5-(1*H*-1,2,3-triazol-1-ylmethyl)-1,3-oxazolidin-2-one;
*(5R)-*3-(3*-*Fluoro-4-(5-ethoxycarbonyl-1,3,4-thiadiazol-2-yl)phenyl)-5-(1*H*-1,2,3-triazol-1-ylmethyl)-1,3-oxazolidin-2-one;
(*5R*)-3-(4-(5-(Aminomethyl)-1,3-thiazol-2-yl)-3-fluorophenyl)-5-(1*H*-1,2,3-triazol-1-ylmethyl)-1,3-oxazolidin-2-one;
(*5R*)-3-(3-Fluoro-4-(5-methyl-1,3,4-thiadiazol-2-yl)phenyl)-5-(1*H*-1,2,3-triazol-1-ylmethyl)-1,3-oxazolidin-2-one;
(*5R*)-3-(3-Fluoro-4-(4-methyl-1,3-thiazol-2-yl)phenyl)-5-(1*H*-1,2,3-triazol-1-ylmethyl)-1,3-oxazolidin-2-one;
(5*R*)-3-(3-Fluoro-4-(4-(trifluoromethyl)-1,3-thiazol-2-yl)phenyl)-5-(1*H* 1,2,3-triazol-1-ylmethyl)-1,3-oxazolidin-2-one; and
(5-(2-Fluoro-4-((5*R*)-5-(1*H*-1,2,3-triazol-1-ylmethyl)-1,3-oxazolidin-2-on-3-yl)phenyl)-1,3,4-thiadiazol-2-yl)acetonitrile;
or a pharmaceutically-acceptable salt or an in-vivo hydrolysable ester thereof.

## Revendications

1. Composé de formule (I), ou un sel pharmaceutiquement acceptable, ou un ester hydrolysable in vivo de celui-ci, 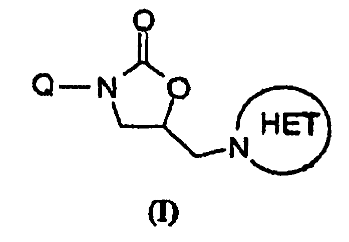 dans laquelle
HET est un cycle hétérocyclique totalement ou partiellement insaturé, à 5 chaînons lié par N, contenant soit (i) 1 à 3 autres hétéroatomes d'azote soit (ii) un autre hétéroatome choisi parmi O et S ensemble avec un autre hétéroatome d'azote éventuel ; lequel cycle est éventuellement substitué sur un atome de C, autre qu'un atome de C adjacent à l'atome de N lieur, par un groupe oxo ou thioxo, et/ou lequel cycle est éventuellement substitué sur tout atome de C disponible, autre qu'un atome de C adjacent à l'atome de N lieur, par un substituant Rs où ;
Rs est choisi dans le groupe :
(Rsa) : l'halogène, un (1-4C)alcoxy, un (2-4C)alcényloxy, un (2-4C) alcényle, un (2-4C)alcynyle, un (3-6C)cycloalkyle, un (3-6C)cycloalcényle, un amino, un (1-4C)alkylamino, un di-(1-4C)alkylamino, un (2-4C)alcénylamino, un (1-4C)alkylcarbonylamino, un (1-4C)alkylthiocarbonylamino, un (1-4C)alkyl-OCO-NH-, (1-4C)alkyl-NH-CO-NH-, un (1-4C)alkyl-NH-CS-NH-, (1-4C)alkyl-SO₂-NH- ou un (1-4C)alkyl-S(O)q- (où q est 0, 1 ou 2) ;
ou Rs est choisi dans le groupe
(Rsb) : un groupe (1-4C)alkyle qui est éventuellement substitué par un substituant choisi parmi un hydroxy, un (1-4C)alcoxy, un amino, un cyano, un azido, un (2-4C)alcényloxy, un (1-4C)alkylcarbonyle, un (1-4C)alcoxycarbonyle, un (1-4C)alkylamino, un (2-4C)alcénylamino, un (1-4C)alkyl-SO₂-NH-, un (1-4C)alkylcarbonylamino, un (1-4C)alkylthiocarbonylamino, un (1-4C)alkyl-OCO-NH-, un (1-4C)alkyl-NO-CO-NH-, (1-4C)alkyl-NH-CS-NH-, un (1-4C)alkyl-SO₂-NH-, un (1-4C)alkyl-S(O)q-(où q est 0, 1 ou 2), un (3-6C)cycloalkyle, un (3-6C)cycloalcényle, ou un cycle hétéroaryle à 5 chaînons lié par N, lequel cycle contient soit (i) 1 à 3 autres hétéroatomes d'azote soit (ii) un autre hétéroatome choisi parmi 0 et S ensemble avec un autre hétéroatome d'azote éventuel ; lequel cycle est éventuellement substitué sur un atome de carbone par un groupe oxo ou thioxo ; et/ou le cycle est éventuellement substitué sur un atome de carbone par 1 ou 2 groupes (1-4C)alkyle ; et/ou sur un atome d'azote disponible (à condition que le cycle ne soit pas ainsi quaternisé) par (1-4C)alkyle ;
et où à chaque apparition d'un substituant Rs contenant un motif alkyle, alcényle, alcynyle, cycloalkyle ou cycloalcényle dans (Rsa) ou (Rsb) chaque motif de ce type est éventuellement substitué de nouveau sur un atome de carbone disponible par un ou plusieurs substituants choisis indépendamment parmi F, cl et Br et/ou par un groupe cyano ; et/ou lequel cycle est éventuellement substitué sur un atome d'azote disponible (à condition que le cycle ne soit pas ainsi quaternisé) par un (1-4C)alkyle ; ou HET est un cycle di-hydro-hétéroaryle à 6 chaînons lié par N contenant jusqu'à trois hétéroatomes d'azote au total (dont l'hétéroatome de liaison), lequel cycle est substitué sur un atome de C convenable, autre qu'un atome de C adjacent à l'atome de N lieur, par oxo ou thioxo et/ou lequel cycle est éventuellement substitué sur tout atome de C disponible, autre qu'un atome de C adjacent à l'atome de N lieur par un ou deux substituants Rs, où Rs est tel que défini précédemment, et/ou sur un atome d'azote disponible (à condition que le cycle ne soit pas ainsi quaternisé) par (1-4C)alkyle ; et où à chaque apparition de substituants alkyle, alcényle et cycloalkyle HET, chacun est éventuellement substitué par un ou plusieurs substituants choisi indépendamment parmi F, Cl et Br et/ou par un groupe cyano ;
Q est Q1 :- 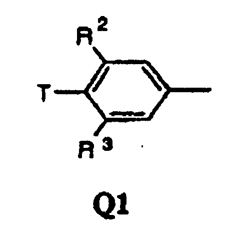 où R² et R³ sont indépendamment hydrogène ou fluoro ;
T est choisi dans les groupes suivants de formules (TAa1) à (TAa6) ci-dessous (où AR1, AR2, AR2a, AR2b, AR3, AR3a, AR3b, AR4, AR4a, CY1 et CY2 sont définis ci-dessous) ; 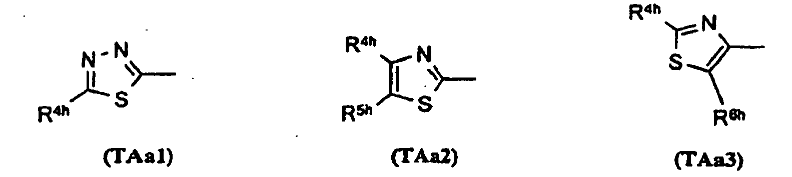 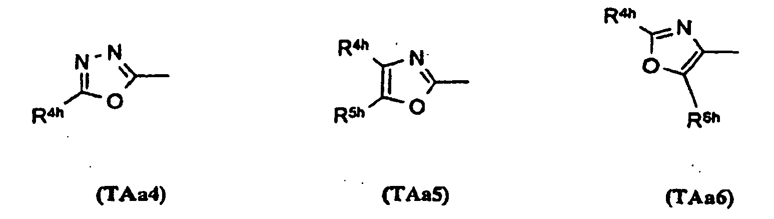 où
R^{6h} est l'hydrogène ou un (1-4C)alkyle, et R^{4h} et R^{5h} sont choisis indépendamment parmi l'hydrogène, un cyano, un (1-4C)alcoxycarbonyle, -CONRvRw, un hydroxy(1-4C)alkyle, un NRvRw(1-4C)alkyle, un -NRcRv(1-4C)alkyle ; où Rv est l'hydrogène ou un (1-4C)alkyle ; Rw est l'hydrogène ou un (1-4C)alkyle}
où Rc est choisi dans les groupes (Rc1) à (Rc5) :
***(Rc1)*** un (1-6C)alkyle {éventuellement substitué par un ou plusieurs groupes (1-4C)alcanoyle (dont une disubstitution géminée) et/ou éventuellement monosubstitué par un cyano, un (1-4C)alcoxy, un trifluorométhyle, un (1-4C)alcoxycarbonyle, un phényle (éventuellement substitué comme pour AR1 défini ci-après), un (1-4C)alkylS(O)_{q}- (q est 0, 1 ou 2) ; ou, sur tout sauf le premier atome de carbone de la chaîne (1-6C)alkyle, éventuellement substitué par un ou plusieurs groupes (dont une disubstitution géminée) choisis chacun indépendamment parmi un hydroxy et un fluoro, et/ou éventuellement monosubstitué par un oxo, -NRvRw [où Rv est l'hydrogène ou un (1-4C)alkyle ; Rw est l'hydrogène ou un (1-4C)alkyle], un (1-6C)-alcanoylamino, un (1-4C)alcoxycarbonylamino, un N-(1-4C)alkyl-N-(1-6C)alcanoylamino, un (1-4C)alkylS(O)ₚNH- ou un (1-4C)alkylS(O)ₚ₋((1-4C)alkyl)N- (p est 1 ou 2)} ;
**(*Rc2)*** R¹³CO-, R¹³SO₂- ou R¹³CS-,
où R¹³ est choisi parmi (Rc2a) à (Rc2e) .
***(Rc2a)*** AR1, AR2, AR2a, AR2b, AR3, AR3a, AR3b, AR4, AR4a, CY1, CY2 ;
***(Rc2b)*** l'hydrogène, un (1-4C)alcoxycarbonyle, un trifluorométhyle, -NRvRw [où Rv est l'hydrogène ou un (1-4C)alkyle ; Rw est l'hydrogène ou un (1-4C)-alkyle], un éthényle, un 2-(1-4C)alkyléthényle, un 2-cyanoéthényle, un 2-cyano-2-((1-4C)alkyl)-éthényle, un 2-nitroéthényle, un 2-nitro-2-((1-4C)alkyl)éthényle, un 2-((1-4C)alkylamino-carbonyl)éthényle, un 2-((1-4C)alcoxycarbonyl)-éthényle, un 2-(AR1)éthényle, un 2-(AR2)éthényle, un 2-(AR2a)éthényle ;
***(Rc2c)*** un (1-10C)alkyle
{éventuellement substitué par un ou plusieurs groupes (dont une disubstitution géminée) choisis chacun indépendamment parmi un hydroxy, un (1-10C)alcoxy, un (1-4C)alcoxy-(1-4C)alcoxy, un (1-4C)alcoxy-(1-4C)alcoxy-(1-4C)alcoxy, un (1-4C)-alcanoyle, un carboxy, un phosphoryle [-O-P(O)(OH)₂ et les dérivés mono- et di-(1-4C)alcoxy de celui-ci], un phosphiryle [-O-P(OH)₂ et les dérivés mono- et di-(1-4C)alcoxy de celui-ci], et un amino ; et/ou éventuellement substitué par un groupe choisi parmi un phosphonate, [un phosphono, -P(O) (OH)₂, et les dérivés mono- et di-(1-4C)alcoxy de celui-ci], un phosphinate [-P(OH)₂ et les dérivés mono- et di-(1-4C)alcoxy de celui-ci], un cyano, un halogéno, un trifluorométhyle, un (1-4C)alcoxycarbonyle, un (1-4C)alcoxy-(1-4C)alcoxy-carbonyle, un (1-4C) alcoxy-(1-4C) alcoxy- (1-4C)-alcoxycarbonyle, un (1-4C)alkylamino, un di-((1-4C)alkyl)amino, un (1-6C)alcanoylamino, un (1-4C)alcoxycarbonylamino, un N-(1-4C)alkyl-N-(1-6C)alcanoylamino, un (1-4C)alkylaminocarbonyle, un di-((1-4C)alkyl)aminocarbonyle, un (1-4C)-alkylS(O)pNH-, un (1-4C)alkylS(O)ₚ-((1-4C)alkyl)N-, un fluoro(1-4C)alkylS(O)pNH-, un fluoro(1-4C)-alkylS(O)p((1-4C)alkyl)N-, un (1-4C)alkylS(O)_{q}- [le groupe (1-4C)alkyle de (1-4C)alkylS(O)q- étant éventuellement substitué par un substituant choisi parmi un hydroxy, un (1-4C)alcoxy, un (1-4C)-alcanoyle, un phosphoryle [-O-P(O)(OH)₂, et les dérivés mono- et di-(1-4C)alcoxy de celui-ci], un phosphiryle [-O-P(OH)₂ et les dérivés mono- et di-(1-4C)alcoxy de celui-ci], un amino, un cyano, un halogéno, un trifluorométhyle, un (1-4C)alcoxy-carbonyle, un (1-4C)alcoxy-(1-4C)alcoxycarbonyle, un (1-4C)alcoxy-(1-4C)alcoxy-(1-4C)alcoxycarbonyle, un carboxy, un (1-4C)alkylamino, un di-((1-4C)alkyl)amino, un (1-6C)alcanoylamino, un (1-4C)alcoxycarbonylamino, un N-(1-4C)alkyl-N-(1-6C)alcanoylamino, un (1-4C)alkylaminocarbonyle, un di-((1-4C)alkyl)aminocarbonyle, un (1-4C)-alkylS(O)ₚNH-, un (1-4C)alkylS(O)ₚ-((1-4C)alkyl)N-, un (1-4C)alkylS(O)_{q}-, AR1-S(O)_{q}-, AR2-S(O)_{q}-, AR3-S(O)_{q}- et également les versions AR2a, AR2b, Ar3a et AR3b de groupes contenant AR2 et AR3], CY1, CY2, AR1, AR2, AR3, AR1-O-, AR2-O-, AR3-O-, AR1-S(O)_{q}-, AR2-S(O)_{q}-, AR3-S(O)_{q}-, AR1-NH-, AR2-NH-, AR3-NH (p est 1 ou 2 et q est 0, 1 ou 2), et également les versions AR2a, AR2b, Ar3a et AR3b de groupes contenant AR2 et AR3} ;
***(Rc2d)*** un R¹⁴C(O) O (1-6C) alkyle où R¹⁴ est AR1, AR2, (1-4C)alkylamino (le groupe (1-4C)alkyle étant éventuellement substitué par (1-4C)alcoxycarbonyle ou par carboxy), benzyloxy-(1-4C)alkyle ou (1-10C)alkyle {éventuellement substitué comme défini pour (Rc2c)} ;
***(Rc2e)*** R¹⁵O- où R¹⁵ est un benzyle, un (1-6C)alkyle {éventuellement substitué comme défini pour (Rc2c)}, CY1, CY2 ou AR2b ;
***(Rc3)*** l'hydrogène, un cyano, un 2-cyanoéthényle, un 2-cyano-2-((1-4C)alkyl)éthényle, un 2-((1-4C)-alkylaminocarbonyl)éthényle, un 2-((1-4C)-alcoxycarbonyl)éthényle, un 2-nitroéthényle, un 2-nitro-2-((1-4C)alkyl)éthényle, un 2-(AR1)éthényle, un 2-(AR2)éthényle, ou de formule **(Rc3a)** 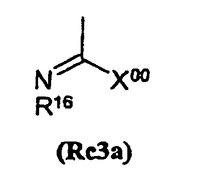 dans laquelle X⁰⁰ est -OR¹⁷, -SR¹⁷, -NHR¹⁷ et -N(R¹⁷)₂ ;
où R¹⁷ est l'hydrogène (lorsque X⁰⁰ est -NHR¹⁷ et -N(R¹⁷)₂), et R¹⁷ est un (1-4C)alkyle, un phényle ou AR2 (lorsque X⁰⁰ est -OR¹⁷, -SR¹⁷ et -NHR¹⁷) ; et R¹⁶ est un cyano, un nitro, un (1-4C)alkylsulfonyle, un (4-7C)cycloalkylsulfonyle, un phénylsulfonyle, un (1-4C)alcanoyle et un (1-4C)alcoxycarbonyle ;
***(Rc4)*** un trityle, AR1, AR2, AR2a, AR2b, AR3, AR3a, AR3b ;
***(Rc5)*** RdOC(Re)=CH(C=O)-, RfC(=O)C(=O)-, RgN=C(Rh)C(=O)- ou RiNHC(Rj)=CHC(=O)- où Rd est un (1-6C)alkyle ; Re est l'hydrogène ou un (1-6C)alkyle, ou Rd et Re forment ensemble une chaîne (3-4C)alkylène ; Rf est l'hydrogène, un (1-6C)alkyle, un hydroxy(1-6C)alkyle, un (1-6C)alcoxy(1-6C)alkyle, -NRvRw [où Rv est l'hydrogène ou un (1-4C)alkyle ; Rw est l'hydrogène ou un (1-4C)alkyle], un (1-6C)alcoxy, un (1-6C)alcoxy(1-6C)alcoxy, un hydroxy(2-6C)alcoxy, un (1-4C)alkylamino(2-6C)alcoxy, un di(1-4C)alkylamino(2-6C)alcoxy ; Rg est un (1-6C)alkyle, un hydroxy ou un (1-6C)alcoxy ; Rh est l'hydrogène ou un (1-6C)alkyle ; Ri est l'hydrogène, un (1-6C)alkyle, AR1, AR2, AR2a, AR2b et Rj est l'hydrogène ou un (1-6C)alkyle ;
où
**AR1** est un phényle éventuellement substitué ou un naphtyle éventuellement substitué ;
**AR2** est un cycle hétéroaryle monocyclique à 5 ou 6 chaînons totalement insaturé (c'est-à-dire avec le degré d'insaturation maximum) éventuellement substitué, contenant jusqu'à quatre hétéroatomes choisis indépendamment parmi 0, N et S (mais ne contenant pas de liaisons 0-0, O-S ou S-S quelconques), et lié par un atome de carbone du cycle, ou un atome d'azote du cycle si le cycle n'est pas ainsi quaternisé ;
**AR2a** est une version partiellement hydrogénée d'AR2 (c'est-à-dire les systèmes AR2 conservant un certain degré, mais pas la totalité du degré d'insaturation), lié par un atome de carbone du cycle ou lié par un atome d'azote du cycle si le cycle n'est pas ainsi quaternisé ;
**AR2b** est une version totalement hydrogénée d'AR2 (c'est-à-dire des systèmes AR2 n'ayant pas d'insaturation), lié par un atome de carbone du cycle ou lié par un atome d'azote du cycle ;
**AR3** est un cycle hétéroaryle bicyclique à 8, 9 ou 10 chaînons totalement insaturé (c'est-à-dire avec le degré d'insaturation maximum) éventuellement substitué, contenant jusqu'à quatre hétéroatomes choisis indépendamment parmi O, N et S (mais ne contenant pas de liaisons O-O, O-S ou S-S quelconques), et lié par un atome de carbone du cycle dans l'un ou l'autre des cycles comprenant le système bicyclique ;
**AR3a** est une version partiellement hydrogénée d'AR3 (c'est-à-dire les systèmes AR3 conservant un certain degré, mais pas la totalité du degré d'insaturation), lié par un atome de carbone du cycle ou lié par un atome d'azote du cycle si le cycle n'est pas ainsi quaternisé, dans l'un ou l'autre des cycles comprenant le système bicyclique ;
**AR3b** est une version totalement hydrogénée d'AR3 (c'est-à-dire des systèmes AR3 n'ayant pas d'insaturation), lié par un atome de carbone du cycle ou lié par un atome d'azote du cycle, dans l'un ou l'autre des cycles comprenant le système bicyclique ;
AR4 est un cycle hétéroaryle tricyclique à 13 ou 14 chaînons totalement insaturé (c'est-à-dire avec le degré d'insaturation maximum) éventuellement substitué, contenant jusqu'à quatre hétéroatomes choisis indépendamment parmi O, N et S (mais ne contenant pas de liaisons O-O, O-S ou S-S quelconques), et lié par un atome de carbone du cycle dans l'un quelconque des cycles comprenant le système tricyclique ;
**AR4a** est une version partiellement hydrogénée d'AR4 (c'est-à-dire les systèmes AR4 conservant un certain degré, mais pas la totalité du degré d'insaturation), lié par un atome de carbone du cycle ou lié par un atome d'azote du cycle si le cycle n'est pas ainsi quaternisé, dans l'un quelconque des cycles comprenant le système tricyclique ;
CY1 est un cycle cyclobutyle, cyclopentyle ou cyclohexyle éventuellement substitué ;
**CY2** est un cycle cyclopentényle ou cyclohexényle éventuellement substitué.

2. Composé de formule (IB), ou un sel pharmaceutiquement acceptable, ou un ester hydrolysable in vivo de celui-ci, 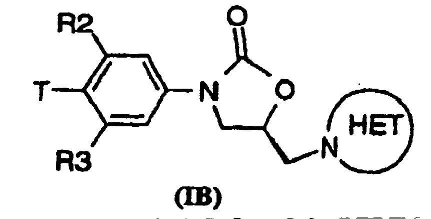 dans laquelle HET est un 1,2,3-triazole, un 1,2,4-triazole ou un tétrazole, ou HET est une version di-hydro de la pyrimidine, de la pyridazine, de la pyrazine, de la 1,2,3-triazine, de la 1,2,4-triazine, de la 1,3,5-triazine et de la pyridine ;
R² et R³ sont indépendamment l'hydrogène ou un fluoro ; et
T est choisi parmi (TAa1 à TAa6).

3. Composé de formule (IB) selon la revendication 2, ou un sel pharmaceutiquement acceptable, ou un ester hydrolysable in vivo de celui-ci, dans laquelle
HET est un 1,2,3-triazole, 1,2,4-triazole ou un tétrazole ;
R² et R³ sont indépendamment l'hydrogène ou un fluoro ; et
T est choisi parmi (TAa1 & 2).

4. Composé de formule (IB) selon la revendication 3, ou un sel pharmaceutiquement acceptable, ou un ester hydrolysable in vivo de celui-ci,
dans laquelle HET est un 1,2,3-triazol-1-yle, un 1,2,4-triazol-1-yle ou un tétrazol-2-yle ;
R² et R³ sont indépendamment l'hydrogène ou un fluoro ; et
T est choisi parmi (TAa1 & 2).

5. Composé de formule (I) selon l'une quelconque des revendications précédentes, ou un sel pharmaceutiquement acceptable, ou un ester hydrolysable in vivo de celui-ci, dans laquelle Rs est choisi parmi un fluorométhyle, un difluorométhyle, un trifluorométhyle, un chlorométhyle, un bromométhyle, un cyanométhyle, un cyano, un amino, un azido, un alkylthioalkyle tel qu'un méthylthiométhyle et un 2-propynyle.

6. Composé de formule (I) selon la revendication 1, ou un sel pharmaceutiquement acceptable, ou un ester hydrolysable in vivo de celui-ci, destiné à être utilisé comme médicament.

7. Utilisation d'un composé de formule (I) selon la revendication 1, ou un sel pharmaceutiquement acceptable, ou un ester hydrolysable in vivo de celui-ci, dans la fabrication d'un médicament destiné à être utilisé dans la production d'un effet antibactérien chez un animal à sang chaud.

8. Composition pharmaceutique comprenant un composé de formule (I) selon la revendication 1, ou un sel pharmaceutiquement acceptable ou un ester hydrolysable in vivo de celui-ci, et un diluant ou un support pharmaceutiquement acceptable.

9. Composé de formule (I), ou un sel pharmaceutiquement acceptable, ou un ester hydrolysable in vivo de celui-ci, destiné à être utilisé dans une méthode de traitement de corps humain ou animal par thérapie.

10. Procédé de fabrication d'un composé de formule (I) comprenant un ou plusieurs des procédés (a) à (i) ci-dessous :
(a) la modification d'un substituant ou 1`introduction d'un substituant dans un autre composé de formule (I) ;
(b) la réaction d'un composé de formule (II) 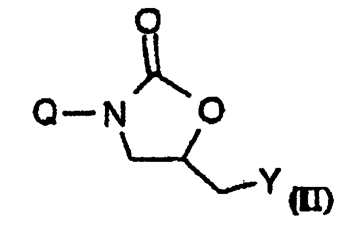 dans laquelle Y est un groupe déplaçable avec un composé de formule (III) :
**HET** **(III)**
dans laquelle HET est une forme base libre HET-H ou HET-anion formé à partir de la forme base libre ;
(c) la réaction d'un composé de formule (IV) :
**Q-Z** **(IV)**
dans laquelle Z est un groupe isocyanate, amine ou uréthane, avec un époxyde de formule (V) : 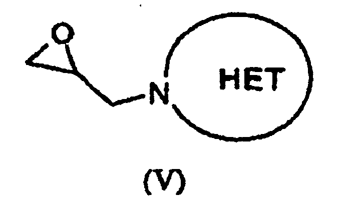
(d) la réaction d'un composé de formule (VI) : 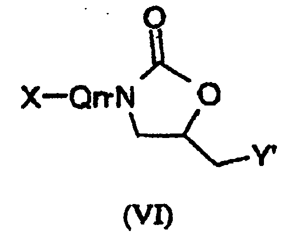 dans laquelle Y' est un groupe HET tel que défini précédemment, X est un substituant remplaçable localisé en une position substituée par T dans l'un quelconque des modes de mise en oeuvre aromatique Q1-Q8 de Qn tel que défini précédemment pour Q, mais avec X à la place du substituant T, avec un composé de formule (VII) :
**T-X'** **(VII)**
dans laquelle T-X' est un hétérocycle à 5 chaînons avec 1-3 hétéroatomes pris en combinaison parmi O, N et S, et X' est un substituant remplaçable lié par C ; où les substituants X et X' sont choisis pour être des pairs de substituants complémentaires connus dans la technique pour être convenables comme substrats complémentaires pour les réactions de couplage catalysées par les métaux de transition tels que le palladium(0) ;
(e) la réaction d'un composé de formule (VIII) 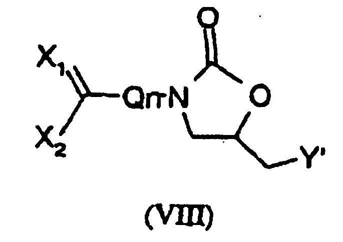 dans laquelle Y' est un groupe HET tel que défini précédemment et X1 et X2 sont ici des hétéroatomes indépendamment et éventuellement substitués pris en combinaison parmi O, N et S de sorte que C(X1)X2 constitue un substituant qui est un substituant dérivé d'acide carboxylique localisé en une position substituée par T dans l'un quelconque des modes de mise en oeuvre aromatique Q1-Q10 de Qn tel que défini précédemment pour Q avec un composé de formule (IX) et X3 et X4 sont des hétéroatomes indépendamment et éventuellement substitués pris en combinaison parmi O, N et S : 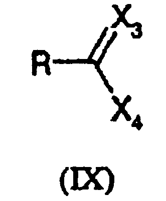 et où l'un de C(X1)X2 et C(X3)X4 constitue un hydrazide, un thiohydrazide ou une amidrazone éventuellement substitué, et l'autre de C(X1)X2 et C(X3)X4 constitue un agent d'acylation, de thioacylation ou d'imidoylation éventuellement substitué de sorte que C(X1)X2 et C(X3)X4 puissent être condensés ensemble pour former un hétérocycle à 5 chaînons contenant 3 hétéroatomes pris en combinaison parmi O, N et S, par exemple un thiadiazole, par des méthodes bien connues dans la technique ;
(f) la réaction d'un composé de formule (X) : 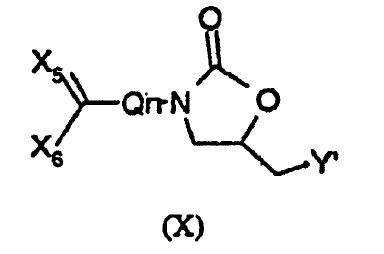 dans laquelle Y' est un groupe HET tel que défini précédemment et C(X5)X6 constitue un substituant localisé en une position substituée par T dans l'un quelconque des modes de mise en oeuvre aromatique Q1-Q8 de Qn tel que défini précédemment pour Q avec un composé de formule (XI) : 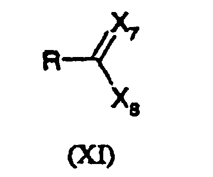 où l'un de C(X5)X6 et C(X7)X8 constitue une cétone éventuellement substituée en alpha par un groupe partant, où le groupe partant est par exemple un groupe halogéno ou un groupe (alkyl ou aryl)-sulfonyloxy, et l'autre de C(X5)X6 et C(X7)X8 constitue un amide, un thioamide ou une amidine éventuellement substitué de sorte que C(X5)X6 et C(X7)X8 soient des groupes qui puissent être condensés ensemble pour former un hétérocycle à 5 chaînons contenant 2 hétéroatomes pris en combinaison parmi 0, N et S, par exemple un thiazole, par des méthodes bien connues dans la technique ;
(g) pour HET en tant que 1,2,3-triazoles éventuellement substitués, les composés de formule (I) peuvent être préparés par cycloaddition par l'intermédiaire de l'azide (où par exemple Y dans (II) est un azide) à des acétylènes, ou à des équivalents d'acétylènes tels que des cyclohexa-1,4-diènes éventuellement substitués ou des éthylènes éventuellement substitués portant des substituants éliminables tels qu'un arylsulfonyle ;
(h) pour HET en tant que 1,2,3-triazole substitué en position 4, les composés de formule (I) peuvent être préparés en faisant réagir des aminométhylisoxazolines avec des 1,1-dihalogénocétone-sulfonylhydrazones ;
(i) pour HET en tant que 1,2,3-triazole substitué en position 4, les composés de formule (I) peuvent également être préparés par réaction des azidométhyl-isoxazolines avec des alcynes terminaux en utilisant la catalyse au Cu(1) ; et ensuite le cas échéant : (i) l'élimination de tout groupe protecteur ; (ii) la formation d'un sel pharmaceutquement acceptable ; (iii) la formation d'un ester hydrolysable in vivo.

11. Composé choisi parmi
la (5*R*)-3-(3-fluoro-4-(5-cyano-1,3,4-thiadiazol-2-yl)phényl)-5-(1*H*-1,2,3-triazol-1-ylméthyl)-1,3-oxazolidin-2-one ;
la (*5R*)-3-(3-fluoro-4-(5-éthoxycarbonyl-1,3,4-thiadiazol-2-yl)phényl)-5-(1*H*-1,2,3-triazol-1-ylméthyl)-1,3-oxazolidin-2-one ;
la (*5R*)-3-(4-(5-aminométhyl)-1,3-thiazol-2-yl)-3-fluorophényl)-5-(1*H*-1,2,3-triazol-1-ylméthyl)-1,3-oxazolidin-2-one ;
la (*5R*)-3-(3-fluoro-4-(5-méthyl-1,3,4-thiadiazol-2-yl)phényl)-5-(1*H*-1,2,3-triazol-1-ylméthyl)-1,3-oxazolidin-2-one ;
la (*5R*)-3-(3-fluoro-4-(4-méthyl-1,3-thiazol-2-yl)phényl)-5-(1*H*-1,2,3-triazol-1-ylméthyl)-1,3-oxazolidin-2-one ;
la (*5R*)-3-(3-fluoro-4-(4-(trifluorométhyl)-1,3-thiazol-2-yl)phényl)-5-(1*H*-1,2,3-triazol-1-ylméthyl)-1,3-oxazolidin-2-one ; et
le (5-(2-fluoro-4-((*5R*)-5-(1*H*-1,2,3-triazol-1-ylméthyl)1,3-oxazolidin-2-on-3-yl)phényl)-1,3,4-thiadiazol-2-yl)acétonitrile ;
ou un sel pharmaceutiquement acceptable ou un ester hydrolysable in vivo de celui-ci.

## Patentansprüche

1. Verbindung der Formel (I) oder ein pharmazeutisch annehmbares Salz oder ein *in vivo* hydrolysierbarer Ester davon, 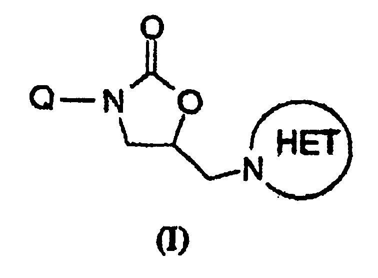 wobei:
HET ein N-gebundener 5-gliedriger vollständig oder teilweise ungesättigter heterocyclischer Ring ist, der entweder (i) 1 bis 3 weitere Stickstoff-Heteroatome, oder (ii) ein weiteres Heteroatom, ausgewählt aus O und S, zusammen mit einem wahlfreien weiteren Stickstoff-Heteroatom, enthält; wobei der Ring gegebenenfalls an einem C-Atom, und zwar nicht an einem C-Atom neben dem Verknüpfungs-N-Atom, durch eine Oxo- oder ThioxoGruppe substituiert ist; und/oder wobei der Ring gegebenenfalls an irgendeinem zugänglichen C-Atom, und zwar nicht an einem C-Atom neben dem Verknüpfungs-N-Atom, durch einen Substituenten Rs substituiert ist, wobei:
Rs ausgewählt ist aus der Gruppe:
(Rsa): Halogen, (1-4C)Alkoxy, (2-4C)Alkenyloxy, (2-4C)Alkenyl, (2-4C)Alkinyl, (3-6C)Cycloalkyl, (3-6C)Cycloalkenyl, Amino, (1-4C)Alkylamino, Di-(1-4C)-alkylamino, (2-4C)Alkenylamino, (1-4C)Alkylcarbonyl-amino, (1-4C)Alkylthiocarbonylamino, (1-4C)Alkyl-OCO-NH-, (1-4C)Alkyl-NH-CO-NH-, (1-4C)Alkyl-NH-CS-NH-, (1-4C)Alkyl-SO₂-NH- oder (1-4C)Alkyl-S(O)_{q} (wobei q 0, 1 oder 2 ist); oder
Rs ausgewählt ist aus der Gruppe:
(Rsb): (1-4C)Alkylrest, welcher gegebenenfalls durch einen Substituenten substituiert ist, der aus Hydroxy, (1-4C)Alkoxy, Amino, Cyano, Azido, (2-4C)Alkenyloxy, (1-4C)Alkylcarbonyl, (1-4C)-Alkoxycarbonyl, (1-4C)Alkylamino, (2-4C)Alkenyl-amino, (1-4C)Alkyl-SO₂-NH-, (1-4C)Alkylcarbonyl-amino, (1-4C)Alkylthiocarbonylamino, (1-4C)Alkyl-OCO-NH-, (1-4C)Alkyl-NH-CO-NH-, (1-4C)Alkyl-NH-CS-NH-, (1-4C)Alkyl-SO₂-NH-, (1-4C)Alkyl-S(O)_{q}- (wobei q 0, 1 oder 2 ist), (3-6C)Cycloalkyl, (3-6C)Cycloalkenyl, oder einem N-verknüpften 5-gliedrigen Heteroarylring ausgewählt ist, wobei der Ring entweder (i) 1 bis 3 weitere Stickstoff-Heteroatome oder (ii) ein weiteres Heteroatom, das aus O und S ausgewählt ist, zusammen mit einem wahlfreien weiteren Stickstoff-Heteroatom enthält; wobei der Ring gegebenenfalls an einem Kohlenstoffatom durch eine Oxo- oder Thioxogruppe substituiert ist; und/oder der Ring gegebenenfalls an einem Kohlenstoffatom durch 1 oder 2 (1-4C)Alkylreste und/oder an einem zugänglichen Stickstoffatom (mit der Maßgabe, dass der Ring nicht hierdurch quaternisiert wird) durch (1-4C)Alkyl substituiert ist;
und wobei bei jedem Vorkommen eines Rs-Substituenten, der eine Alkyl-, Alkenyl-, Alkinyl-, Cycloalkyl- oder Cycloalkenyl-Einheit in (Rsa) oder (Rsb) enthält, eine jede solche Einheit an einem zugänglichen Kohlenstoffatom durch eine oder mehrere Substituenten, welche aus F, Cl und Br unabhängig ausgewählt sind, und/oder durch eine Cyanogruppe gegebenenfalls weiter substituiert ist; und/oder wobei der Ring an einem zugänglichen Stickstoffatom (mit der Maßgabe, dass der Ring **dadurch** nicht quaternisiert wird) durch (1-4C)Alkyl gegebenenfalls substituiert ist; oder HET ein N-verknüpfter 6-gliedriger Dihydroheteroarylring ist, der insgesamt bis zu drei Stickstoff-Heteroatome enthält (einschließlich des Verbindungs-Heteroatoms), wobei der Ring an einem geeigneten C-Atom, und zwar nicht an einem C-Atom neben dem Verbindungs-N-Atom, durch Oxo oder Thioxo substituiert ist, und/oder wobei der Ring an einem zugänglichen C-Atom, und zwar nicht an einem C-Atom neben dem Verbindungs-N-Atom, durch ein oder zwei Substituenten Rs, wobei Rs die vorstehend definierte Bedeutung hat, und/oder an einem zugänglichen Stickstoffatom (mit der Maßgabe, dass der Ring **dadurch** nicht quaternisiert wird) durch (1-4C)Alkyl gegebenenfalls substituiert ist; und wobei bei jedem Vorkommen von Alkyl-, Alkenyl- und Cycloalkyl-HET-Substituenten, ein jeder gegebenenfalls mit einem oder mehreren Substituenten, die unabhängig voneinander aus F, Cl und Br ausgewählt sind, und/oder durch eine Cyanogruppe substituiert ist;
Q für Q1 steht: 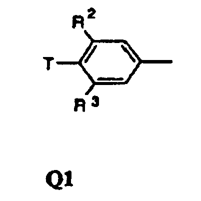
wobei:
R² und R³ unabhängig Wasserstoff oder Fluor sind;
T aus den folgenden Gruppen der Förmel (TAa1) bis (TAa6) unten (wobei AR1, AR2, AR2a, AR2b, AR3,
AR3a, AR3b, AR4, AR4a, CY1 und CY2 die nachstehend definierte Bedeutung haben) ausgewählt ist; 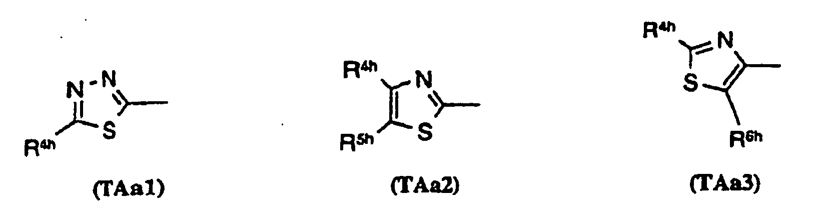 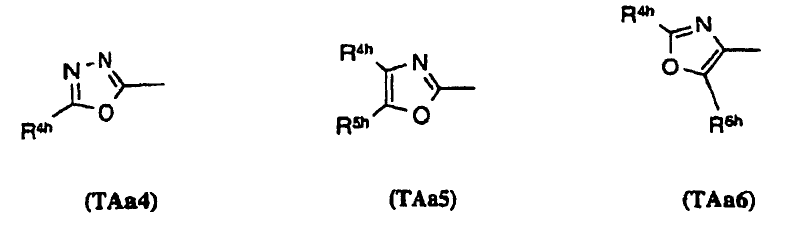
wobei:
R^{6h} Wasserstoff oder (1-4C)Alkyl ist, und R^{4h} und R^{5h} unabhängig aus Wasserstoff, Cyano, (1-4C)Alkoxycarbonyl, -CONRvRw, Hydroxy(1-4C)alkyl, NRvRw(1-4C)Alkyl, -NRcRv(1-4C)alkyl ausgewählt sind; wobei Rv Wasserstoff oder (1-4C)Alkyl ist; Rw Wasserstoff oder (1-4C)Alkyl ist;
wobei Rc aus den Gruppen (Rc1) bis (Rc5) ausgewählt ist:-
***(Rc1)*** (1-6C)Alkyl (gegebenenfalls durch einen oder mehrere (1-4C)Alkanoylreste substituiert (einschließlich geminaler Disubstitution) und/oder gegebenenfalls durch Cyano, (1-4C)Alkoxy, Trifluormethyl, (1-4C)Alkoxycarbonyl, Phenyl (gegebenenfalls substituiert wie für AR1 nachstehend definiert), (1-4C)AlkylS(O)_{q}- (q ist 0, 1 oder 2) monosubstituiert; oder an einem beliebigen Kohlenstoffatom außer am ersten der (1-6C)Alkylkette gegebenenfalls durch eine oder mehrere Gruppen (einschließlich geminaler Disubstitution) substituiert, welche jeweils unabhängig aus Hydroxy und Fluor ausgewählt sind, und/oder gegebenenfalls monosubstituiert durch Oxo, -NRvRw [wobei Rv Wasserstoff oder (1-4C)Alkyl ist; Rw Wasserstoff oder (1-4C)Alkyl ist], (1-6C)Alkanoylamino, (1-4C)Alkoxycarbonylamino, N- (1-4C)Alkyl-N-(1-6C)alkanoylamino, (1-4C)AlkylS(O)ₚNH-oder (1-4C)AlkylS(O)ₚ-((1-4C)Alkyl)N- (p ist 1 oder 2)};
***(Rc2)*** R¹³CO-, R¹³ SO₂- oder R₁₃CS-,
wobei R¹³ aus (Rc2a) bis (Rc2e) ausgewählt ist:-
***(Rc2a)*** AR1, AR2, AR2a, AR2b, AR3, AR3a, AR3b, AR4, AR4a, CY1, CY2;
***(Rc2b)*** Wasserstoff, (1-4C)Alkoxycarbonyl, Trifluormethyl, -NRvRw [wobei Rv Wasserstoff oder (1-4C)Alkyl ist; Rw Wasserstoff oder (1-4C)Alkyl ist], Ethenyl, 2-(1-4C)Alkylethenyl, 2-Cyanoethenyl, 2-Cyano-2-((1-4C)alkyl)ethenyl, 2-Nitroethenyl, 2-Nitro-2-((1-4C)alkyl)ethenyl, 2-((1-4C)Alkylaminocarbonyl)ethenyl, 2-((1-4C)Alkoxycarbonyl)ethenyl, 2-(AR1)Ethenyl, 2-(AR2)Ethenyl, 2-(AR2a)Ethenyl;
***(Rc2c)*** (1-10C)Alkyl (gegebenenfalls substituiert durch eine oder mehrere Gruppen (einschließlich geminaler Disubstitution), die jeweils unabhängig aus Hydroxy, (1-10C)Alkoxy, (1-4C) Alkoxy-(1-4C)alkoxy, (1-4C)Alkoxy-(1-4C)alkoxy-(1-4C)alkoxy, (1-4C)Alkanoyl, Carboxy, Phosphoryl [-O-P(O) (OH)₂, und Mono- und Di-(1-4C)alkoxy-Derivaten davon], Phosphiryl [-O-P(OH)₂ und Mono- und Di-(1-4C)alkoxy-Derivaten davon] und Amino ausgewählt sind; und/oder gegebenenfalls substituiert durch eine Gruppe, die aus Phosphonat [Phosphono, - P(O) (OH)₂, und Mono- und Di-(1-4C)alkoxy-Derivaten davon], Phosphinat [-P(OH)₂ und Mono- und Di-(1-4C)alkoxy-Derivaten davon], Cyano, Halogen, Trifluormethyl, (1-4C)Alkoxycarbonyl, (1-4C)Alkoxy-(1-4C)alkoxycarbonyl, (1-4C)Alkoxy-(1-4C)alkoxy-(1-4C)alkoxycarbonyl, (1-4C)Alkylamino, Di((1-4C)alkyl)amino, (1-6C)Alkanoylamino, (1-4C)Alkoxycarbonylamino, N-(1-4C)Alkyl-N-(1-6C)alkanoyl-amino, (1-4C)Alkylaminocarbonyl, Di((1-4C)alkyl)-aminocarbonyl, (1-4C)AlkylS(O)ₚNH-, (1-4C)AlkylS(O)ₚ₋((1-4C)alkyl)N-, Fluor(1-4C)alkylS(O)ₚNH-, Fluor(1-4C)alkylS(O)ₚ((1-4C)alkyl)N-, (1-4C)AlkylS(O)_{q}- [wobei der (1-4C)Alkylrest von (1-4C)AlkylS(O)_{q-}gegebenenfalls durch einen Substituenten, ausgewählt aus Hydroxy, (1-4C)Alkoxy, (1-4C)Alkanoyl, Phosphoryl [-O-P(O)(OH)₂, und Mono- und Di-(1-4C)alkoxy-Derivaten davon], Phosphiryl [-O-P(OH)₂ und Mono- und Di-(1-4C)alkoxy-Derivaten davon], Amino, Cyano, Halogen, Trifluormethyl, (1-4C)Alkoxycarbonyl, (1-4C)Alkoxy-(1-4C)alkoxycarbonyl, (1-4C)Alkoxy-(1-4C)alkoxy-(1-4C)alkoxycarbonyl, Carboxy, (1-4C)Alkylamino, Di((1-4C)alkyl)-amino, (1-6C)Alkanoylamino, (1-4C)Alkoxycarbonylamino, N-(1-4C)Alkyl-N-(1-6C)alkanoylamino, (1-4C)Alkylaminocarbonyl, Di((1-4C)alkyl)aminocarbonyl, (1-4C)AlkylS(O)ₚNH-, (1-4C)AlkylS(O)ₚ-((1-4C)alkyl)N-, (1-4C) AlkylS (O)_{q}-, AR1-S (O)_{q}-, AR2-S(O)_{q}-, AR3-S(O)_{q}- und ebenfalls durch die AR2a-, AR2b-, AR3a- und AR3b-Versionen von AR2- und AR3-haltigen Gruppen substituiert ist], CY1, CY2, AR1, AR2, AR3, AR1-O-, AR2-O-, AR3-O-, AR1-S(O)_{q}-, AR2-S(O)_{q}-, AR3-S(O)_{q}-, AR1-NH-, AR2-NH, AR3-NH- (p ist 1 oder 2 und q ist 0, 1 oder 2) und ebenfalls den AR2a-, AR2b-, AR3a- und AR3b-Versionen von AR2-und AR3-haltigen Gruppen ausgewählt ist};
***(Rc2d)*** R¹⁴C (O)O(1-6C)Alkyl, wobei R¹⁴ AR1, AR2, (1-4C)Alkylamino (wobei der (1-4C)Alkylrest gegebenenfalls durch (1-4C)Alkoxycarbonyl oder durch Carboxy substituiert ist), Benzyloxy-(1-4C)alkyl oder (1-10C)Alkyl {gegebenenfalls substituiert wie für (Rc2c) definiert} ist;
***(Rc2e)*** R¹⁵O-, wobei R¹⁵ Benzyl, (1-6C)Alkyl {gegebenenfalls substituiert, wie für (Rc2c) definiert}, CY1, CY2 oder AR2b ist;
***(Rc3)*** Wasserstoff, Cyano, 2-Cyanoethenyl, 2-Cyano-2-((1-4C)alkyl)ethenyl, 2-((1-4C)Alkylaminocarbonyl)ethenyl, 2-((1-4C)Alkoxycarbonyl)ethenyl, 2-Nitroethenyl, 2-Nitro-2((1-4C)alkyl)ethenyl, 2-(AR1) Ethenyl, 2-(AR2)Ethenyl ist oder die Formel ***(Rc3a)*** hat: 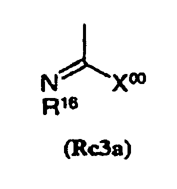 wobei X⁰⁰ -OR¹⁷, -SR¹⁷, -NHR¹⁷ und -N(R¹⁷)₂ ist;
wobei R¹⁷ Wasserstoff ist (wenn X⁰⁰ -NHR¹⁷ und N(R¹⁷)2 ist), und R¹⁷ (1-4C)Alkyl, Phenyl oder AR2 ist (wenn X⁰⁰ -OR¹⁷, -SR¹⁷ und -NHR¹⁷ ist); und R¹⁶ Cyano, Nitro, (1-4C)Alkylsulfonyl, (4-7C)-Cycloalkylsulfonyl, Phenylsulfonyl, (1-4C)Alkanoyl und (1-4C)Alkoxycarbonyl ist;
***(Rc4)*** Trityl, AR1, AR2, AR2a, AR2b, AR3, AR3a, AR3b;
***(Rc5)*** RdOC(Re)=CH(C=O)-, RfC(=O)C(=O)-, RgN=C(Rh)C(=O)- oder RiNHC(Rj)=CHC(=O)-, wobei Rd (1-6C)Alkyl ist; Re Wasserstoff oder (1-6C)Alkyl ist, oder Rd und Re zusammen eine (3-4C)Alkylenkette bilden; Rf Wasserstoff, (1-6C)Alkyl, Hydroxy(1-6C)alkyl, (1-6C)Alkoxy(1-6C)alkyl, -NRvRw [wobei Rv Wasserstoff oder (1-6C)Alkyl ist; Rw Wasserstoff oder (1-4C)Alkyl ist], (1-6C)Alkoxy, (1-6C)Alkoxy(1-6C)alkoxy, Hydroxy(2-6C)alkoxy, (1-4C)Alkylamino(2-6C)alkoxy, Di-(1-4C)alkylamino(2-6C)alkoxy ist; Rg (1-6C)Alkyl, Hydroxy oder (1-6C)Alkoxy ist; Rh Wasserstoff oder (1-6C)Alkyl ist; Ri Wasserstoff, (1-6C)Alkyl, AR1, AR2, AR2a, AR2b ist, und Rj Wasserstoff oder (1-6C)Alkyl ist;
wobei:
**AR1** ein gegebenenfalls substituiertes Phenyl oder gegebenenfalls substituiertes Naphthyl ist;
**AR2** ein gegebenenfalls substituierter 5- oder 6-gliedriger vollständig ungesättigter (d.h. mit dem maximalen Grad an Ungesättigtheit) monocyclischer Heteroarylring ist, der bis zu vier Heteroatome enthält, die unabhängig aus O, N und S ausgewählt sind (jedoch ohne irgendeine O-O-, 0-S- oder S-S-Bindung), und die über ein Ring-Kohlenstoffatom oder Ring-Stickstoffatom gebunden sind, wenn der Ring **dadurch** nicht quaternisiert wird;
**AR2a** eine partiell hydrierte Version von AR2 ist (d.h AR2-Systeme, die einen gewissen, aber nicht den vollständigen Grad an Ungesättigtheit beibehalten), die über ein Ring-Kohlenstoffatom gebunden ist oder über ein Ring-Stickstoffatom gebunden ist, wenn der Ring **dadurch** nicht quaternisiert wird;
**AR2b** eine vollständig hydrierte Version von AR2 ist (d.h. AR2-Systeme ohne Ungesättigtheit), die über ein Ring-Kohlenstoffatom gebunden ist oder über ein Ring-Stickstoffatom gebunden ist;
**AR3** ein gegebenenfalls substituierter 8-, 9- oder 10-gliedriger, vollständig ungesättigter (d.h. mit dem maximalen Grad an Ungesättigtheit) bicyclischer Heteroarylring ist, der bis zu vier Heteroatome enthält, die unabhängig aus O, N und S ausgewählt sind (jedoch ohne irgendeine O-O-, O-S- oder S-S-Bindung), und die in einem der Ringe, die das bicyclische System umfassen, über ein Ring-Kohlenstoffatom gebunden sind;
**AR3a** eine partiell hydrierte Version von AR3 ist (d.h. AR3-Systeme, die einen gewissen aber nicht den vollständigen Grad an Ungesättigtheit beibehalten), die in einem der Ringe, die das bicyclische System umfassen, über ein Ring-Kohlenstoffatom gebunden ist oder über ein Ring-Stickstoffatom gebunden ist, wenn der Ring **dadurch** nicht quaternisiert wird;
**AR3b** eine vollständig hydrierte Version von AR3 ist (d.h. AR3-Systeme, die keine Ungesättigtheit aufweisen), die in einem der Ringe, die das bicyclische System umfassen, über ein Ring-Kohlenstoffatom gebunden ist oder über ein Ring-Stickstoffatom gebunden ist;
**AR4** ein gegebenenfalls substituierter 13- oder 14-gliedriger, vollständig ungesättigter (d.h. mit dem maximalen Grad an Ungesättigtheit) tricyclischer Heteroarylring mit bis zu vier Heteroatomen ist, die unabhängig aus O, N und S ausgewählt sind (jedoch ohne irgendeine O-O-, O-S- oder S-S-Bindung), und die in einem der Ringe, die das tricyclische System umfassen, über ein Ring-Kohlenstoffatom gebunden sind;
**AR4a** eine partiell hydrierte Version von AR4 ist (d.h. AR4-Systeme, die einen gewissen, aber nicht den vollständigen Grad an Ungesättigtheit beibehalten), die in einem der Ringe, die das tricyclische System umfassen, über ein Ring-Kohlenstoffatom gebunden ist oder über ein Ring-Stickstoffatom gebunden ist, wenn der Ring **dadurch** nicht quaternisiert wird;
**CY1** ein gegebenenfalls substituierter Cyclobutyl-, Cyclopentyl- oder Cyclohexylring ist;
**CY2** ein gegebenenfalls substituierter Cyclopentenyl- oder Cyclohexenylring ist.

2. Verbindung der Formel (IB) oder ein pharmazeutisch annehmbares Salz oder ein *in vivo* hydrolysierbarer Ester davon, 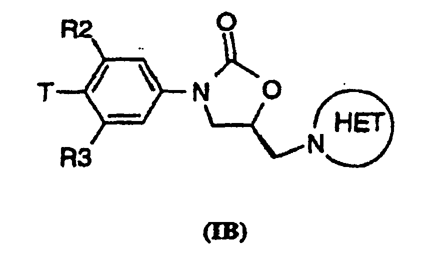 wobei HET 1,2,3-Triazol, 1,2,4-Triazol oder Tetrazol ist oder HET eine Dihydro-Version von Pyrimidin, Pyridazin, Pyrazin, 1,2,3-Triazin, 1,2,4-Triazin, 1,3,5-Triazin und Pyridin ist; R² und R³ unabhängig Wasserstoff oder Fluor sind; und
T aus (TAa1 bis TAa6) ausgewählt ist.

3. Verbindung der Formel (IB) nach Anspruch 2 oder ein pharmazeutisch annehmbares Salz oder ein in vivo hydrolysierbarer Ester davon, wobei:
HET 1,2,3-Triazol, 1,2,4-Triazol oder Tetrazol ist;
R² und R³ unabhängig Wasserstoff oder Fluor sind; und
T aus (TAa1 & 2) ausgewählt ist.

4. Verbindung der Formel (IB) nach Anspruch 3 oder ein pharmazeutisch annehmbares Salz oder ein in vivo hydrolysierbarer Ester davon, wobei:
HET 1,2,3-Triazol-1-yl, 1,2,4-Triazol-1-yl oder Tetrazol-2-yl ist;
R² und R³ unabhängig Wasserstoff oder Fluor sind; und
T aus (TAa1 & 2) ausgewählt ist.

5. Verbindung der Formel (I) nach einem vorhergehenden Anspruch oder ein pharmazeutisch annehmbares Salz oder ein in vivo hydrolysierbarer Ester davon, wobei Rs aus Fluormethyl, Difluormethyl, Trifluormethyl, Chlormethyl, Brommethyl, Cyanomethyl, Cyano, Amino, Azido, Alkylthioalkyl, wie Methylthiomethyl, und 2-Propinyl ausgewählt ist.

6. Verbindung der Formel (I) nach Anspruch 1 oder ein pharmazeutisch annehmbares Salz oder ein *in vivo* hydrolysierbarer Ester davon, zur Verwendung als Medikament.

7. Verwendung einer Verbindung der Formel (I) nach Anspruch 1 oder eines pharmazeutisch annehmbaren Salzes oder eines in vivo hydrolysierbaren Esters davon, bei der Herstellung eines Medikamentes zur Verwendung bei der Erzeugung einer antibakteriellen Wirkung in einem warmblütigen Tier.

8. Pharmazeutische Zusammensetzung, umfassend eine Verbindung der Formel (I) nach Anspruch 1 oder ein pharmazeutisch annehmbares Salz oder einen in vivo hydrolysierbaren Ester davon und ein pharmazeutisch annehmbares Verdünnungsmittel oder einen pharmazeutisch annehmbaren Träger.

9. Verbindung der Formel (I) oder ein pharmazeutisch annehmbares Salz oder ein *in vivo* hydrolysierbarer Ester davon zur Verwendung bei einem Verfahren zur Behandlung des Körpers von Mensch oder Tier durch Therapie.

10. Verfahren zur Herstellung einer Verbindung der Formel (I), umfassend ein oder mehrere der Verfahren (a) bis (i) unten:
(a) durch Modifizieren eines Substituenten in einer oder Einbringen eines Substituenten in eine andere Verbindung der Formel (I);
(b) durch Umsetzen einer Verbindung der Formel (II): 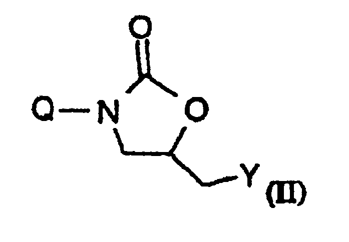 wobei Y eine austauschbare Gruppe ist, mit einer Verbindung der Formel (III):
HET (III)
wobei HET eine HET-H-freie Basenform oder ein HET-Anion ist, das aus der freien Basenform gebildet wurde;
(c) durch Umsetzen einer Verbindung der Formel (IV):
Q-Z (IV)
wobei Z eine Isocyanat-, Amin- oder Urethan-Gruppe ist, mit einem Epoxid der Formel (V): 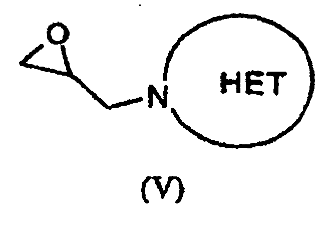
(d) durch Umsetzen einer Verbindung der Formel (VI): 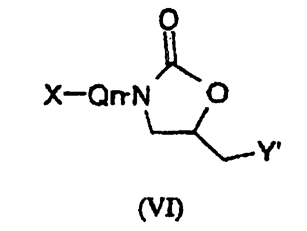 wobei Y' eine Gruppe HET wie vorstehend definiert ist, X ein ersetzbarer Substituent ist, der sich an einer Stelle befindet, die in einer der aromatischen Ausführungsformen Q1 bis Q8 von Qn, wie vorstehend für Q definiert, durch T substituiert ist, jedoch mit X statt mit dem Substituenten T, mit einer Verbindung der Formel (VII):
T-X' (VII)
wobei T-X' ein 5-gliedriger Heterozyklus mit 1 bis 3 Heteroatomen ist, die in Kombination aus O, N und S gezogen werden, und X' ein austauschbarer C-gebundener Substituent ist; wobei die Substituenten X und X' so ausgewählt sind, dass sie komplementäre Substituentenpaare sind, von denen im Stand der Technik bekannt ist, dass sie sich als komplementäre Substrate für Kopplungsreaktionen eignen, die durch Übergangsmetalle, wie Palladium(0), katalysiert werden;
(e) durch Umsetzen einer Verbindung der Formel (VIII): 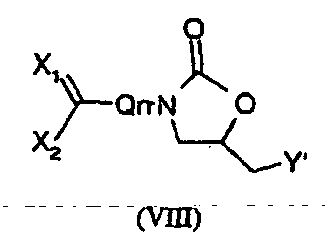 wobei Y' eine Gruppe HET wie hier vorstehend definiert ist, und X1 und X2 hier unabhängig gegebenenfalls substituierte Heteroatome sind, die in Kombination aus O, N und S gezogen werden, so dass C(X1)X2 einen Substituenten ausmacht, der ein Carbonsäurederivat-Substituent ist, der sich an einer Stelle befindet, die in einer der aromatischen Ausführungsformen Q1 bis Q10 von Qn, wie vorstehend für Q definiert, durch T substituiert ist, mit einer Verbindung der Formel (IX), und X3 und X4 sind unabhängig gegebenenfalls substituierte Heteroatome, die in Kombination aus O, N und S gezogen werden: 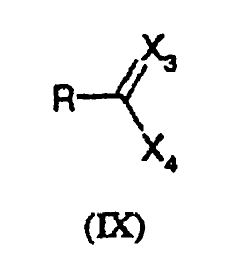 und wobei einer der Reste C(X1)X2 und C(X3)X4 ein gegebenenfalls substituiertes Hydrazid, Thiohydrazid oder Amidrazon ausmacht und der andere der Reste C(X1)X2 und C(X3)X4 ein gegebenenfalls substituiertes Acylierungs-, Thioacylierungs- oder Imidoylierungsmittel ausmacht, so dass C(X1)X2 und C(X3)X4 miteinander kondensiert sein können, so dass sie einen 5-gliedrigen Heterozyklus bilden, der 3 Heteroatome enthält, die in Kombination aus O, N und S gezogen werden, beispielsweise Thiadiazol, durch Verfahren, die im Stand der Technik bekannt sind;
(f) durch Umsetzen einer Verbindung der Formel (X): 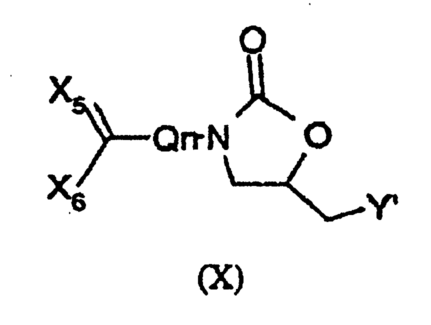 wobei Y' eine Gruppe HET ist, wie sie hier vorstehend definiert ist, und C(X5)X6 einen Substituenten ausmacht, der sich an einer Stelle befindet, die in einer der aromatischen Ausführungsformen Q1 bis Q8 von Qn, wie für Q. vorstehend definiert, durch T substituiert ist, mit einer Verbindung der Formel (XI): 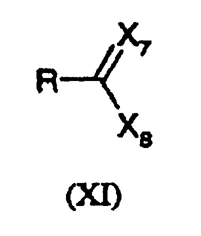 wobei einer der Reste C(X5)X6 und C(X7)X8 ein gegebenenfalls substituiertes alpha-(Abgangsgruppen-substituiertes) Keton ausmacht, wobei die Abgangsgruppe beispielsweise ein Halogenatom oder eine (Alkyl- oder Aryl-)-Sulfonyloxy-Gruppe ist, und der andere der Reste C(X5)X6 und C(X7)X8 ein gegebenenfalls substituiertes Amid, Thioamid oder Amidin ausmacht, so dass C(X5)X6 und C(X7)X8 Gruppen sind, die miteinander kondensiert sein können, so dass sie einen 5-gliedrigen Heterozyklus bilden, der 2 Heteroatome enthält, die in Kombination aus O, N und S gezogen werden, beispielsweise Thiazol, durch Verfahren, die im Stand der Technik wohlbekannt sind;
(g) für HET als gegebenenfalls substituierte 1,2,3-Triazole können Verbindungen der Formel (I) durch Cycloaddition über das Azid (wobei beispielsweise Y in (II) Azid ist) an Acetylene oder an Acetylen-Äquivalente hergestellt werden, wie gegebenenfalls substituierte Cyclohexa-1,4-diene oder gegebenenfalls substituierte Ethylene, die eliminierbare Substituenten, wie Arylsulfonyl tragen;
(h) für HET als 4-substituiertes 1,2,3-Triazol können Verbindungen der Formel (I) durch Umsetzen von Aminomethylisoxazolinen mit 1,1-Dihaloketonsulfonylhydrazonen hergestellt werden;
(i) für HET als 4-substituiertes 1,2,3-Triazol können Verbindungen der Formel (I) auch durch Umsetzen von Azidomethylisoxazolinen mit terminalen Alkinen mittels Cu(1)-Katalyse hergestellt werden; und danach nötigenfalls; (i) Entfernen jeglicher Schutzgruppen: (ii) Bilden eines pharmazeutisch annehmbaren Salzes; (iii) Bilden eines *in vivo* hydrolysierbaren Esters.

11. Verbindung, ausgewählt aus:
(*5R*)-3-(3-Fluor-4-(5-cyano-1,3,4-thiadiazol-2-yl)phenyl)-5-(1*H-*1,2,3-triazol-1-ylmethyl)-1,3-oxazolidin-2-on;
(5*R*)-3-(3-Fluor-4-(5-ethoxycarbonyl-1,3,4-thiadiazol-2-yl)phenyl)-5-(1*H*-1,2,3-triazol-1-ylmethyl)-1,3-oxazolidin-2-on;
(5*R*)-3-(4-(5-(Aminomethyl)-1,3-thiazol-2-yl)-3-fluorphenyl)-5-(1*H*-1,2,3-triazol-1-ylmethyl)-1,3-oxazolidin-2-on;
(*5R*)-3-(3-Fluor-4-(5-methyl-1,3,4-thiadiazol-2-yl)phenyl)-5-(1*H*-1,2,3-triazol-1-ylmethyl)-1,3-oxazolidin-2-on;
(5*R*)-3-(3-Fluor-4-(4-methyl-1,3-thiazol-2-yl)phenyl)-5-(1*H*-1,2,3-triazol-1-ylmethyl)-1,3-oxazolidin-2-on;
(*5R*)-3-(3-Fluor-4-(4-trifluormethyl)-1,3-thiazol-2-yl)phenyl)-5-(1*H*-1,2,3-triazol-1-ylmethyl)-1,3-oxazolidin-2-on; und
(5-(2-Fluor-4-((5R)-5-(1H-1,2,3-triazol-1-ylmethyl)-1,3-oxazolidin-2-on-3-yl)phenyl)-1,3,4-thiadiazol-2-yl)acetonitril;
oder ein pharmazeutisch annehmbares Salz oder ein *in vivo* hydrolysierbarer Ester davon.
